(19) Europäisches Patentamt
European Patent Office
Office européen des brevets

(11) EP 3 190 191 A1

(12) EUROPEAN PATENT APPLICATION

(43) Date of publication:
12.07.2017 Bulletin 2017/28

(51) Int Cl.:
*C12Q 1/68* (2006.01)

(21) Application number: 16382007.9

(22) Date of filing: 11.01.2016

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicants:
- Institut d'Investigació Biomèdica de Bellvitge (IDIBELL)
08908 Hospitalet de Llobregat (ES)
- Fundación Para la Investigación del Hospital Universitario y Politécnico La Fe de la Comunidad Valenciana
46026 Valencia (ES)
- Fundacion para la Investigacion Medica Aplicada
31008 Pamplona (ES)

(72) Inventors:
- SANDOVAL DEL AMOR, Juan
46026 Valencia (ES)
- MONTUENGA BADIA, Luís
31008 Pamplona (ES)
- DÍAZ LAGARES, Ángel
08908 Hospitalet de Llobregat (Barcelona) (ES)
- MÉNDEZ GONZÁLEZ, Jesús
08908 Hospitalet de Llobregat (Barcelona) (ES)
- HERVÁS MARÍN, David
46026 Valencia (ES)
- ESTELLER BADOSA, Manel
08908 Hospitalet de Llobregat (Barcelona) (ES)

(74) Representative: Carpintero Lopez, Francisco et al
Herrero & Asociados, S.L.
Cedaceros 1
28014 Madrid (ES)

(54) **METHOD AND KIT FOR THE DIAGNOSIS OF LUNG CANCER**

(57) The present invention refers to the field of cancer and, in particular, to an *in vitro* method for the diagnosis of lung cancer by determining in a biologicla sample, taken from a subject, the methylation level of particular genes. It further relates to biomarkers and kits useful for said method.

EP 3 190 191 A1

## Description

### Field of the invention

The present invention refers to the field of cancer and, in particular, to an *in vitro* method for the diagnosis of lung cancer by determining in a biological sample, taken from a subject, the methylation level of particular genes.

### Background of the invention

Despite intense research in the field of early cancer detection, there is still a lack of biomarkers for the reliable detection of malignant tumors, including lung cancer, which is the leading cause of cancer-related death worldwide with 1.3 million deaths annually, following data from the World Health Organization (WHO) in 2011. Late diagnosis in lung cancer is one of the main reasons of the extremely high mortality of this diasease. On one hand, screening by means of low-dose helical computed tomography (LDCT) has shown to reduce mortality in a large randomized trial, however the positive predictive value is still low. On the other hand, low sensitivity associated with minimally invasive cytologies is also a current hurdle for the accurate diagnosis of lung cancer. Thus, lung cancer early detection using non-invasive strategies is a major challenge to improve survival and its refinement is urgently needed to ameliorate the overall mortality figures for lung cancer worldwide. Epigenetic biomarkers, mainly DNA methylation, have emerged as one of the most promising approaches to improve cancer diagnosis and present several advantages as compared to other markers, such as gene expression or genetic signatures. DNA methylation alterations are covalent modifications that are remarkably stable and often occur early during carcinogenesis. Additionally, DNA methylation can be detected by a wide range of sensitive and cost-efficient techniques even in samples with low tumor purity. This epigenetic modification can also be detected in different biological fluids which represents a promising tool for non-invasive cancer detection. CpG island hypermethylation of *MGMT* and *GSTP1* has already proven useful for the chemotherapy response prediction in gliomas (Barault L et al., Digital PCR quantification of MGMT methylation refines prediction of clinical benefit from alkylating agents in glioblastoma and metastatic colorectal cancer. Ann Oncol 2015;26:1994-9) and the screening of prostate cancer (Hoque MO et al. Quantitative methylation-specific polymerase chain reaction gene patterns in urine sediment distinguish prostate cancer patients from control subjects. J Clin Oncol 2005;23:6569-75), respectively. Great efforts have been undertaken in identifying suitable DNA methylation markers to improve lung cancer diagnosis. However, only one biomarker -SHOX2 methylation - has been commercialized to date (Dietrich D et al,. Performance evaluation of the DNA methylation biomarker SHOX2 for the aid in diagnosis of lung cancer based on the analysis of bronchial aspirates. Int J Oncol 2012;40:825-32), although is not routinely used in the clinic.
Interestingly, the inventors have identified DNA methylation biomarkers already present in early stage lung cancer and globally absent in normal tissue, providing a novel epigenetic tool to improve lung cancer diagnosis.

### Summary of the invention

In a first aspect, the present invention refers to an *in vitro* method for the diagnosis of lung cancer comprising the step of:

a) determining the methylation level of a gene in a test sample, taken from a subject, wherein the gene is one gene, a two-gene combination, a three-gene combination or a four-gene combination, and wherein the gene(s) is(are) selected from the group consisting of BCAT1, TRIM58, ZNF177 and CDO1, and at least one gene is BCAT1;
b) comparing the methylation level determined in step a) to a reference; and
c) identifying the subject as being likely to have lung cancer, if the methylation level of the test sample is higher than the methylation level of the reference, and identifying the subject as unlikely to have lung cancer if the methylation level of the test sample is below the methylation level of the reference.

In second aspect, the present invention refers to a biomarker for *in vitro* lung cancer diagnosis, wherein the biomarker comprises a methylated gene, containing one or more methylated CpG site(s), wherein the gene is selected from one gene, a two-gene combination, a three-gene combination or a four-gene combination, and wherein the gene(s) is(are) selected from the group consisting of BCAT1, TRIM58, ZNF177 and CDO1, and at least one gene is BCAT1.
In a third aspect, the present invention refers to a kit to carry out the method according to the first aspect of the invention, comprising:

- primers for amplifying a CpG-containing nucleic acid of a gene, and/or
- means for detecting the presence of methylated CpG site(s) in said amplified nucleic acid,

wherein the gene is selected from one gene, a two-gene combination, a three-gene combination or a four-gene combination, and
wherein the gene(s) is(are) selected from the group consisting of BCAT1, TRIM58, ZNF177 and CDO1, and at least one gene is BCAT1.

In a fourth aspect, the present invention refers to the use of a method according to the first aspect of the invention, or of a biomarker according to the second aspect of the invention, or of a kit according to the third aspect of the invention, for *in vitro* lung cancer diagnosis.

Other objects, features, advantages and aspects of the present application will become apparent to those skilled in the art from the following description and appended claims.

Description of the drawings

**Figure 1. Epigenetic signature in bronchial aspirates (BAS). (A-D)** DNA methylation levels in bronchial aspirates from patients with lung cancer and control donors of Branched Chain Aminoacid Transaminase 1 (BCAT1) gene (A); Tripartite Motif Containing 58 (TRIM58) gene (B); Cysteine Dioxygenase type 1 (CDO1) gene (C), and Zinc Finger Protein 177 (ZNF177) gene (D). NT (light grey circle dots) stands for non-tumoral and T (dark grey square dots) for tumor. P values for all the analyses were calculated using the two-sided Mann-Whitney U test. *** corresponds to $p<0.001$. **(E-H)** Receiver Operating Characteristics (ROC) curves and Area Under the Curve (AUC) for BCAT1 gene (E); TRIM58 (F); CDO1 gene (G); ZNF177 gene (H). **(I)** The AUC for the combination of BCAT1, TRIM58, CDO1 and ZNF177 (referred to as "4-gene epigenetic signature"), using a logistic regression model. **(J)** Sensitivity (continuous line) and specificity (dotted line) profiles for the different possible cut-off values of the results from the logistic regression model of (I).

**Figure 2.- Results of the epigenetic prediction model for BAS.** Nomogram for prediction of cancer risk. To calculate the probability of cancer (POC), a vertical line straight upward from each factor (*BCAT1*, *CDO1*, *TRIM58, ZNF177)* to the Points line had to be drawn. Then, the points from each predictor were summed and with the result, a vertical line was drawn from the Total Points line of the nomogram downwards where the Probability of tumor line was depicted. As a practical example, a patient with the following methylation levels for each gene *(BCAT1*: 2%, *CDO1*: *4%; TRIM58:* 10% and *ZNF177*: 15%) would get the corresponding points from the Points line: *BCAT11*: 8 points, *CDO1*: 12 points, *TRIM58*: 12 points and *ZNF177*: 30 points. The sum of the four values yielded a total points value of 62. These points correspond to a POC higher than 95%.

**Figure 3. Epigenetic signature in bronchioalveolar lavages (BAL). (A-D)** DNA methylation levels in bronchioalveolar lavages from patients with lung cancer and control donors of BCAT1 gene (A); TRIM58 gene (B); CDO1 gene (C); ZNF177 gene (D). NT (light grey circle dots) stands for non-tumoral and T (dark grey square dots) for tumor. P values for all the analyses were calculated using the two-sided Mann-Whitney U test. *** corresponds to $p<0.001$; * $p<0.05$. **(E-H)** ROC curves and AUCs for BCAT1 gene (E); TRIM58 (F); CDO1 gene (G); ZNF177 gene (H). **(I)** The AUC for the combination of BCAT1, TRIM58, CDO1 and ZNF177, using a logistic regression model. **(J)** Sensitivity (continuous line) and specificity (dotted line) profiles for the different possible cut-off values of the results from the logistic regression model of (I).

**Figure 4. Epigenetic signature in sputums. (A-D)** DNA methylation levels in sputums from patients with lung cancer and control donors of BCAT1 gene (A); TRIM58 gene (B); CDO1 gene (C); ZNF177 gene (D). NT (light grey circle dots) stands for non-tumoral and T (dark grey square dots) for tumor. P values for all the analyses were calculated using the two-sided Mann-Whitney U test. *** corresponds to $p<0.001$; ** $p<0.01$ and * $p<0.05$. **(E-H)** ROC curves and AUCs for BCAT1 gene (E); TRIM58 (F); CDO1 gene (G); ZNF177 gene (H). **(I)** The AUC for the combination of BCAT1, TRIM58, CDO1 and ZNF177, using a logistic regression model. **(J)** Sensitivity (continuous line) and specificity (dotted line) profiles for the different possible cut-off values of the results from the logistic regression model of (I).

**Figure 5. Epigenetic signature in formalin-fixed paraffin-embedded (FFPE) samples. (A-D)** DNA methylation levels of BCAT1 gene (A); TRIM58 gene (B); CDO1 gene (C); and ZNF177 gene (D), in paraffin-embedded sections from patients with lung cancer and control donors. P values for all the analyses were calculated using the two-sided Mann- Whitney U test. NT (light grey circle dots) stands for non-tumoral and T (dark grey square dots) for tumor. *** correspond to $p<0.001$. **(E-H)** ROC curves and AUCs with 95% confidence intervals of BCAT1 gene (E); TRIM58 (F); CDO1 gene (G); ZNF177 gene (H).

**Figure 6. Differentially methylated levels in neighboring CpGs on the selected candidate genes.** Each data point represents the mean $\beta$-value of the group (control: continuous line with empty circles; adenocarcinoma: dotted line and squamous: continuous line) and whiskers show standard error of the mean (s.e.m). Surrounding CpGs are displayed on X axis (significant and selected CpG is highlighted in bold). Empty and crosswise striped squares indicated CpG islands and CpG shores regions respectively. BCAT1 gene (A); CDO1 gene (B); TRIM58 gene (C); ZNF177 gene (D).

**Figure 7.- Expression analysis in lung primary tumor patients using genomewide DNA methylation datasets.** Expression values of BCAT1 gene (A); TRIM58 gene (B); CDO1 gene (C); ZNF177 gene (D), using the TCGA database. P values for all the analyses were calculated using the two-sided Mann-Whitney U test. NT (light grey circle dots) stands for non-tumoral and T (dark grey square dots) for tumor. *** correspond to p<0.001.

**Figure 8.- Expression analysis based on histological subtypes from primary tissues of the TCGA database.** Expression values of BCAT1 gene (A); TRIM58 gene (B); CDO1 gene (C); ZNF177 gene (D), in primary tumor samples subclassified by histological subtypes adenocarcinomas (ADC) and squamous cell carcinomas (SCC). Non-Tumour in ADC (light grey circle dots), Tumour in ADC (dark grey circle dots), Non-Tumour in SCC (light grey square dots) and Tumour in SCC (dark grey square dots). P-values for all the analyses were calculated using the two-sided Mann-Whitney U test. *** corresponds to p<0.001, · corresponds to p<0.1 and n.s. to p>0.1.

Detailed description of the invention

It must be noted that as used in the present application, the singular forms "a", "an" and "the" include their correspondent plurals unless the context clearly dictates otherwise. Unless defined otherwise, all technical and scientific terms used herein have the same meaning as commonly understood by one of ordinary skill in the art to which this invention belongs.

The authors of the present invention have found that four genes, BCAT1, TRIM58, CDO1 and ZNF177, are differentially methylated in lung cancer. As shown in the Examples, the level of methylation of any one of the genes BCAT1, TRIM58, ZNF177 and CDO1 was higher in the samples taken from subjects with lung cancer than in control samples (samples taken from tumor-free subjects) (see panels A-D of Figs 1, 3-5). Thus, in a first aspect, the present invention refers to an *in vitro* method for the diagnosis of lung cancer (referred to as method of the invention) comprising the steps of:

a) determining the methylation level of a gene in a test sample, taken from a subject, wherein the gene is one gene, a two-gene combination, a three-gene combination or a four-gene combination, and wherein the gene(s) is(are) selected from the group consisting of BCAT1, TRIM58, ZNF177 and CDO1;

b) comparing the methylation level determined in step a) to a reference; and

c) identifying the subject as being likely to have lung cancer, if the methylation level of the test sample is higher than the methylation level of the reference, and identifying the subject as unlikely to have lung cancer if the methylation level of the test sample is below the methylation level of the reference.

The present invention may be practiced using each gene separately or using combinations of two, three or four genes. Thus, any of the genes identified in the present application may be used individually or as a set of genes in any combination with any of the other genes that are recited in the application, i.e. a two-gene combination, a three-gene combination or a four-gene combination, wherein the genes are selected from the group consisting of BCAT1, TRIM58, ZNF177 and CDO1. In the context of the present invention the term "diagnosis" refers to determining the likelihood of having or suffering from lung cancer. It also refers to identifying or determining the presence of lung cancer.

The term "lung cancer" refers to non-small cell lung cancer (NSCLC) and small cell lung cancer (SCLC). NSCLC accounts for about 80% of the lung cancers and is a heterogeneous clinical entity with major histological subtypes such as squamous cell carcinoma (SCC), adenocarcinoma (ADC) and large cell carcinoma. According to the histological classification of the WHO/International Association for the Study of Lung Cancer (Travis et al., Histological typing of lung and pleural tumours, 3rd ed. Berlin: Springer-Verlag, 1999) other subtypes of NSCLC are large cell carcinoma; adenosquamous carcinoma; carcinoma with pleomorphic, sarcomatoid or sarcomatous elements; carcinoid tumour; carcinoma of salivary gland type and unclassified carcinomas. Preferably, in the present invention the lung cancer is a NSCLC, more preferably NSCLC is of the subtype ADC, SCC or large cell carcinoma, and even more preferably ADC or SCC.

A common feature of the different subtypes of NSCLC is the somewhat slower growth and spread compared to SCLC, enabling surgical eradiaction is its early stages. Disappointingly, only a minor fraction of NSCLC cases are currently diagnozed in clinical stages I to II, where surgical removal is the therapy of choice. The major reasons for late diagnosis are the late appearance of symptoms and, as mentioned above, a lack of reliable biomarkers for its early detection. Interestingly, the present invention provides methods that allow diagnosis of lung cancer in early stage. Thus, preferably, the methods of the first aspect of the present invention refers to method for detecting lung cancer in early stage and more preferably NSCLC in early stage. In the context of the present invention, lung cancer in early stage refers to stage 0, stage I and stage II, i.e. NSCLC in stage 0, I and II and SCLC in stage 0, I and II; and more preferably it refers to stage I. The classification of the different stages of lung cancer and its (sub)types is well known by the skilled in the art and in the present invention the classification according to AJCC Cancer Staging Manual 7th edition; Chapter 25; Lung - original pages 253-266 is used.

In the context of the present invention the term "subject" refers to any member of the class Mammalia, including, without limitation, humans and non-human primates such as chimpazees and other apes and monkey species; farm animal such as cattle, sheep, pigs, goats and horses; domestic mammals such as dogs and cats; laboratory animals incluiding rodents such as mice, rats and guinea pigs, and the like. The term does no denote a particular age or sex. Thus, adult and newborn subjects, as well as fetuses, whether male or female, are intended to be included within the scope of this term. The subject is preferably a human.

The term "test sample", as used herein, refers to a biological sample taken from a subject under study. The biological sample contains any biological material suitable for detecting the desired methylation level in one or more CpG site(s) and is a material comprising genetic material from the subject. In the present invention, the sample comprises genetic material, e.g., DNA, genomic DNA (gDNA), complementary DNA (cDNA), RNA, heterogeneous nuclear RNA (hnRNA), mRNA, etc., from the subject under study. In a particular embodiment, the genetic material is DNA. In a preferred embodiment the DNA is genomic DNA. In another preferred embodiment, the DNA is circulating DNA. Isolating the nucleic acid of the sample can be perfomed by standard methods known by the person skilled in the art, such as those described in Sambrook et al., (Molecular Cloning: A Laboratory Manual, New York: Cold Spring Harbor Press, 1989).

Methylated genes are expressed in tumor tissue samples and they can also be detected in biological fluids comprising tumor cells. Therefore, in a particular embodiment, the biological sample is a lung tissue sample or a biological fluid, and in yet another more particular embodiment the biological sample is a biological fluid, so the method is less invasive than the ones requiring a tissue sample taken by means of biopsy. In a preferred embodiment of any of the methods of the present invention defined above, the biological sample is a biological fluid selected from BAS, BAL, sputum, saliva, whole blood, serum, plasma, urine, feces, ejaculate, a buccal or buccal-pharyngeal swab, pleural fluid, peritoneal fluid, pericardic fluid, cerebrospinal fluid and intra-articular fluid. Preferably the biological fluid is selected from BAS, BAL, blood and sputum, more preferably BAS, BAL and blood, and even more preferably the biological sample is BAS.

The term "gene" is intended to include not only regions encoding gene products but also regulatory regions including, e.g., promoters, termination regions, translational regulatory sequences (such as ribosome binding sites and internal ribosome entry sites), enhancers, silencers, insulators, boundary elements, replication origins, matrix attachment sites, and locus control regions. The term "gene" further includes all introns and other DNA sequences spliced from the mRNA transcript, along with variants resulting from alternative splice sites. The term "gene" further includes any portion of a gene, e.g. any portion of the regions mentioned above. The gene(s) or gene portion(s) of the invention are also referred herein as marker(s) or marker gene(s).

The genes according to the invention include:

- BCAT1, which refers to the gene Branched Chain Amino-Acid Transaminase 1. BCAT1 is a cytosolic enzyme that promotes cell proliferation though aminoacid catabolism and high frequency of methylation on *BCAT1* promoter in colorectal cancer has been reported. Its sequence reference is GenBank NM_005504.
- TRIM58, which refers to the gene tripartite motif containing 58. TRIM58 is an E3 ubiquitin ligase superfamily member that has been shown methylated in hepatocytes derived from hepatitis B virus-related hepatocellular carcinoma. Its sequence reference is GenBank NM_015431.
- CDO1, which refers to the gene cysteine dioxygenase type 1. CDO1 has been postulated as a tumor suppressor gene silenced by promoter methylation in multiple human cancers, including breast, esophagus, lung, bladder and stomach. Its sequence reference is GenBank NM_001801.
- ZNF177, which refers to the gene of a zinc finger transcription factor that has been reported to be methylation-silenced in gastric cancer cell lines. Its sequence reference is GenBank NM_003451.

For each of the genes mentioned above, the inventors have identified portions of said genes that are particularly useful in the method of the present invention. Thus, in a particular embodiment of the present invention, BCAT1 gene refers to a portion of BCAT1 gene comprising a sequence selected from the group consisting of SEQ ID NOs 1-3. In another particular embodiment, TRIM58 gene refers to a portion of TRIM58 gene comprising a sequence selected from the group consisting of SEQ ID NOs 4-8. In another particular embodiment, CDO1 gene refers to a portion of CDO1 gene comprising a sequence selected from the group consisting of SEQ ID NOs 9-11. In another particular embodiment, ZNF177 gene refers to a portion of ZNF177 gene comprising a sequence selected from the group consisting of SEQ ID NOs 12-15. In a preferred embodiment of any of the embodiments of this paragraph each gene is represented by any one of the mentioned sequences. Variants according to the present invention include nucleotide sequences that are at least 60%, 65%, 70%, 75%, 80%, 85%, 90%, or 95% similar or identical to any one of sequences SEQ ID NO 1-15. The degree of identity between two nucleic acid molecules is determined using computer algorithms and methods that are widely known for the persons skilled in the art. The identity between two nucleic acid sequences is preferably determined by using the BLASTN algorithm (BLAST Manual, Altschul et al., 1990, NCBI NLM NIH Bethesda, Md. 20894, Altschul, S., et al., J. Mol Biol 215:403-10).

The term "methylation" or "DNA methylation", as used herein, refers to a biochemical process involving the addition of a methyl group to the cytosine (C) or adenine (A) DNA nucleotides, preferably to cytosine. DNA methylation at the 5 position of cytosine, resulting in 5-methylcytosine (5-mC), may have the specific effect of reducing gene expression and has been found in every vertebrate examined. In adult non-gamete cells, DNA methylation typically occurs in a CpG site. The term "CpG site", as used herein, refers to regions of DNA where a cytosine nucleotide occurs next to a guanine (G) nucleotide in the linear sequence of bases along its length. "CpG" is shorthand for "C-phosphate-G", that is, cytosine and guanine separated by only one phosphate; phosphate links any two nucleosides together in DNA. The terms "CpG" and "CpG site" may be used interchangeably in the present context.

The methylation level of a gene can be determined at one or more CpG site(s). If more than one CpG sites are used, methylation can be determined at each site separately or as an average of the CpG sites taken together. Preferably, the methylation of more than one CpG site is determined and the methylation level is given as an averange value of the CpG sites, in particular in the form of average beta-value or percentage. The techniques for detection of DNA methylation are known in the art and include, without limitation, bisulfite modification based technologies, enzymatic digestions based methodologies, affinity-enriched based technologies and high throughput analysis. These techniques include bisulfite sequencing, Methylation Specific PCR (MSP), pyrosequencing, ConLight-MSP (Conversion-specific Detection of DNA Methylation Using Real-time Polymerase Chain Reaction), SMART_MSP (Sensitive Melting Analysis after Real Time-Methylation Specific PCR), Matrix-assisted laser desorption/ionization-time of flight (Mass Array Epityper Sequenom), HPLC (High perfomance liquid chromatography), Methyl-Beaming, droplet digital PCR, COBRA (Combined Bisulfite Restriction Analysis), reduced representation bisulfite sequencing (RRBS), HELP assay (Hpall tiny fragment Enrichment by Ligationmediated PCR) and MethDet (methylation detection), Methylated DNA immunoprecipitation (MeDIP), Methyl-Cap, methylation binding domain assays, arrays and Whole Genome Bisulfite Sequencing.

Preferably the detection of methylation in any one of the methods described in the present invention is performed by bisulfite sequencing or pyrosequencing. More preferably the level of methylation is determined by pyrosequecing since pyrosequencing is an affordable and quantitative method that counterbalances some weaknesses of previous and extensively used methods, due to its easy standardization and lower false positive rate. Moreover, pyrosequencing is a suitable approach in a clinical setting because it represents a quantitative and reproducible method able to detect multiple CpGs not only in FFPE tissues but also in non-invasive samples as biological fluids, as shown in the Examples of the present application. Methods for pyrosequencing are well known in the art and described, for example, in Nyrén, P. (The History of Pyrosequencing. 2007. Methods Mol Biology 373: 1-14). Thus, in a preferred embodiment of any one of the embodiments of the first aspect of the invention, the methylation level is determined by pyrosequencing.

Bisulfite sequencing method for detecting a methylated CpG-containing nucleic acid comprises the steps of: bringing a nucleic acid-containing sample into contact with an agent that modifies unmethylated cytosine; and amplifying the CpG containing nucleic acid in the sample using CpG-specific oligonucleotide primers, wherein the oligonucleotide primers distinguish between modified non-methylated nucleic acid and methylated nucleic acid and detect the methylated nucleic acid. The amplification step is optional and desirable, but not essential. The method relies on the PCR reaction to distinguish between modified (e.g., chemically modified) unmethylated DNA and methylated DNA. Such methods are described in US Patent No. 5,786,146 relating to bisulfite sequencing for detection of methylated nucleic acid.

The pyrosequencing method is a quantitative real-time sequencing method modified from the bisulfite sequencing method. Similarly to bisulfite sequencing, genomic DNA is converted by bisulfite treatment, and then, PCR primers corresponding to a region containing no CpG base sequence are constructed. Specifically, the genomic DNA is treated with bisulfite, amplified using the PCR primers, and then subjected to real-time base sequence analysis using a sequencing primer. The level of methylation is expressed as percentage or beta-value.

In the context of the present invention the term "reference" or "reference level" refers to a value or level, which has been determined by measuring the methylation level of the same gene(s) as the test sample in a biological sample taken from a subject or a population of subjects not suffering from lung cancer, i.e. lung cancer-free (also referred to as non-tumoral) subject/population. The sample taken from a lung cancer-free subject is also referred as "control sample", thus reference also refers to methylation level of a control sample. Preferably, the control sample is a sample of subjects matched on age and body mass index to the subject analysed. Preferably, the reference is a renference value, a cut-off value or a threshold.

As mentioned above, the method of the invention may comprise determining the methylation level of a combination of genes selected from the group consisting of BCAT1, TRIM58, CDO1 and ZNF177 (two-gene, three-gene or four-gene combination), in which case the methylation level is compared to a combined reference-level of said combination of genes. The measured methylation levels can be combined by arithmetic operations such as addition, substraction, multiplication and arithmetic manipulations of percentages, square root, exponentiation, and logarithmic funcions. Levels can also be combined following manipulation using various models e.g. logistic regression and maximum likelihood estimates. Various means of calculating the combined reference-value can be performed by means known to the skilled in the art.

In a particular embodiment of the method of the invention according to any one of the embodiments mentioned above, the level of methylation of the test sample is higher than the level of methylation of the control sample, when it is at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% or more higher than in the control sample. Preferably it is at least 50% higher.

In a particular embodiment of the method of the invention, the methylation level of the test sample is considered to be higher than the reference when the differences in average beta-values between groups (tumoral and non-tumoral) is higher than a set threshold, preferably higher than 0.20.

The first aspect of the invention also refers to an *in vitro* method for the diagnosis of lung cancer comprising the steps of:

a) determining the methylation level of a gene in a test sample, taken from a subject, wherein the gene is one gene, a two-gene combination, a three-gene combination or a four-gene combination, and wherein the gene(s) is(are) selected from the group consisting of BCAT1, TRIM58, ZNF177 and CDO1;
b) constructing a percentile plot of the methylation level of said gene or combination of genes obtained from a sample from a non-tumoral population;
c) constructing a ROC curve based on the methylation level determined in the non-tumoral population and on the methylation level determined in a population with lung cancer;
d) selecting from the ROC-curve the desired combination of sensitivity and specificity;
e) determining from the percentile plot the methylation level corresponding to the determined or chosen specificity; and
f) predicting that the subject is likely to have lung cancer, if the methylation level of said gene or combination of genes in the test sample is equal to or higher than said methylation level corresponding to the desired combination of sensitivity/specificity, and predicting that the subject is unlikely to have lung cancer, if the methylation level in the test sample is lower than said methylation level corresponding to the desired combination of sensitivity/specificity.

The sensitivity of any given screening test is the proportion of individuals with the condition who are correctly identified or diagnosed by the test, e.g. the sensitivity is 100%, if all individuals with a given condition have a positive test. The specificity of a given screening test is the proportion of individuals without the condition who are correctly identified or diagnosed by the test, e.g. the specificity is 100%, if all individuals without the condition have a negative test result. Thus, the sensitivity is defined as the (number of true-positive test results) / (number of true-positive + number of false-negative test results). The specificity is defined as (number of true-negative results) / (number of true-negative + number of false-positive results). The specificity of the method according to the invention is preferably from 70% to 100%, such as from 75% to 100%, more preferably 80% to 100%, more preferably 90% to 100%. Thus, in one embodiment of the present invention the specificity is 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100%. The sensitivity of the method according to the invention is preferably from 70% to 100%, such as from 75% to 100%, more preferably 80% to 100%, more preferably 90% to 100%. Thus, in one embodiment of the present invention the sensitivity is 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100%.

In another embodiment of the first aspect of the invention according to any one of the embodiments described above, when the method for the diagnosis refers to a method for determing the likelihood of having or suffering from lung cancer, the method of the invention comprises the use of an algorithm to calculate the likelihood of having lung cancer or the probability of cancer (POC).

The inventors generated a mathematical algorithm to calculate the probability of cancer for any sample type and gene combination. The general form of said algorithm was (formula I):

$$\Pr(Cancer) = \frac{e^{a+b*TRIM58+c*ZNF177+d*CDO1+e*BCAT1}}{1+e^{a+b*TRIM58+c*ZNF177+d*CDO1+e*BCAT1}} * 100$$

where the coefficients a, b, c, d and e take the log-odd values of cancer estimated by a multivariable logistic regression model adjusted using maximum likelihood to methylation values data from a specific sample type and a specific combination of the four genes.

In a particular embodiment, the method of the invention comprises determining the methylation level of BCAT1 gene and the algorithm used to calculate the POC is of formula II:

$$\Pr(Cancer)_{BAS} = \frac{e^{-1.86+0.63*BCAT1}}{1+e^{-1.86+0.63*BCAT1}} * 100$$

In another embodiment, the method comprises determining the methylation level of BCAT1 and TRIM58 genes and the algorithm is of formula III:

$$\Pr(Cancer)_{BAS} = \frac{e^{-3.03+0.24*TRIM58+0.55*BCAT1}}{1+e^{-3.03+0.24*TRIM58+0.55*BCAT1}} * 100$$

In another embodiment, the method comprises determining the methylation level of BCAT1, TRIM58 and ZNF177 genes and the algorithm is of formula IV:

$$\Pr(Cancer)_{BAS} = \frac{e^{-5.15+0.20*TRIM58+0.32*ZNF177+0.66*BCAT1}}{1+e^{-5.15+0.20*TRIM58+0.32*ZNF177+0.66*BCAT1}} * 100$$

In another embodiment, the method comprises determining the methylation level of BCAT1, TRIM58, ZNF177 and CDO1 genes and the algorithm is of formula V:

$$\Pr(Cancer)_{BAS} = \frac{e^{-6.13+0.18*TRIM58+0.30*ZNF177+0.47*CDO1+0.59*BCAT1}}{1+e^{-6.13+0.18*TRIM58+0.30*ZNF177+0.47*CDO1+0.59*BCAT1}} * 100$$

In a preferred embodiment according to any of the embodiments of the six previous paragraphs, the sample taken from the subject, of which the methylation level is determined, is a BAS. More preferably, the methylation level is determined by pyrosequencing and using the primers depicted in Table 7 (below).

In a particular embodiment of the invention according to any one of the preceding seven paragraphs, the likelihood of having lung cancer or the POC is at least 50%, at least 60%, at least 70%, at least 80%, at least 85%, at least 90%, at least 95%, or 100%, preferably it is between 80% and 100%, and more preferably, between 90% and 100%.

As mentioned above, the method of the invention may comprise determining the methylation level of one gene or of a combination of genes (two-gene, three-gene or four-gene combination) selected from the group consisting of BCAT1, TRIM58, ZNF177 and CDO1. Thus, in a particular embodiment of any of the methods according to the first aspect of the invention described above, the methylation level of at least BCAT1 or at least TRIM58 or at least CDO1 or at least ZNF177 is determined. In a particular embodiment methylation of BCAT1 is determined, in another particular embodiment methylation of TRIM58 is determined, in another particular embodiment methylation of ZNF177 is determined, and in another particular embodiment methylation of CDO1 is determined. As shown in Figs 1, 3-5 the level of methylation (panels A-D) and the AUC (panels E-H) of any of these genes was higher in the test samples than in control samples. In a preferred embodiment, the methylation of the gene BCAT1 or TRIM58 is determined, since any one of these genes provides the highest AUC (see Table 9, in Example 2) and thus the highest accuracy for a diagnostic method determining only the methylation level of one gene. Interestingly, by combination of the different marker genes according to the invention a synergistic effect is achieved (see Table 9). Specifically as used herein synergy refers to the phenomenon in which several markers acting together created a "gene combination" with greater sensitivity or specificity for diagnosis, than that predicted by knowing only the separate genes sensitivity or specificity.

Thus, in a preferred embodiment of the method of the first aspect of the invention, according to any one of the embodiments mentioned above, the methylation level is determined in a combination of two genes (two-gene combination) selected from the group consisting of BCAT1, TRIM58, CDO1 and ZNF177. In a preferred embodiment, the methylation level is determined in two genes selected from the group consisting of BCAT1, TRIM58, CDO1 and ZNF177, wherein one of said genes is BCAT1. In another embodiment, the methylation level is determined in two genes selected from the group consisting of BCAT1, TRIM58, CDO1 and ZNF177, wherein one of said genes is TRIM58. In another embodiment, the methylation level is determined in two genes selected from the group consisting of BCAT1, TRIM58, CDO1 and ZNF177, wherein one of said genes is ZNF177. In another embodiment, the methylation level is determined in two genes selected from the group consisting of BCAT1, TRIM58, CDO1 and ZNF177, wherein one of said genes is CDO1. In a preferred embodiment according to the previous embodiments, methylation of a two-gene combination is determined and the two-gene combination is selected from BCAT1 and TRIM58; BCAT1 and ZNF177; BCAT1 and CDO1; TRIM58 and ZNF177; TRIM58 and CDO1; or ZNF177 and CDO1. Preferably, the methylation of any two-gene combination in which one of the genes is BCAT1 is determined, i.e. BCAT1 and TRIM58; BCAT1 and ZNF177; BCAT1 and CDO1. As shown in Table 9, these BCAT1 containing two-gene combinations have higher AUC in all the different biological samples indicating that the combination improves specificity and sensitivity leading to a higher prediction efficacy.

In another embodiment of the method of the first aspect of the invention according to any one of the embodiments described above, the methylation level is determined in a combination of three genes (three-gene combination) selected from the group consisting of BCAT1, TRIM58, CDO1 and ZNF177. In a particular embodiment, one of the three genes is BCAT1, in another embodiment, one of the three genes is TRIM58, in another particular embodiment, one of the three genes is ZNF177, in another embodiment, one of the three genes is CDO1. In a preferred embodiment according to any of the previous embodiments, it is detected the methylation of a three-gene combination selected from BCAT1, TRIM58 and ZNF177; BCAT1, TRIM58 and CDO1; BCAT1, ZNF177 and CDO1; or TRIM58, ZNF177 and CDO1. Preferably, the methylation of any one of the three-gene combination in which one of the genes is BCAT1 is determined, i.e. BCAT1, TRIM58 and ZNF177; BCAT1, TRIM58 and CDO1; BCAT1, ZNF177 and CDO1. As shown in Table 9, these BCAT1 containing three-gene combinations have higher AUC in all the different biological samples. The combination improves specificity and sensitivity leading to a maximized prediction efficacy, identifying cancer cases that would not be detected with two-gene combinations.

In a further embodiment of the first aspect of the invention according to any one of the embodiments described above, the methylation of a four-gene combination is determined, wherein the four-gene combination is BCAT1, TRIM58, CDO1 and ZNF177. As shown in Figs 1, 3-5 and in Table 9, the AUC of the combination of these four genes was equal or higher than 0.85, in particular 0.91 for BAS samples, 0.85 for BAL samples, and 0.93 for sputum. Interestingly, internal validation of the AUC estimate for this combination yielded optimism corrected AUC of 0.90, showing high generalization of the predictive capacity of the combination.

Moreover, a nomogram based on the results of this four-gene combination is provided as a predictive tool for clinical diagnostic use (Example 2, Fig. 2). The use of this nomogram for *in vitro* lung cancer diagnosis is within the scope of the present invention. The nomogram has been obtained using the algorithm of formula (V). Results of the nomogram provide an individual probability (0%-100%) for suffering lung cancer for each patient (Fig. 2). Evaluation of the full range of predictions of the model shows that shifting the cut-off to POC=30% would yield a sensitivity of 100% and a specificity of 65.4% and shifting the cut-off to POC=80% would yield a sensitivity of 71.4% and a specificity of 92.3%. Sensitivity and specificity at the optimal cut-off (POC=63%) were 84.6% and 81.0% respectively. Thus, it has been shown by the inventors that this embodiment of the four-gene combination allows a particularly accurate and reliable diagnosis of lung cancer.

Interestingly, the methods of the invention described above allow an early diagnosis mainly based in non-invasive or minimally invasive samples. The performance of the methods in these type of samples, such as BAS, BAL and sputum, was outstanding despite the limited number of tumoral cells compared to FFPE samples. Surprisingly, the methods of the present invention provide a balanced and flexible approach able to cater to both extreme scenarios: the high sensitivity and low specificity of LDCT in screening programs and the high specificity and low sensitivity of cytology in respiratory specimens routinely used for lung cancer diagnosis. The epigenetic signatures of the present invention improves the predictions of cytology by providing a method for continuous predictions, in particular the four-gene epigenetic signature (See Example 2). In the context of the present invention, any one of the genes or the two-, three- and four-gene combinations described herein are also referred to as "epigenetic signatures of the invention" or as "one-gene epigenetic signature", "two-gene epigenetic signature", "three-gene epigenetic signature" or "four-gene epigenetic signature", respectively. Cytology is a useful dichotomized classifier producing two types of predictions: 100 % positive or 0% positive (100% negative). Therefore, the final output will be either a complete success or a total failure. In contrast, the epigenetic signatures of the present invention based in a logistic regression model, represented by a nomogram, are able to produce a continuous range of predictions between 100% positive and 0% positive. This way, not all predictions are a complete success or a total failure, uncertainty can be measured for each prediction and errors are almost always lower. In a virtual situation where the method of the invention predicts two negative samples with different probability of being positive: such as 5% and 49%, the bimodal classifier predictor (cytology) would have output only absolute responses: negative and negative. Therefore, no information about uncertainty and chances of being positive for patient 1 (very low) and patient 2 (almost 50%) would have been delivered. Thus, the four-gene epigenetic signature achieved higher diagnostic efficacy in bronchial fluids as compared with conventional cytology for early lung cancer detection. It also yielded a notably high specificity, one of the Achilles heels of LDCT and other methylation genes, and also improved sensitivity, which is generally limited when using cytology for early lung cancer diagnosis.

In a particular embodiment of any one of the methods of the first aspect of the invention described above, the methylation level of one or more of the genes BCAT1, TRIM58, CDO1 and ZNF177 is determined at one or more CpG site(s). In a particular embodiment, the CpG site(s) is/are located at a CpG island. In a particular embodiment, the CpG site(s) is/are located at the promoter region. In a particular embodiment, the CpG site(s) is/are located at the gene body. In a particular embodiment, the CpG site(s) is/are located at a CpG shore. In a particular embodiment, the CpG site(s) is/are located at both at a CpG island and a CpG shore. In a particular embodiment, the CpG site(s) is/are located at the N-shore, at the S-shore or at both the N-and the S-shores of said gene(s). The term "promoter region", as used herein, refers to an upstream region of DNA that initiates transcription of a particular gene. The term "CpG island", as used herein, relates to a DNA sequence, generally in a window of 200 to 2000 bp, with a GC content greater than 50% and an observed:expected CpG ratio of more than 0.6. The term "gene body" (also referred to as "body" in the present invention) refers to the entire gene from the transcription start site to the end of the transcript. The term "CpG shore", as used herein, relates to the DNA sequences, up to 2kb long, flanking a CpG island and showing a comparatively low GC density.

The start and end positions of the promoter, CpG island and shore/CpG island/shore of the genes of the present invention are depicted in Table 1.

Table 1: Start and end positions of the CpG island, of the shores flanking the CpG island and of the promoter regions in the BCAT1, TRIM58, CDO1 and ZNF177 genes.

| **Gene** | **Island** | | **Shore/Island/Shore** | | **Start promoter** | **End promoter** |
|---|---|---|---|---|---|---|
| | **start** | **end** | **start** | **end** | **TSS1500** | **1st exon** |
| **BCAT1** | 25055599 | 25056246 | 25053599 | 25058246 | 25103643 | 25102072 |
| **TRIM58** | 248020330 | 248021252 | 248018330 | 248026252 | 248019234 | 248020812 |
| **CDO1** | 115151548 | 115152713 | 115149548 | 115152713 | 115153223 | 115152019 |
| **ZNF177** | 9473589 | 9474001 | 9471589 | 9476001 | 9472210 | 9476402 |

Island start and island end indicate, respectively, the starting and ending positions of the CpG island by reference to the chromosome numbering according to Infinium HumanMethylation450 BeadChip, Manifest v1.2 or according to UCSC database, as in Genome Reference Consortium Human Build 37 (GRCh37) and UCSC hg19 as released on February 2009 (hereafter referred to as Infinium/UCSC). Shore/Island/Shore start indicates the starting position of the shore 5' to the CpG island by reference to the chromosome numbering according to Infinium/UCSC. Shore/Island/Shore end indicates the end position of the shore located 3' with respect of the CpG island by reference to the chromosome numbering according to Infinium/UCSC. The end position of the shore located 5' of the CpG island is the position adjacent in 5' to the island start position. The start position of the shore located 3' of the CpG island is the position adjacent in 3' to the island end position. Start promoter (TSS1500) indicates the start position of the promoter region by reference to the chromosome numbering according to Infinium/UCSC.

End promoter (1 st exon) indicates the last position of the first exon of the gene, which is adjacent to the last position of the promoter, by reference to the chromosome numbering as indicated above (Infinium/UCSC).

In a preferred embodiment of any one of the methods described above of the first aspect of the invention, the methylation level is determined at one or more of the CpG site(s) comprised in SEQ ID NO 1-3 for determining BCAT1's methylation, in SEQ ID NO 4-8 for determining TRIM58's methylation, in SEQ ID NO 9-11 for determining CDO1's methylation, and in SEQ ID NO 12-15 for determining ZNF177's methylation.

In a more preferred embodiment of any one of the methods described above of the first aspect of the invention, the methylation level of any of BCAT1, TRIM58, CDO1, ZNF177 and combinations thereof, is determined at one or more of the CpG site(s) of said genes, and the position(s) of the CpG site(s) is(are) selected from the ones depicted in Table 2 for BCAT1, Table 3 for TRIM58, Table 4 for CDO1 and Table 5 for ZNF177. In Tables 2-5 the indicated positions correspond to the C nucleotide of a CpG site according to MAPINFO/Illumina Infinium HumanMethylation450 BeadChip, Manifest v1.2 or according to UCSC database, as in Genome Reference Consortium Human Build 37 (GRCh37) and UCSC hg19 as released on February 2009.

In a particular embodiment according to any one of the embodiments of the first aspect of the invention mentioned above, in particular the ones described in the two previous paragraphs, the methylation of one or more of the genes BCAT1, TRIM58, CDO1 and ZNF177, is determined at at least two CpG sites, at least 3, at least 4, at least 5, at least 6, at least 7, at least 8, at least 9, at least 10, at least 12, at least 15 CpG sites or at all CpG sites of said gene(s). Preferably, the methylation level of said gene(s) is determined as the average value of said at least 2, 3, 4, 5, 6, 7, 8, 9, 10, 12, 15 or all CpG sites, and more preferably as the average value of all CpG sites.

Table 2.- Preferred CpG sites of BCAT1 gene

| targetID | Position MAPINFO | ucscRefGene_NAME | CpG content |
|---|---|---|---|
| cg15990629 | 25054873 | BCAT1 | N_Shore |

(continued)

| targetID | Position MAPINFO | ucscRefGene_NAME | CpG content |
|---|---|---|---|
| cg08980987 | 25054905 | BCAT1 | N_Shore |
| cg21172322 | 25055108 | BCAT1 | N_Shore |
| cg01494454 | 25055214 | BCAT1 | N_Shore |
| cg02585702 | 25055262 | BCAT1 | N_Shore |
| cg08724310 | 25055304 | BCAT1 | N_Shore |
| cg20342079 | 25055381 | BCAT1 | N_Shore |
| cg22229906 | 25055421 | BCAT1 | N_Shore |
| cg22814146 | 25055518 | BCAT1 | N_Shore |
| cg04543413 | 25055676 | BCAT1 | Island |
| | 25055938 | BCAT1 | Island |
| | 25055948 | BCAT1 | Island |
| | 25055957 | BCAT1 | Island |
| | 25055959 | BCAT1 | Island |
| | 25055961 | BCAT1 | Island |
| cg20399616 | 25055967 | BCAT1 | Island |
| | 25055978 | BCAT1 | Island |
| cg23930313 | 25056083 | BCAT1 | Island |
| cg23792314 | 25056243 | BCAT1 | Island |

Table 3.- Preferred CpG sites of TRIM58 gene

| targetID | Position MAPINFO | ucscRefGene_NAME | CpG content |
|---|---|---|---|
| cg04902327 | 248019234 | TRIM58 | N_Shore |
| cg15094634 | 248019757 | TRIM58 | N_Shore |
| cg04982874 | 248019816 | TRIM58 | N_Shore |
| cg26052730 | 248020331 | TRIM58 | Island |
| cg20855565 | 248020350 | TRIM58 | Island |
| cg10983544 | 248020377 | TRIM58 | Island |
| cg20429172 | 248020436 | TRIM58 | Island |
| cg20810478 | 248020632 | TRIM58 | Island |
| cg26157385 | 248020641 | TRIM58 | Island |
| | 248020671 | TRIM58 | Island |
| | 248020680 | TRIM58 | Island |
| | 248020688 | TRIM58 | Island |
| cg23054189 | 248020692 | TRIM58 | Island |
| | 248020695 | TRIM58 | Island |
| cg20146541 | 248020697 | TRIM58 | Island |
| | 248020704 | TRIM58 | Island |

(continued)

| targetID | Position MAPINFO | ucscRefGene_NAME | CpG content |
|---|---|---|---|
| | 248020707 | TRIM58 | Island |
| | 248020713 | TRIM58 | Island |
| cg07533148 | 248020812 | TRIM58 | Island |
| cg16021909 | 248021091 | TRIM58 | Island |
| cg09789636 | 248021163 | TRIM58 | Island |

Table 4.- Preferred CpG sites of CDO1 gene

| targetID | Position MAPINFO | ucscRefGene_NAME | CpG content |
|---|---|---|---|
| cg06682875 | 115150172 | CDO1 | N_Shore |
| cg07712493 | 115151427 | CDO1 | Island |
| cg07405021 | 115152019 | CDO1 | Island |
| cg16265906 | 115152326 | CDO1;CDO1 | Island |
| cg12880658 | 115152386 | CDO1;CDO1 | Island |
| cg16707405 | 115152413 | CDO1 | Island |
| cg02792792 | 115152420 | CDO1 | Island |
| cg14470895 | 115152431 | CDO1 | Island |
| cg23180938 | 115152485 | CDO1 | Island |
| cg08516516 | 115152492 | CDO1 | Island |
| | 115152466 | CDO1 | Island |
| | 115152468 | CDO1 | Island |
| | 115152475 | CDO1 | Island |
| | 115152484 | CDO1 | Island |
| cg11036833 | 115152494 | CDO1 | Island |
| | 115152496 | CDO1 | Island |
| | 115152503 | CDO1 | Island |
| | 115152509 | CDO1 | Island |
| | 115152522 | CDO1 | Island |
| cg07644368 | 115152785 | CDO1 | S_Shore |
| cg16198692 | 115152835 | CDO1 | S_Shore |
| cg04676799 | 115152938 | CDO1 | S_Shore |
| cg23029474 | 115153223 | CDO1 | S_Shore |

Table 5.- Preferred CpG sites of ZNF177 gene

| targetID | Position MAPINFO | ucscRefGene_NAME | CpG content |
|---|---|---|---|
| cg09492640 | 9472210 | ZNF177 | N_Shore |
| cg14323854 | 9473058 | ZNF177 | N_Shore |
| cg19275200 | 9473240 | ZNF177 | N_Shore |

(continued)

| targetID | Position MAPINFO | ucscRefGene_NAME | CpG content |
|---|---|---|---|
| cg05250458 | 9473565 | ZNF177 | N_Shore |
| cg05928342 | 9473598 | ZNF177 | Island |
| | 9473668 | ZNF177 | Island |
| cg13703871 | 9473674 | ZNF177 | Island |
| cg08065231 | 9473684 | ZNF177 | Island |
| cg09578475 | 9473688 | ZNF177 | Island |
| cg07788092 | 9473691 | ZNF177 | Island |
| cg09643544 | 9473696 | ZNF177;ZNF177 | Island |
| | 9473715 | ZNF177;ZNF177 | Island |
| cg12089570 | 9473715 | ZNF177;ZNF177 | Island |
| cg24189904 | 9473781 | ZNF177 | Island |
| cg17283453 | 9473880 | ZNF177 | Island |
| cg14737994 | 9474128 | ZNF177 | S_Shore |

In an even more preferred embodiment of the any one of the methods of the first aspect of the invention described above, the methylation level of any of BCAT1, TRIM58, CDO1, ZNF177 genes or combinations thereof is determined at one or more, preferably all, CpG site(s), selected from the ones depicted in Table 6, which are located in CpG islands. As shown in the examples of the present application, see Fig. 6, the CpG site(s) of Table 6 are the ones given the stadistically significant higher degree of methylation in test samples compared to control samples.

Table 6.- Most preferred CpG sites of BCAT1, TRIM58, CDO1, ZNF177

| NAME | TargetID | Group |
|---|---|---|
| BCAT1 | cg20399616 | Body (Island) |
| TRIM58 | cg23054189, cg07533148, cg20810478, cg16021909 | Promoter (Island) |
| ZNF177 | cg08065231 | Promoter (Island) |
| CDO1 | cg11036833 | Promoter (Island) |

In a particular embodiment of the first aspect of the present invention, the method of detecting the methylation of any of the genes BCAT1, TRIM58, CDO1, ZNF177 or combinations thereof of two, three or four genes, as described above in the methods of the first aspect of the invention, comprises the steps of: (a) isolating DNA from a biological sample; (b) treating the isolated DNA with bisulfite; (c) amplifying the treated DNA using primers capable of amplifying a fragment comprising the CpG site(s) of the above-mentioned genes; and (d) subjecting the product amplified in step (c) to pyrosequencing to determine the methylation of the gene(s). In a preferred embodiment, the primers for the pyrosequencing are the ones depicted in Table 7 (below) depending on the genes to be analysed, i.e. primers comprising SEQ ID NO 16-18 for determining the methylation level of BCAT1, primers comprising SEQ ID NO 19-21 for determining the methylation level of TRIM58, primers comprising SEQ ID NO 22-24 for determining the methylation level of ZNF177, primers comprising SEQ ID NO 25-27 for determining the methylation level of CDO1. In a more preferred embodiment, the methylation of the four-gene combination is determined, and more preferably in BAS samples. If the POC is to be determined, the algorithm of formula V is used.

The use of the methylated genes described above allows early diagnosis of lung cancer. Thus, a second aspect of the invention refers to a biomarker (referred to as biomarker of the invention) for *in vitro* lung cancer diagnosis, wherein the biomarker comprises a methylated gene selected from the group consisting of BCAT1, TRIM58, ZNF177, CDO1 and combinations thereof. That is, the biomarker comprises a methylated gene, wherein the gene is selected from one gene, a two-gene combination, a three-gene combination or a four-gene combination, and wherein the gene(s) is(are) selected from the group consisting of BCAT1, TRIM58, ZNF177 and CDO1. As used herein, "methylated gene" refers to a gene containing one or more methylated CpG site(s).

In a particular embodiment of the second aspect of the invention, the biomarker is a methylated gene selected from BCAT1, TRIM58, CDO1 or ZNF177. More preferably the biomarker is methylated BCAT1 gene or methylated TRIM58 gene.

In another embodiment of the second aspect of the invention, the biomarker comprises a methylated two-gene combination wherein the two genes are selected from the group consisting of BCAT1, TRIM58, CDO1 and ZNF177. In a particular embodiment, the two genes are selected from the group consisting of BCAT1, TRIM58, CDO1 and ZNF177, wherein one of said genes is BCAT1. In another embodiment, the two genes are selected from the group consisting of BCAT1, TRIM58, CDO1 and ZNF177, wherein one of said genes is TRIM58. In another embodiment, the two genes are selected from the group consisting of BCAT1, TRIM58, CDO1 and ZNF177, wherein one of said genes is ZNF177. In another embodiment, the methylation level is determined in two genes selected from the group consisting of BCAT1, TRIM58, CDO1 and ZNF177, wherein one of said genes is CDO1. In a preferred embodiment, the two-gene combination is selected from BCAT1 and TRIM58; BCAT1 and ZNF177; BCAT1 and CDO1; TRIM58 and ZNF177; TRIM58 and CDO1; or ZNF177 and CDO1. Preferably, the methylated two-gene combination is selected from BCAT1 and TRIM58, BCAT1 and ZNF177, or BCAT1 and CDO1.

In another embodiment of the second aspect of the invention, the biomarker comprises a methylated three-gene combination wherein the three genes are selected from the group consisting of BCAT1, TRIM58, CDO1 and ZNF177. In a particular embodiment, the three genes are selected from the group consisting of BCAT1, TRIM58, CDO1 and ZNF177, wherein one of said genes is BCAT1. In another embodiment, the three genes are selected from the group consisting of BCAT1, TRIM58, CDO1 and ZNF177, wherein one of said genes is TRIM58. In another embodiment, the three genes are selected from the group consisting of BCAT1, TRIM58, CDO1 and ZNF177, wherein one of said genes is ZNF177. In another embodiment, the methylation level is determined in three genes selected from the group consisting of BCAT1, TRIM58, CDO1 and ZNF177, wherein one of said genes is CDO1. In a preferred embodiment, the three-gene combination is selected from the group consisting of BCAT1, TRIM58 and ZNF177; BCAT1, TRIM58 and CDO1; BCAT1, ZNF177 and CDO1; and TRIM58, ZNF177 and CDO1. Preferably, the methylated three-gene combination is selected from BCAT1, TRIM58 and ZNF177; BCAT1, TRIM58 and CDO1; or BCAT1, ZNF177 and CDO1.

In a further embodiment of the second aspect of the invention, the biomarker comprises a methylated four-gene combination, wherein the genes are BCAT1, TRIM58, CDO1 and ZNF177. The advantages of the biomarkers comprising a combination of genes for use in lung cancer diagnosis are similar to the ones already described in detail in the first aspect of the invention for the embodiment in which the methylation level of the two-, three- or four-gene combination is determined.

In a particular embodiment according to any one of the embodiments described in the previous five paragraphs, the BCAT1 gene comprises any one of sequences SEQ ID NO 1-3, the TRIM58 gene comprises any one of sequences SEQ ID NO 4-8, the CDO1 gene comprises any one of sequences SEQ ID NO 9-11, the ZNF177 gene comprises any one of sequences SEQ ID NO 12-15. In a preferred embodiment of any one of the embodiments of this paragraph each gene is represented by any one of the mentioned sequences.

In a preferred embodiment according to any one of the embodiments described in the previous six paragraphs, the methylated gene contains one or more methylated CpG site(s), and the position(s) of said one or more CpG site(s) is(are) selected from the ones depicted in Tables 2-5. In a more preferred embodiment, said one or more methylated CpG site(s) is(are) selected from the ones depicted in Table 6.

In a particular embodiment according to any one of the embodiments of the second aspect of the invention, in particular the ones of the previous paragraph, the methylated gene contains at least two methylated CpG sites, at least 3, at least 4, at least 5, at least 6, at least 7, at least 8, at least 9, at least 10, at least 12, at least 15 methylated CpG sites or all the CpG sites are methylated, preferably, all the CpG site(s) are methylated.

The second aspect of the invention also refers to a methylated gene selected from the group consisting of BCAT1, TRIM58, ZNF177 and CDO1, or a methylated two-gene combination, a methylated three-gene combination or a methylated four-gene combination of said genes, for use as a biomarker for *in vitro* lung cancer diagnosis, preferably early lung cancer diagnosis. "Methylated gene combination" refers to a gene combination containing one or more methylated CpG sites. In particular embodiments, the methylated genes and combinations, and the methylated CpG sites are the ones described in the previous eight paragraphs for the biomarker of the invention.

The method of any of the embodiments described in the first aspect of the present invention can be carried out using a kit. Thus, a third aspect of the present invention refers to a kit for the *in vitro* diagnosis of lung cancer in a subject, comprising:

- primers for amplifying a CpG-containing nucleic acid of a gene, and/or
- means for detecting the presence of methylated CpG site(s) in said amplified nucleic acid,

wherein the gene is selected from one gene, a two-gene combination, a three-gene combination or a four-gene combination, and

wherein the gene(s) is(are) selected from the group consisting of BCAT1, TRIM58, ZNF177 and CDO1.

In a particular embodiment, the third aspect of the invention refers to a kit to carry out the method according to any one of the embodiments described in the first aspect of the invention, comprising:

- primers for amplifying a CpG-containing nucleic acid of a gene, and/or
- means for detecting the presence of methylated CpG site(s) in said amplified nucleic acid,

wherein the gene is selected from one gene, a two-gene combination, a three-gene combination or a four-gene combination, and
wherein the gene(s) is(are) selected from the group consisting of BCAT1, TRIM58, ZNF177 and CDO1.

In a particular embodiment of the third aspect of the invention according to any of the two previous paragraphs, the kit comprises primers for amplifying a CpG-containing nucleic acid of a gene selected from BCAT1 or TRIM58 or CDO1 or ZNF177. Preferably the gene is BCAT1 or TRIM58, more preferably the gene is BCAT1. In another embodiment, the kit comprises primers for amplifying two CpG-containing nucleic acids, one of each gene of the two-gene combinations described in the first aspect of the invention, i.e. the two-gene combination is selected from BCAT1 and TRIM58; BCAT1 and ZNF177; BCAT1 and CDO1; TRIM58 and ZNF177; TRIM58 and CDO1; or ZNF177 and CDO1, preferably the two-gene combinations contains BCAT1. In another embodiment, the kit comprises primers for amplifying three CpG-containing nucleic acids, one of each gene of the three-gene combinations described in the first aspect of the invention, i.e. the three-gene combination is selected from BCAT1, TRIM58 and ZNF177; BCAT1, TRIM58 and CDO1; BCAT1, ZNF177 and CDO1; or TRIM58, ZNF177 and CDO1, preferably the three-gene combinations contains BCAT1. In another embodiment, the kit comprises primers for amplifying four CpG-containing nucleic acids, in particular a CpG-containing nucleic acid of BCAT1 gene, a CpG-containing nucleic acid of TRIM58 gene, a CpG-containing nucleic acid of CDO1 gene, and a CpG-containing nucleic acid of ZNF177 gene.

In a particular embodiment, the kit of the third aspect of the invention comprises means for detecting the presence of methylated CpG site(s). More preferably, the kit comprises primers for amplifying a CpG-containing nucleic acid of the gene(s) according to any one of the embodiments of the previous paragraph, and means for detecting the presence of methylated CpG site(s) in said amplified nucleic acid(s).

In a particular embodiment of the third aspect of the invention according to any of the embodiments described in the previous four paragraphs, the BCAT1 gene comprises any one of sequences SEQ ID NO 1-3, the TRIM58 gene comprises any one of sequences SEQ ID NO 4-8, the CDO1 gene comprises any one of sequences SEQ ID NO 9-11, the ZNF177 gene comprises any one of sequences SEQ ID NO 12-15. In a preferred embodiment of any of the embodiments of this paragraph each gene is represented by any one of the mentioned sequences.

In a preferred embodiment according to any of the embodiments described in the previous five paragraphs, the amplified CpG-containing nucleic acids contains one or more, preferably all, CpG site(s) selected from the CpG sites of the positions depicted in Tables 2-5. In a more preferred embodiment, said one or more, preferably all, CpG site(s) is(are) selected from the ones depicted in Table 6.

The term "primer", as used herein, refers to a single-stranded DNA or RNA molecule, with up to 30, 25, 20, 19, 18, 17, 16, 15, 14 or 13 bases in length (upper limit). The oligonucleotides of the invention are preferably DNA or RNA molecules of at least 2, 3, 4, 5, 6, 7, 8, 9, 10, 11, 12, or 13 bases in length (lower limit). Ranges of base lengths can be combined in all different manners using the afore-mentioned lower and upper limits, for example at least 2 and up to 30 bases, at least 8 and up to 15 bases, at least 5 and up 15 bases or at least 8 and up to 18 bases. In a preferred embodiment, the sequence of the primers for amplifying the CpG-containing nucleic acid hybridize with CpG-free sites to ensure methylation-independent amplification, i.e. the primers are flanking the CpG sites, one upstream and the other downstream of the CpG site(s) of interest. In a preferred embodiment according to any one of the embodiments of the previous six paragraphs, the primers comprise a sequence selected from SEQ ID NO 16 and 17 for amplifying BCAT1, SEQ ID NO 19 and 20 for amplifying TRIM58, SEQ ID NO 22 and 23 for amplifying ZNF177, and/or SEQ ID NO 25 and 26 for amplifying CDO1 (see Table 7). Preferably the means of detection are also primers, more preferably said primers comprise a sequence selected from SEQ ID NO 18 (for detecting methylated BCAT1), 21 (for detecting methylated TRIM58), 24 (for detecting methylated ZNF177) and/or 27 (for detecting methylated CDO1), depending on which methylated gene(s) is/are to be detected (see Table 7).

Table 7.- Primer sequences

| Target ID | CpG | PRIMER SEQUENCE F:FORWARD; R:REVERSE; S:SEQUENCING | LENGTH (bp) |
|---|---|---|---|
| BCAT1 | cg20399616 | F [btn]GAGGTTTTTTTTTAAGGGATGTTGGA (SEQ ID NO 16) | 279 |
| | | R TCCAATCCTCCCCCCTTC (SEQ ID NO 17) | |
| | | S AACTAACCATAAAAAAACTAC (SEQ ID NO 18) | |

(continued)

| Target ID | CpG | PRIMER SEQUENCE F:FORWARD; R:REVERSE; S:SEQUENCING | LENGTH (bp) |
|---|---|---|---|
| TRIM58 | cg23054189 | F TGTTYGGTGTGTTTGGATTTTTTGAG (SEQ ID NO 19) | 201 |
| | | R [btn]CACRCTCTCCACCAAACCC (SEQ ID NO 20) | |
| | | S ATAGTTTTTGTTTTAGGT (SEQ ID NO 21) | |
| ZNF177 | cg08065231 | F AATGTGYGAGTTGGGTAGTTTATTTTT (SEQ ID NO 22) | 122 |
| | | R [btn]CTACTAAAACAACAACCCTTTCTCAA (SEQ ID NO 23) | |
| | | S AGTTTATTTTTTTTTAGTTGTTGG (SEQ ID NO 24) | |
| CDO1 | cg11036833 | F GTTAAAGTGGGGGAGAGATT (SEQ ID NO 25) | 237 |
| | | R [btn]TCATCCTCCCCAARCCCTTTTAAAC (SEQ ID NO 26) | |
| | | S GGGTTTTTGGGAAGG (SEQ ID NO 27) | |

Suitable kits may include primers for amplification and/or means for detection, and various reagents for use in accordance with the present invention in suitable containers and packaging materials, including tubes, vials, and shrink-wrapped and blow-moulded packages. In one embodiment, the kit includes reagents for amplifying and detecting methylation. Optionally, the kit includes sample preparation reagents and/or articles (e.g. tubes) to extract nucleic acids from samples. Additionally, the kits of the invention can contain instructions for the simultaneous, sequential or separate use of the different components which are in the kit.

The method, the biomarker and the kit according to the present invention make it possible to diagnose lung cancer at an early stage in an accurate and rapid manner compared to conventional methods. Thus, a fourth aspect of the invention refers to the use of a method according to any one of the embodiments of the first aspect of the invention described above, for *in vitro* lung cancer diagnosis. The fourth aspect of the invention also refers to the use of a biomarker according to any one of the embodiments of the second aspect of the invention, for *in vitro* lung cancer diagnosis. The fourth aspect of the invention also refers to the use of a biomarker for *in vitro* lung cancer diagnosis, wherein the biomarker is a biomarker according to any one of the embodiments of the second aspect of the invention. The fourth aspect of the invention also refers to the use of a kit according to any one of the embodiments of the third aspect of the invention, for *in vitro* lung cancer diagnosis. Preferably the use of the fourth aspect of the invention is an *in vitro* use. More preferably, the use is for *in vitro* diagnosis of lung cancer in early stage.

Terms used in context of the aspects second to fourth, have the meaning as defined in the first aspect of the present invention.

All publications mentioned herein are hereby incorporated in their entirety by reference. While the foregoing invention has been described in some detail for purposes of clarity and understanding, it will be appreciated by one skilled in the art from a reading of this disclosure that various changes in form and detail can be made without departing from the true scope of the invention and appended claims.

The examples below serve to further illustrate the invention, and to provide those of ordinary skill in the art with a complete disclosure and description of how the methods and uses herein are carried out, and are not intended to limit the scope of the present invention.

EXAMPLES

**Example 1. Samples, procedure, statistics**

Samples, cohorts

Methylation of the biomarkers was conducted by pyrosequencing in four independent cohorts. Lung cohorts were obtained from different institutions in Spain. i) A total of 201 FFPE samples were obtained from Health Institute Carlos III (ISCIII), Madrid and Centre for Applied Medical Research/ Hospital of the University of Navarre, (CIMA/CUN) Pamplona. Regarding minimally invasive samples, ii) 80 BAS and iv) 98 sputums were obtained from Catalan Institute of Oncology and Bellvitge University Hospital, Barcelona. iii) 111 BAL came from CIMA, Pamplona and Hospital of Talavera de la Reina, Talavera de la Reina.

All DNA extractions from different specimens were developed and run by the same technicians to avoid interlaboratory variation. The study was approved by the corresponding institutional review board and patients signed up the informed consent to participate.

The TCGA (lung adenocarcinoma LUAD or Lung squamous cell carcinoma LUSC) cohort was previously described in Sandoval et al. J Clin Oncol 2013; 31:4140-7. The main clinic-characteristics of the different cohorts are described in Table 8.

Table 8.- Clinical characteristics of TCGA (A), Paraffin (B), BAS (C), BALs (D) and Sputum (E) cohorts.

| PATIENT | A. TCGA cohort | | B. FFPE cohort | |
|---|---|---|---|---|
| | Tumor (n=350) | Non-Tumoral (n=62) | Tumor (n=122) | Non-tumoral (n=79) |
| Age (years) | 66.9 (33-90) | 66.9 (40-86) | 63.8 (40-80) | 62.5 (42-85) |
| Gender | | | | |
| Male | 190 (54.1%) | 36 (58.0%) | 108 (88.5%) | 66 (83.5%) |
| Female | 160 (45.9%) | 26 (42.0%) | 14 (11.5%) | 13 (16.5%) |
| Smoking history | | | | |
| Current or former smoker | 313 (89.4%) | 55 (89.4%) | 70 (57.4%) | 71 (89.9%) |
| Nonsmoker | 32 (9.1%) | 2 (9.1%) | 36 (29.5%) | 8 (10.1%) |
| Unknown | 5 (1.5%) | 5 (1.5%) | 16 (13.1%) | 0 (0%) |
| Stage | | | | |
| I | 350 (100%) | | 122 (100%) | |
| Histology | | | | |
| ADC | 217 (62.1%) | | 62 (50.8%) | |
| SCC | 133 (37.9%) | | 60 (49.2%) | |
| Pack-years | 46.7 (1-94.5) | 46.4 (5-192) | 35.9 (0-130) | 46.7 (0-141) |
| Data are average (range) or number (%). | | | | |

Table 8 (cont.)

| PATIENTS | C. BAS COHORT Lung cancer patient (n=51) | Cancer-free donor (n=29) | D. BALs COHORT Lung cancer patient (n=82) | Cancer-free donor (n=29) | E. SPUTUM COHORT Lung cancer patient (n=72) | Cancer-free donor (n=26) |
|---|---|---|---|---|---|---|
| Age (years) | 65.6 (47-85) | 64.0 (35-87) | 62.1 (38-83) | 57.5 (30-82) | 65.1 (40-83) | 52.7 (29-69) |
| Gender | | | | | | |
| Male | 46 (90.2%) | 16 (55.2%) | 66 (80.4%) | 19 (65.5%) | 62 (86.1%) | 17 (65.4%) |
| Female | 5 (9.8%) | 10 (34.5%) | 16 (19.6%) | 9 (31.0%) | 7 (9.7%) | 9 (34.6%) |
| Unknown | 0 (0%) | 3 (10.3%) | 0 (0%) | 1 (3.5%) | 3 (4.2%) | |
| Smoking history | | | | | | |
| Current or former smoker | 45 (88.2%) | 16 (55.2%) | 42 (51.2%) | 15 (51.7%) | 62 (86.1%) | 20 (76.9%) |
| Nonsmoker | 4 (7.8%) | 8 (27.6%) | 39 (47.5%) | 12 (41.3%) | 7 (9.7%) | 6 (23.1%) |
| Unknown | 2 (4.0%) | 5 (17.2%) | 1 (1.3%) | 2 (7%) | 3 (4.2%) | 0 (0%) |
| Stage | | | | | | |
| I | 5 (9.8%) | | 17 (20.7%) | | 12 (16.7%) | |
| II | 5 (9.8%) | | 8 (9.8%) | | 12 (16.7%) | |
| III | 17 (33.4%) | | 20 (24.4%) | | 18 (25.0%) | |
| IV | 17 (33.3%) | | 18 (22.0%) | | 19 (26.4%) | |
| Unknown | 7 (13.7%) | | 19 (23.1%) | | 11 (15.2%) | |
| Histology | | | | | | |
| Adenocarcinoma | 17 (33.3%) | | 25 (30.5%) | | 38 (52.7%) | |
| Squamous cell carcinoma | 19 (37.3%) | | 40 (48.8%) | | 24 (33.3%) | |
| Large cell carcinoma | 2 (4.0%) | | 2 (2.4%) | | 2 (3%) | |
| Small cell carcinoma | 2 (4.0%) | | 12 (14.6%) | | 5 (7%) | |
| NSCLC (NOS) | 11 (21.4%) | | 3 (3.7%) | | 3 (4%) | |
| Pack-years | 49.6 (0-120) | 32.4 (0-100) | 45.5 (0-120) | 26.3 (0-90) | 49.1 (0-120) | 24.1 (0-114) |

Data are average (range) or number (%) NSCLC (NOS): Non-small cell lung cancer. Not otherwise specified

Preparation of lung specimens

DNA was extracted from minimally and non-invasive specimens using a standard phenol chloroform extraction method. DNA from FFPE tissue blocks was extracted from two sequential unstained sections, each 10 μm thick. For each sample of tumor tissue, subsequent sections were stained with hematoxylin and eosin for histological confirmation of the presence (>50%) of tumor cells. Unstained tissue sections were deparaffinized, and DNA was extracted using the same protocol as for minimally invasive specimens. Extracted DNA was checked for integrity and quantity with 1.3% agarose gel electrophoresis and picogreen quantification, respectively. Bisulfite conversion of 500 ng of DNA for each sample was performed using the EZ DNA Methylation Gold (ZYMO RESEARCH) bisulfite conversion kit according to the manufacturer's recommendation.

Pyrosequencing

Pyrosequencing analyses to determine CpG methylation level were developed as previously described (Sandoval J. et al., A prognostic DNA methylation signature for stage I non-small-cell lung cancer. J Clin Oncol 2013;31:4140-7). Briefly, a set of primers for PCR amplification and sequencing were designed using a specific software pack (PyroMark assay design version 2.0.01.15). Primer sequences were designed to hybridize with CpG-free sites to ensure methylation-independent amplification (see Table 7). DNA was converted using the EZ DNA Methylation Gold (ZYMO RESEARCH) bisulfite conversion kit following the manufacturer's recommendations and used as a template for subsequent PCR step. PCR was performed under standard conditions with primers biotinylated ([btn]) to convert the PCR product to single-stranded DNA templates. We used the Vacuum Prep Tool (Biotage, Sweden) to prepare single-stranded PCR products according to manufacturer's instructions. PCR products were observed at 2% agarose gels before pyrosequencing. Pyrosequencing reactions and methylation quantification were performed in a PyroMark Q24 System version 2.0.6 (Qiagen) using appropriate reagents and protocols, and the methylation value was obtained from the average of the CpG dinucleotides included in the sequence analyzed, with a minimum of 3 valid CpGs per primer. Only those average methylation values within the region analyzed with coefficient of variation lower than 1 were accepted as valid. Controls to assess correct bisulfite conversion of the DNA were included in each run, as well as sequencing controls to ensure the fidelity of the measurements.

Statistical analysis

Data were summarized by mean, standard deviation, median and first and third quartiles in the case of continuous variables and by relative and absolute frequencies in the case of categorical variables. Differences in expression values and methylation levels among groups were assessed using the non-parametric Wilcoxon rank sum test. Receiver Operating Characteristic (ROC) curves were used to assess the predictive capacity of each marker. Area under the curve (AUC) was computed for each ROC curve, and 95% confidence intervals (CI) were also estimated by bootstrapping with 1000 iterations. A predictive model for each sample type was built including all selected markers in a multivariable logistic regression model. ROC curves and AUC were also computed for the predictive models. Internal validation of the models was performed using 10-fold crossvalidation. The final predictive models were represented in nomograms to facilitate their use by clinicians. Sensitivity and specificity were estimated at the optimal cut-off point according to Youden's criterion. Additionally, the sensitivity and specificity curves were estimated for the whole range of predictions of the model to allow for personalized decisions in different clinical scenarios. Globally, a two-tailed p-value of less than 0.05 was considered to indicate statistical significance. P-values were adjusted for multiple comparisons using the FDR procedure by Benjamini and Hochberg. All statistical analyses were performed using R software (version 3.2.0) and the pROC R-package (version 1.7.3).

The inventors generated a mathematical algorithm to calculate the probability of cancer for any sample type and gene combination. The general form of said algorithm was (formula I):

$$\Pr(Cancer) = \frac{e^{a+b*TRIM58+c*ZNF177+d*CDO1+e*BCAT1}}{1+e^{a+b*TRIM58+c*ZNF177+d*CDO1+e*BCAT1}} * 100$$

Where the coefficients a, b, c, d and e take the log-odd values of cancer estimated by a multivariable logistic regression model adjusted using maximum likelihood to methylation values data from a specific sample type and a specific combination of the four genes.

**Example 2.- Early lung cancer detection in minimally-invasive respiratory samples: BAS**

One of the most important aspects for early diagnostics is to identify markers associated with cancer using non-invasive or minimally-invasive methods for sample collection. In line, the inventors collected an independent cohort of BAS from patients diagnosed with lung cancer (n= 51) and cancer-free patients (n= 29) (Table 8). This cohort included different lung cancer subtypes, especially ADC and SCC. The inventors compared by pyrosequencing the median methylation levels and generated ROC curves to assess the performance of each marker independently. Airways fluids from lung cancer patients presented significant differences in DNA methylation levels (Fig. 1A-1 D) and high AUCs for all four genes (Fig. 1 E-1 H).

The inventors analysed the AUC of different combinations of *BCAT1, CDO1*, *TRIM58* and *ZNF177* in a logistic regression model yielding significant AUCs higher than or equal than 0.73 in BAS samples (see Table 9). Thus, any of these combinations, and in particular the ones with higher AUC, may be of high value to detect lung cancer in BAS samples.

Table 9.- AUC of single genes and different combinations thereof in BAS, BAL and sputum samples.

| Gene | AUC - BAS | AUC - BAL | AUC - ESPUT |
|---|---|---|---|
| BCAT1 | 0.76 | 0.8 | 0.92 |
| TRIM58 | 0.8 | 0.72 | 0.67 |
| ZNF177 | 0.76 | 0.66 | 0.69 |
| CDO1 | 0.73 | 0.65 | 0.67 |
| BCAT1 + TRIM58 | 0.87 | 0.85 | 0.92 |
| BCAT1 + ZNF177 | 0.86 | 0.84 | 0.92 |
| BCAT1 + CDO1 | 0.82 | 0.79 | 0.92 |
| TRIM58 + ZNF177 | 0.86 | 0.73 | 0.68 |
| TRIM58 + CDO1 | 0.83 | 0.72 | 0.74 |
| ZNF177 + CDO1 | 0.83 | 0.73 | 0.76 |
| BCAT1 + TRIM58 + ZNF177 | 0.9 | 0.86 | 0.92 |
| BCAT1 + TRIM58 + CDO1 | 0.88 | 0.84 | 0.92 |
| BCAT1 + ZNF177 + CDO1 | 0.88 | 0.83 | 0.92 |
| TRIM58 + ZNF177 + CDO1 | 0.88 | 0.74 | 0.76 |
| BCAT1 + TRIM58 + ZNF177 + CDO1 | 0.91 | 0.85 | 0.93 |

Combination of *BCAT1, CDO1*, *TRIM58* and *ZNF177* in a logistic regression model yielded a significant AUC of 0.91 (95% CI [0.83, 0.98] p<0.001, Fig. 1I). Calibration of the model showed no evident deviations from the ideal identity slope (data not shown). Internal validation of the AUC estimate for this model yielded optimism corrected AUC of 0.90, showing high generalization of the predictive capacity of the model for future samples. A nomogram based on the results of this model is proposed as a predictive tool for clinical diagnostic use. Results of the nomogram provide an individual probability (0%-100%) for suffering lung cancer for each patient (Fig. 2). Evaluation of the full range of predictions of the model shows that shifting the cut-off to POC=30% would yield a sensitivity of 100% and a specificity of 65.4% and shifting the cut-off to POC=80% would yield a sensitivity of 71.4% and a specificity of 92.3%. Sensitivity and specificity at the optimal cut-off (POC= 63%) were 84.6% and 81.0% respectively (Fig. 1J).

It is important to point out that current protocols for lung cancer diagnosis are based mainly in bronchioalveolar cytology and further lung biopsy. There are cases where the cytology is doubtful or inconclusive. Moreover, there are a notable number of cases where cytology and biopsy are negative for cancer cells, but there is high suspicion of cancer. The present results not only improve the overall prediction accuracy of BAS cytology in this cohort (sensitivity=43.8%, specificity=100%), but also permit a flexible and personalized approach for the clinicians in every possible scenario by simply adapting the cut-off value of the probabilistic model. In this sense, in the studied cohort 24 of 51 tumor samples were misinterpreted as non-tumoral by the cytology test. However, using the predictive four-gene epigenetic signature of the present invention, 19 out of the 24 false negative cytologies (79%) would have been considered as positive setting the threshold at 50% probability of cancer (Table 10).

Table 10.- Probability of cancer

| Model prediction (Probability of Cancer) | N° patients |
|---|---|
| 0% - 20% | 0 |
| 20% - 30% | 1 |
| 30% - 40% | 1 |

(continued)

| Model prediction (Probability of Cancer) | N° patients |
|---|---|
| 40% - 50% | 3 |
| 50% - 60% | 0 |
| 60% - 70% | 1 |
| 70% - 80% | 2 |
| 80% - 100% | 16 |

Of note, the majority of them (16 of 24) with a predicted probability of cancer higher than 80%. Also three of them were classified as borderline non-tumor, with a predicted probability of cancer between 40% and 50%. In these three doubtful cases, clinical patient manage would require further additional studies. Thus, the epigenetic signatures described in the present invention, in particular the four-gene epigenetic signature, is a useful clinical diagnostic tool in BAS specimens, especially in doubtful cases.

The concrete values for the coefficients of the general algorithm indicated in Example 1 (formula I) are specified below for particular gene-combinations in the BAS samples. Four-gene signature: Combination of BCAT1, TRIM58, ZNF177 and CDO1 (formula V)

$$\Pr(Cancer)_{BAS} = \frac{e^{-6.13+0.18*TRIM58+0.30*ZNF177+0.47*CDO1+0.59*BCAT1}}{1+e^{-6.13+0.18*TRIM58+0.30*ZNF177+0.47*CDO1+0.59*BCAT1}} * 100$$

That is, the coefficients are: a = -6.13, b = 0.18, c = 0.30, d = 0.47, e = 0.59.

Three-gene signature: Combination of BCAT1, TRIM58 and ZNF177 (formula IV)

a = -5.15 b = 0.20 c = 0.32 d = 0 e = 0.66

$$\Pr(Cancer)_{BAS} = \frac{e^{-5.15+0.20*TRIM58+0.32*ZNF177+0.66*BCAT1}}{1+e^{-5.15+0.20*TRIM58+0.32*ZNF177+0.66*BCAT1}} * 100$$

That is, the coefficients are: a = -5.15, b = 0.20, c = 0.32, d = 0, e = 0.66.

Two-gene signature: Combination of BCAT1 and TRIM58 (formula III)

$$\Pr(Cancer)_{BAS} = \frac{e^{-3.03+0.24*TRIM58+0.55*BCAT1}}{1+e^{-3.03+0.24*TRIM58+0.55*BCAT1}} * 100$$

That is, the coefficients are: a = -3.03, b = 0.24, c = 0, d = 0, e = 0.55.

One-gene signature: BCAT1 gene (formula II)

$$\Pr(Cancer)_{BAS} = \frac{e^{-1.86+0.63*BCAT1}}{1+e^{-1.86+0.63*BCAT1}} * 100$$

That is, the coefficients are: a = -1.86, b = 0, c = 0, d = 0, e = 0.63.

**Example 3.- Early lung cancer detection in minimally-invasive respiratory samples: BAL**

Additionally, the inventors evaluated DNA methylation levels in BAL from patients with lung cancer (n=82) as compared to non-malignant lung diseases (n=29) (Table 8).

The methylation levels of the four markers individually were significantly higher in BAL fluid from cancer patients than non-cancer patients (Fig. 3A-3D). AUCs were significant for all four genes with the following values $AUC_{BCAT1}$=0.80, $AUC_{CDO1}$=0.65, $AUC_{TRIM58}$=0.72 and $AUC_{ZNF177}$=0.66 (Fig. 3E-3H). Combination of the four genes in a logistic regression model achieved a significant AUC of 0.85 (95% CI [0.78, 0.93] p < 0.001) (Fig. 3I), with an optimism-corrected value of 0.83, confirming that the model is valid. Evaluation of the full range of predictions of the model is also shown (Fig. 3J). The AUC of different combinations of *BCAT1, CDO1*, *TRIM58* and *ZNF177* in a logistic regression model yielding significant AUCs higher than or equal to 0.65 in BAL samples (see Table 9). Thus, as in the case with BAS specimens, the different epigenetic signatures of the present invention, in particular the four-gene epigenetic signature, may be of high value to detect lung cancer in BAL samples and may be highly valuable for doubtful patients with negative cytology.

**Example 4.- Early lung cancer detection in non-invasive sputum samples**

There are many reports that the DNA hypermethylation of various genes can also be detected in the sputum of lung cancer patients. Thus, the methylation level of these 4 markers was examined in sputums samples from 72 lung cancer patients and 26 cancer-free individuals (Table 8). Methylation levels were significantly higher in individuals with lung cancer for all the genes tested, except for CDO1 (Fig. 4A-4D). Individual AUC values were $AUC_{BCAT1}$=0.92, $AUC_{CDO1}$=0.67, $AUC_{TRIM58}$=0.67 and $AUC_{ZNF177}$=0.69 (Fig. 4E-4H). The combination of logistic regression model yielded an AUC value of 0.93 (95% CI [0.86, 1.0], p<0.001) (Fig. 4I). Sensitivity and specificity for the different threshold values of the model are depicted (Fig. 4J). The AUC of different combinations of *BCAT1, CDO1*, *TRIM58* and *ZNF177* in a logistic regression model yielding significant AUCs higher than or equal to 0.67 in sputum samples (see Table 9). Thus, as in the case with BAS and BAL specimens, the different epigenetic signatures of the present invention, in particular the 4-gene epigenetic signature, may be of high value to detect lung cancer in sputum samples and may be highly valuable for doubtful patients with negative cytology.

**Example 5.- Early lung cancer detection in primary tumors: FFPE**

An independent cohort of FFPE primary tumors (122 stage I NSCLC and 79 non-lung cancer samples was recruited and DNA methylation levels for BCAT1, CDO1, TRIM55 and ZNF177 were determined by pyrosequencing. Clinical characteristics for this cohort are described in Table 8. The four biomarkers had significantly higher levels of DNA methylation in tumor samples as compared to non-tumoral controls (Fig. 5A-5D). Importantly, all the genes of the signature showed significant areas under the ROC curve (AUC) greater than 0.8 ($AUC_{BCAT1}$=0.94, $AUC_{CDO1}$=0.84, $AUC_{TRIM58}$=0.98 and $AUC_{ZNF177}$=0.96), suggesting a great diagnostic accuracy of these biomarkers for NSCLC detection (Fig. 5E-5H). Similarly, when samples were classified based on histological subtypes (ADC and SCC), the inventors observed for all the biomarkers significant differences in methylation level (data not shown) and AUCs close to 1.0 ($AUC_{BCAT1}$=0.94 (95% CI [0.91, 0.98] p<0.001); $AUC_{CDO1}$=0.87 (95% CI [0.79, 0.94] p<0.001); $AUC_{TRIM55}$=0.95 (95% CI [0.92, 0.99] p<0.001); $AUC_{ZNF177}$=0.95 (95% CI [0.87,0.98] p<0.001)forADC; $AUC_{BCAT1}$=0.94 (95% CI [0.91, 0.98] p<0.001); $AUC_{CDO1}$=0.81 (95% CI [0.72, 0.90] p<0.001); $AUC_{TRIM55}$=0.99 (95% CI [0.97, 1.0] p<0.001); $AUC_{ZNF177}$=0.99 (95% CI [0.91, 0.99] p<0.001) for SCC). Results from the four-gene epigenetic signature presented high diagnostic accuracy and were extremely similar to those obtained from public databases (data not shown) and FFPE samples, as shown in this Example. Importantly, the inventors analyzed a total of 79 non-tumoral control tissues, and DNA methylation was almost negligible in the vast amount of samples. These results confirmed the diagnostic value of evaluating DNA methylation levels by the method of the present invention, since even when minimally or non-invasive samples were used, in which the number of cancer cells is lower than in tissue samples, the results were the same as when using FFPE samples.

**Example 6.- Epigenetic silencing of the cancer-specific methylated genes in lung cancer primary tumors**

Promoter hypermethylation of multiple consecutive CpGs is recognized as an important mechanism by which genes may be silenced in both physiologically and pathological conditions. This mechanism for gene silencing has also been shown to play a relevant functional role in the development and progression of many common human tumors. In this regard, analyzing the CURELUNG FP7 publicly available dataset and TCGA (lung adenocarcinoma LUAD or Lung squamous cell carcinoma LUSC) datasets (Sandoval et al. J Clin Oncol 2013; 31:4140-7), the inventors observed a similar methylation pattern between the significant differentially DNA methylated CpGs (DMCpGs) of the selected biomarkers and their surrounding CpGs (Fig 6). Importantly, gene expression analysis from the TCGA cohort samples showed a significantly decreased expression in *BCAT1, CDO1*, *TRIM58* and *ZNF177* (Fig. 7). Interestingly, expression results were also obtained for ADCs and SCCs separately (Fig. 8). These results reinforced the role of DNA methylation in the functional regulation of *BCAT, CDO1*, *TRIM58* and *ZNF177.* Importantly, the data obtained suggest that the methylation values of these four genes represent an epigenetic signature that may be relevant in early steps of lung carcinogenesis.

## Claims

1. An *in vitro* method for the diagnosis of lung cancer comprising the steps of:

a) determining the methylation level of a gene in a test sample, taken from a subject, wherein the gene is one gene, a two-gene combination, a three-gene combination or a four-gene combination, and wherein the gene(s) is(are) selected from the group consisting of BCAT1, TRIM58, ZNF177 and CDO1, and at least one gene is BCAT1;
b) comparing the methylation level determined in step a) to a reference; and
c) identifying the subject as being likely to have lung cancer, if the methylation level of the test sample is higher than the methylation level of the reference, and identifying the subject as unlikely to have lung cancer if the methylation level of the test sample is below the methylation level of the reference.

2. The method according to claim 1, wherein the gene is a two-gene combination selected from BCAT1 and TRIM58; BCAT1 and CDO1; or BCAT1 and ZNF177.

3. The method according to claim 1, wherein the gene is a three-gene combination selected from BCAT1, TRIM58 and CDO1; BCAT1, TRIM58 and ZNF177; or BCAT1, CDO1 and ZNF177.

4. The method according to claim 1, wherein the gene is a four-gene combination of BCAT1, TRIM58, CDO1 and ZNF177 genes.

5. The method according to any one of the preceding claims, wherein BCAT1 gene comprises any one of SEQ ID 1-3, TRIM58 gene comprises any one of SEQ ID 4-8, CDO1 gene comprises any one of SEQ ID 9-11, and ZNF177 gene comprises any one of SEQ ID 12-15.

6. Method according to any one of the preceding claims, wherein methylation is determined at one or more CpG site(s) and the position of the CpG site(s) is(are) selected from the group consisting of:

- 25054873, 25054905, 25055108, 25055214, 25055262, 25055304, 25055381, 25055421, 25055518, 25055676, 25055938, 25055948, 25055957, 25055959, 25055961, 25055967, 25055978, 25056083, 25056243, 25101448, 25102072, 25102274, 25102311, 25102431, 25102469, 25102521, 25103173 and 25103643 in BCAT1 gene,
- 248019234, 248019757, 248019816, 248020331, 248020350, 248020377, 248020436, 248020632, 248020641, 248020671, 248020680, 248020688, 248020692, 248020695, 248020697, 248020704, 248020707, 248020713, 248020812, 248021091 and 248021163 in TRIM58 gene,
- 115150172, 115151427, 115152019, 115152326, 115152386, 115152413, 115152420, 115152431, 115152485, 115152492, 115152466, 115152468, 115152475, 115152484, 115152494, 115152496, 115152503, 115152509, 115152522, 115152785, 115152835, 115152938 and 115153223 in CDO1 gene, and
- 9472210, 9473058, 9473240, 9473565, 9473598, 9473668, 9473674, 9473684, 9473688, 9473691, 9473696, 9473715, 9473715, 9473781, 9473880 and 9474128 in ZNF177 gene.

7. Method according to any one of claims 1-5, wherein the methylation level is determined at one or more CpG site(s) selected from the group consisting of:

- cg20399616 in BCAT1 gene,
- cg23054189, cg07533148, cg20810478, cg16021909 in TRIM58 gene,
- cg08065231 in ZNF177 gene, and
- cg11036833 in CDO1 gene.

8. Method according to any one of the preceding claims, wherein the methylation level is determined by bisulfite sequencing or by pyrosequencing, and preferably by pyrosequencing.

9. Method according to any one of the preceding claims, wherein the test sample, taken from the subject, is selected from the group consisting of BAS, BAL, blood and sputum, and preferably is BAS.

**10.** A biomarker for *in vitro* lung cancer diagnosis, wherein the biomarker comprises a methylated gene, containing one or more methylated CpG site(s), wherein the gene is selected from one gene, a two-gene combination, a three-gene combination or a four-gene combination, and wherein the gene(s) is(are) selected from the group consisting of BCAT1, TRIM58, ZNF177 and CDO1, and at least one gene is BCAT1.

**11.** A kit to carry out the method according to any one of claims 1-9, comprising:

- primers for amplifying a CpG-containing nucleic acid of a gene, and/or
- means for detecting the presence of methylated CpG site(s) in said amplified nucleic acid,

wherein the gene is selected from one gene, a two-gene combination, a three-gene combination or a four-gene combination, and
wherein the gene(s) is(are) selected from the group consisting of BCAT1, TRIM58, ZNF177 and CDO1, and at least one gene is BCAT1.

**12.** The biomarker according to claim 10 or the kit according to claim 11, wherein BCAT1 gene comprises any one of SEQ ID 1-3, TRIM58 gene comprises any one of SEQ ID 4-8, CDO1 gene comprises any one of SEQ ID 9-11, and ZNF177 gene comprises any one of SEQ ID 12-15.

**13.** The biomarker according to claim 10 or 12, or the kit according to claim 11 or 12, wherein the position of the CpG site(s) is(are) selected from the group consisting of:

- 25054873, 25054905, 25055108, 25055214, 25055262, 25055304, 25055381, 25055421, 25055518, 25055676, 25055938, 25055948, 25055957, 25055959, 25055961, 25055967, 25055978, 25056083, 25056243, 25101448, 25102072, 25102274, 25102311, 25102431, 25102469, 25102521, 25103173 and 25103643 in BCAT1,
- 248019234, 248019757, 248019816, 248020331, 248020350, 248020377, 248020436, 248020632, 248020641, 248020671, 248020680, 248020688, 248020692, 248020695, 248020697, 248020704, 248020707, 248020713, 248020812, 248021091 and 248021163 in TRIM58,
- 115150172, 115151427, 115152019, 115152326, 115152386, 115152413, 115152420, 115152431, 115152485, 115152492, 115152466, 115152468, 115152475, 115152484, 115152494, 115152496, 115152503, 115152509, 115152522, 115152785, 115152835, 115152938 and 115153223 in CDO1, and
- 9472210, 9473058, 9473240, 9473565, 9473598, 9473668, 9473674, 9473684, 9473688, 9473691, 9473696, 9473715, 9473715, 9473781, 9473880 and 9474128 in ZNF177.

**14.** The biomarker according to claim 10 or 12, or the kit according to claim 11 or 12, wherein the one or more CpG site(s) is(are) selected from the group consisting of:

- cg20399616 in BCAT1,
- cg23054189, cg07533148, cg20810478, cg16021909 in TRIM58,
- cg08065231 in ZNF177, and
- cg11036833 in CDO1.

**15.** Use of a method according to any one of claims 1-9, or of a biomarker according to any one of claims 10, 12-14, or of a kit according to any one of claims 11-14, for *in vitro* lung cancer diagnosis.

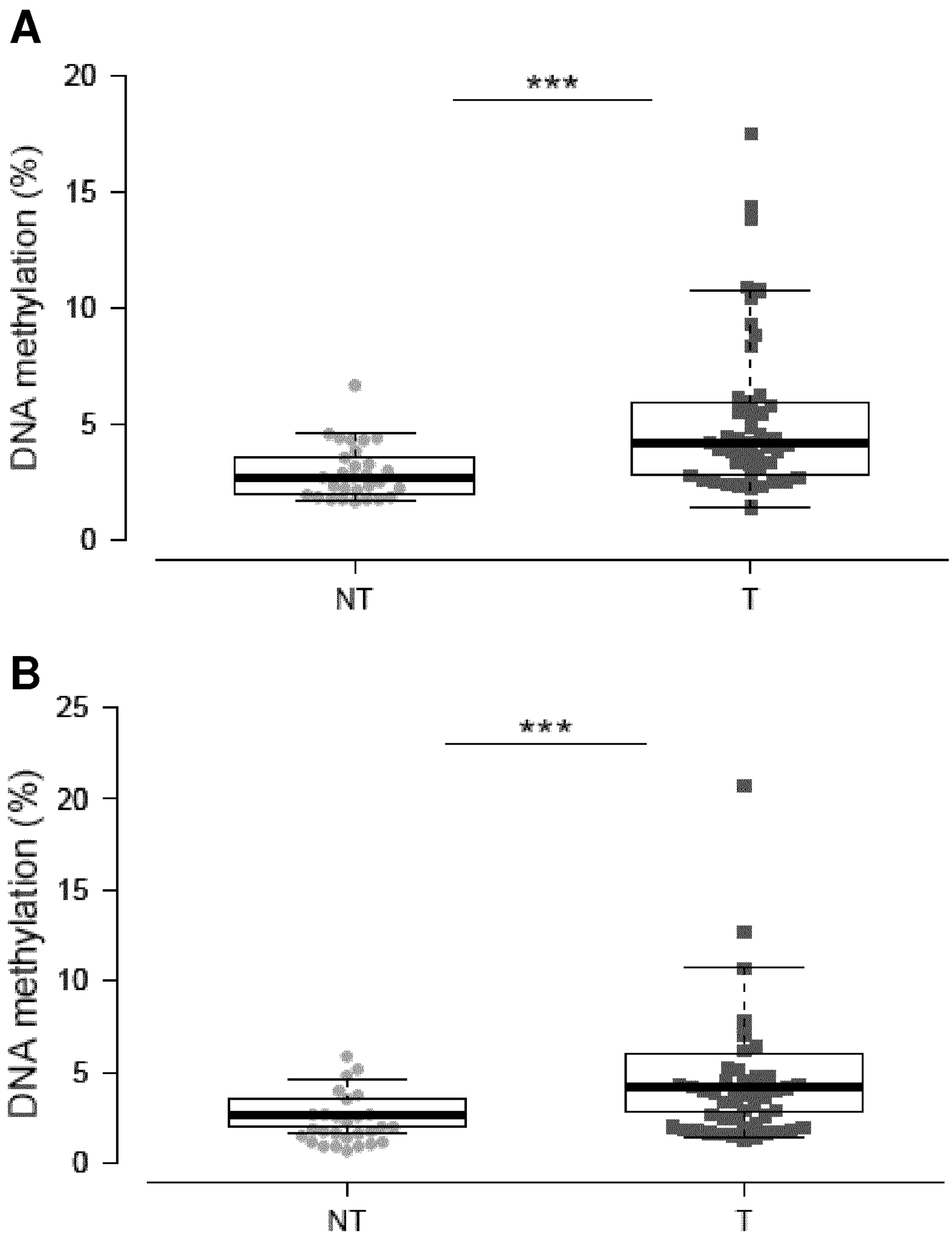
A
DNA methylation (%)
20
15
10
5
0
***
NT
T
B
DNA methylation (%)
25
20
15
10
5
0
***
NT
T

FIG. 1

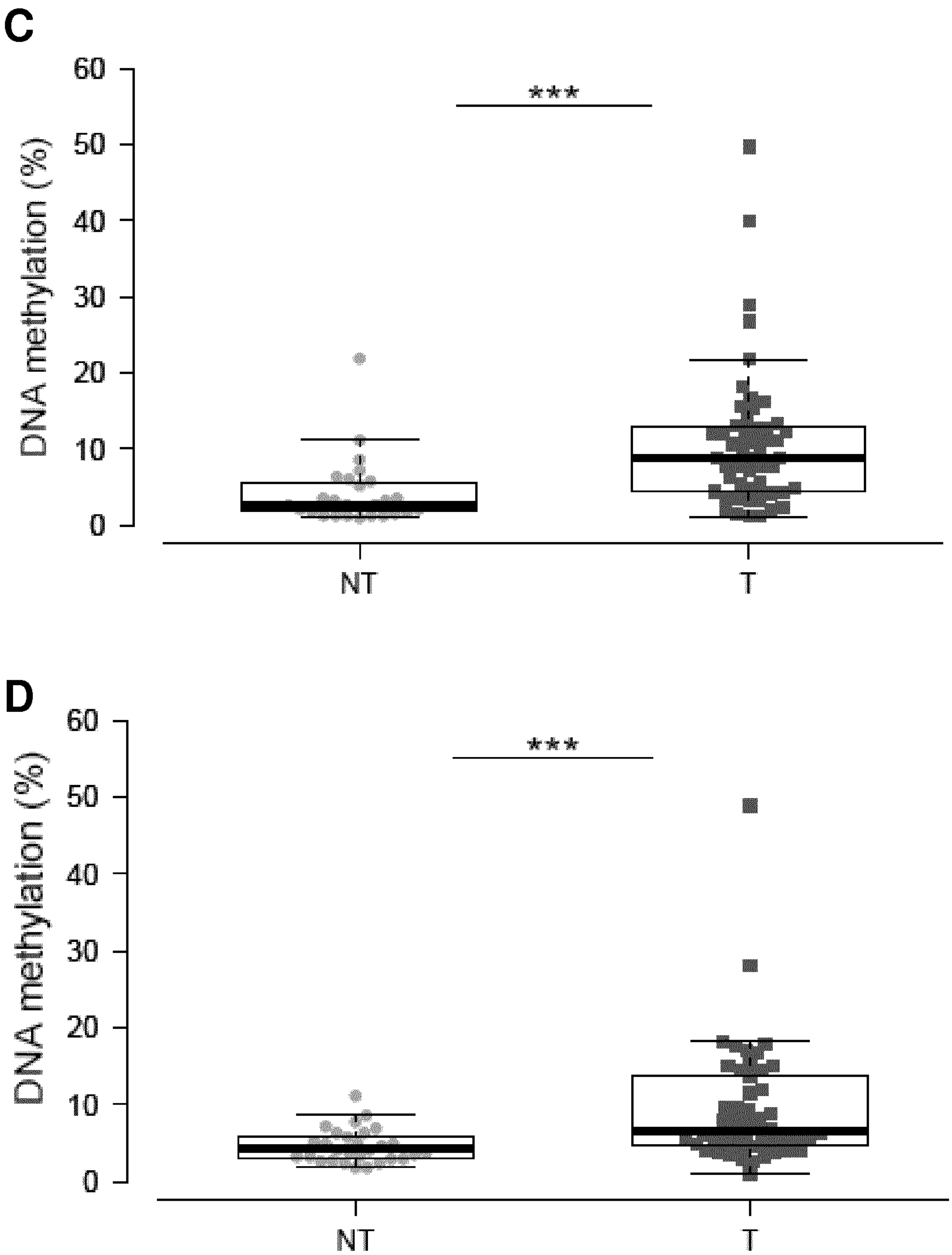
C
60
50
40
30
20
10
0
DNA methylation (%)
***
NT
T
D
60
50
40
30
20
10
0
DNA methylation (%)
***
NT
T

FIG. 1 (cont.)

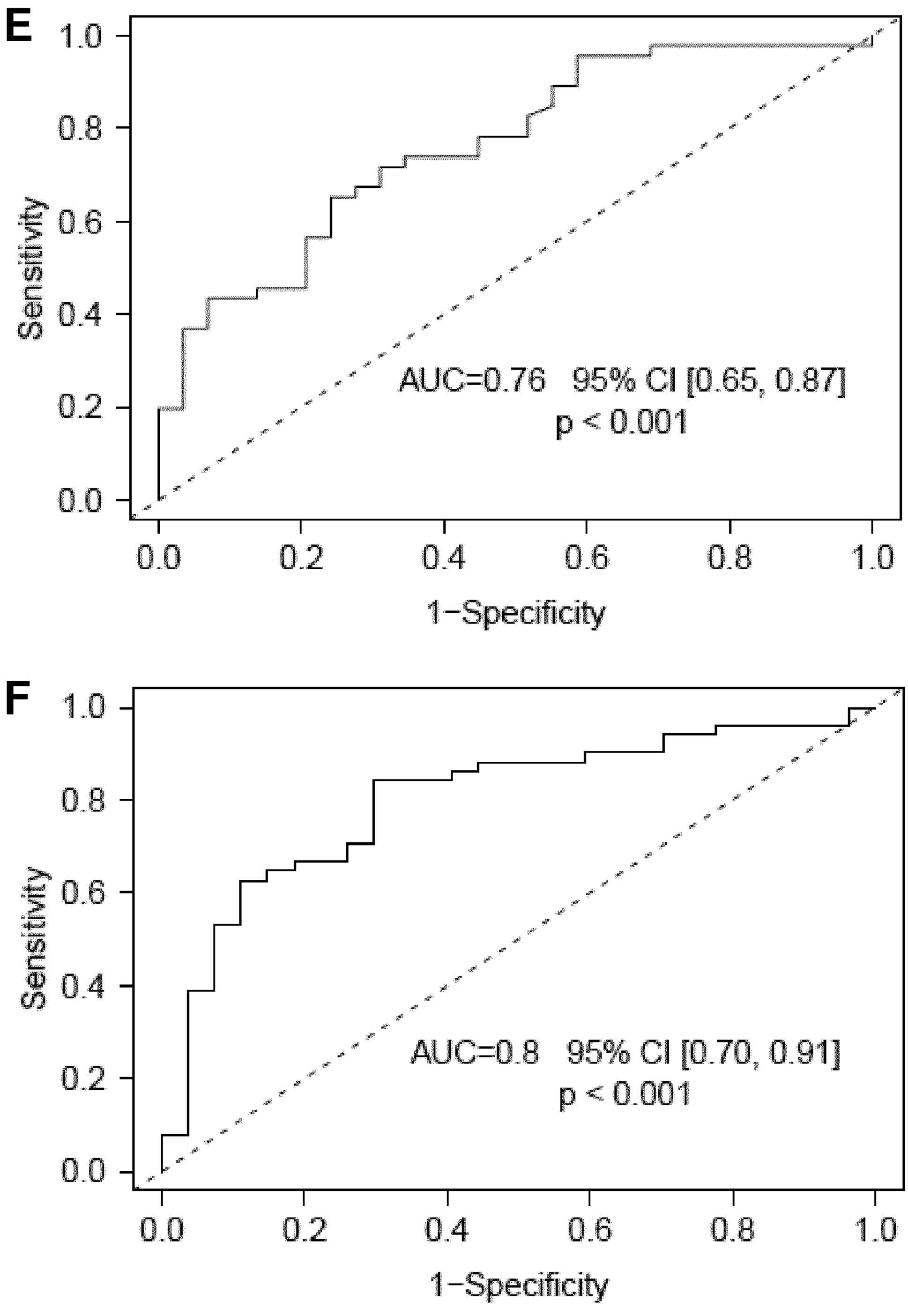
E
1.0
0.8
0.6
0.4
0.2
0.0
Sensitivity
AUC=0.76 95% CI [0.65, 0.87]
p < 0.001
0.0 0.2 0.4 0.6 0.8 1.0
1−Specificity
F
1.0
0.8
0.6
0.4
0.2
0.0
Sensitivity
AUC=0.8 95% CI [0.70, 0.91]
p < 0.001
0.0 0.2 0.4 0.6 0.8 1.0
1−Specificity

FIG. 1 (cont.)

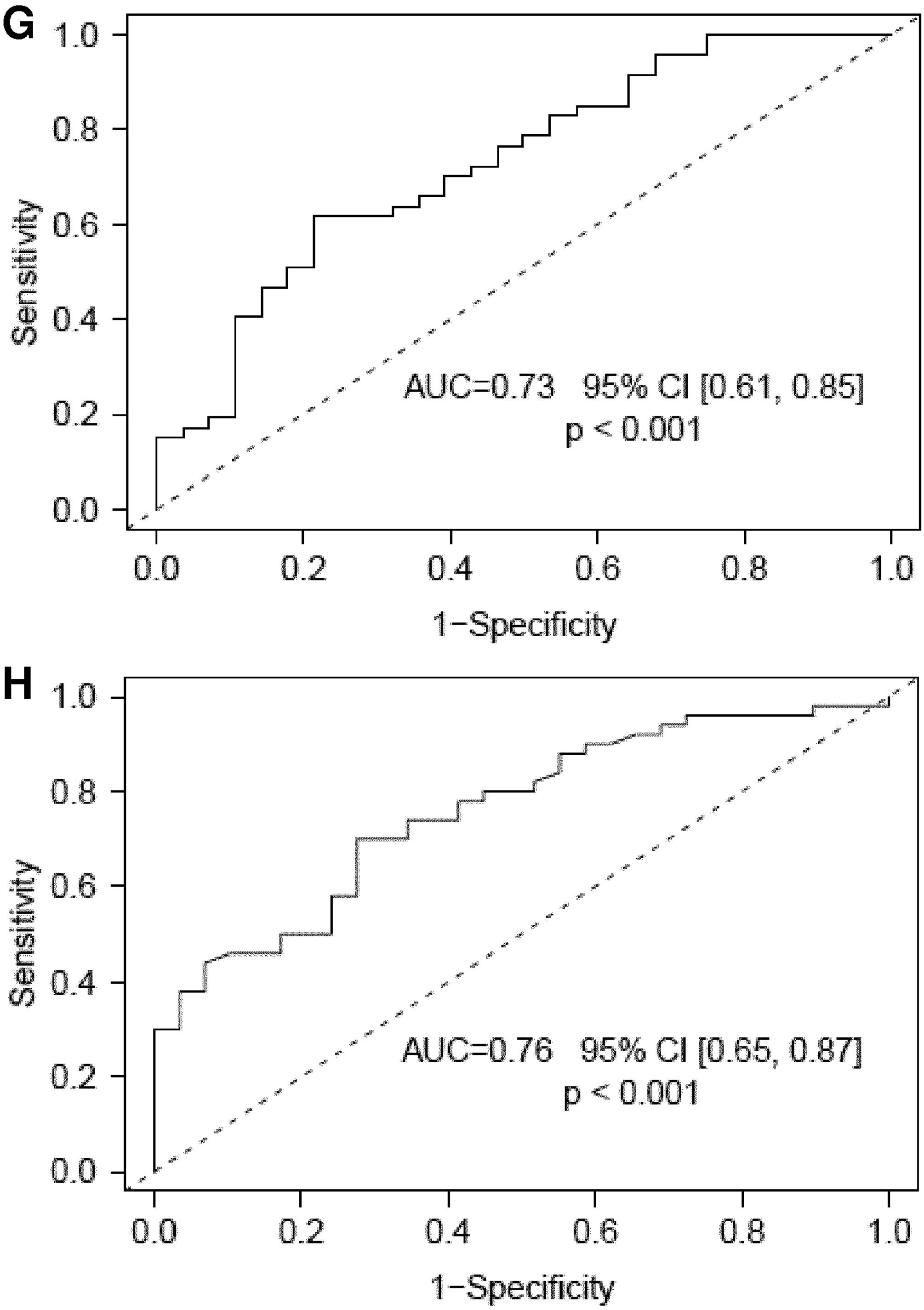
G
1.0
0.8
0.6
0.4
0.2
0.0
Sensitivity
AUC=0.73 95% CI [0.61, 0.85]
p < 0.001
0.0
0.2
0.4
0.6
0.8
1.0
1−Specificity
H
1.0
0.8
0.6
0.4
0.2
0.0
Sensitivity
AUC=0.76 95% CI [0.65, 0.87]
p < 0.001
0.0
0.2
0.4
0.6
0.8
1.0
1−Specificity

FIG. 1 (cont.)

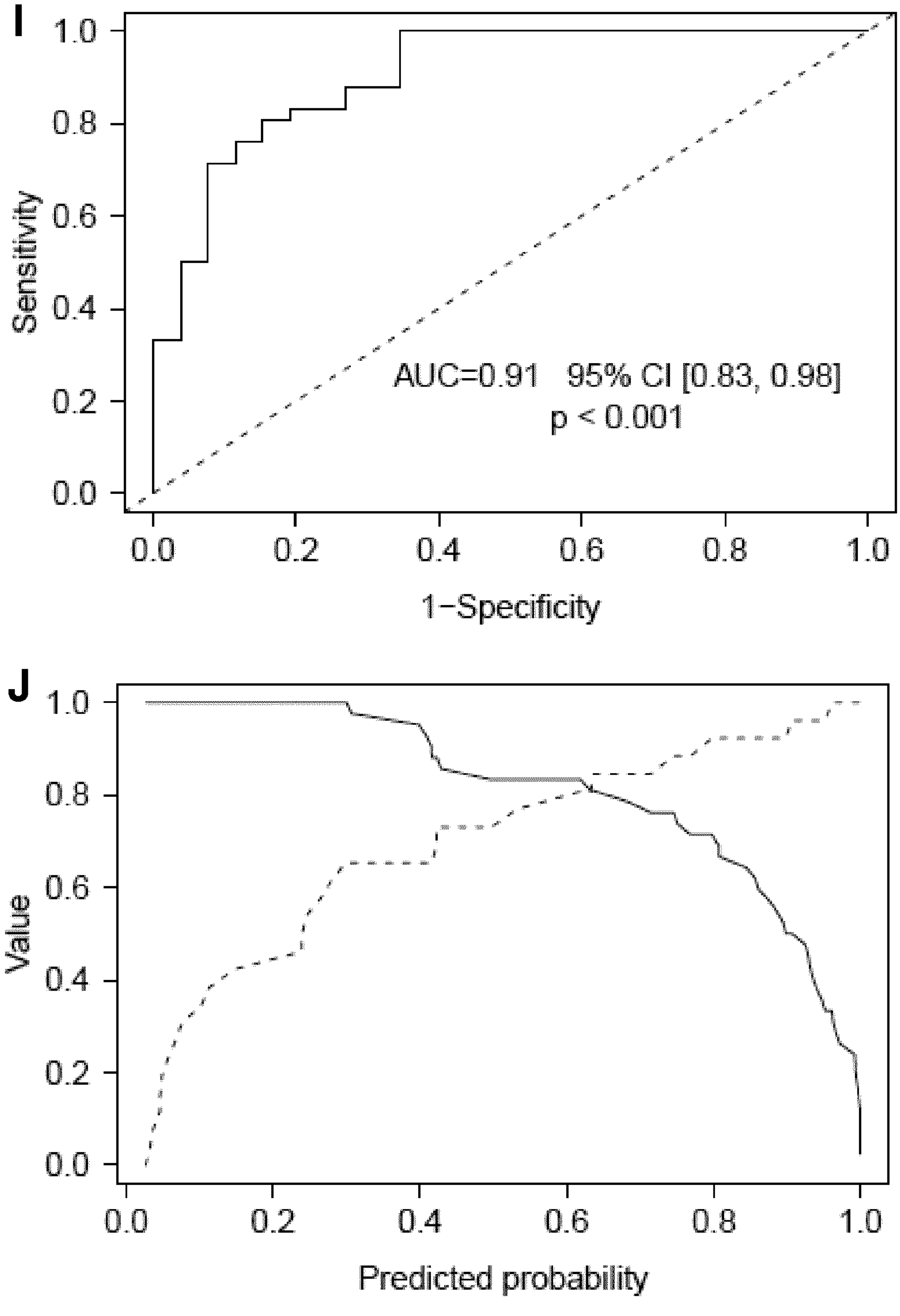
I
1.0
0.8
0.6
0.4
0.2
0.0
Sensitivity
AUC=0.91 95% CI [0.83, 0.98]
p < 0.001
0.0
0.2
0.4
0.6
0.8
1.0
1−Specificity
J
1.0
0.8
0.6
0.4
0.2
0.0
Value
0.0
0.2
0.4
0.6
0.8
1.0
Predicted probability

FIG. 1 (cont.)

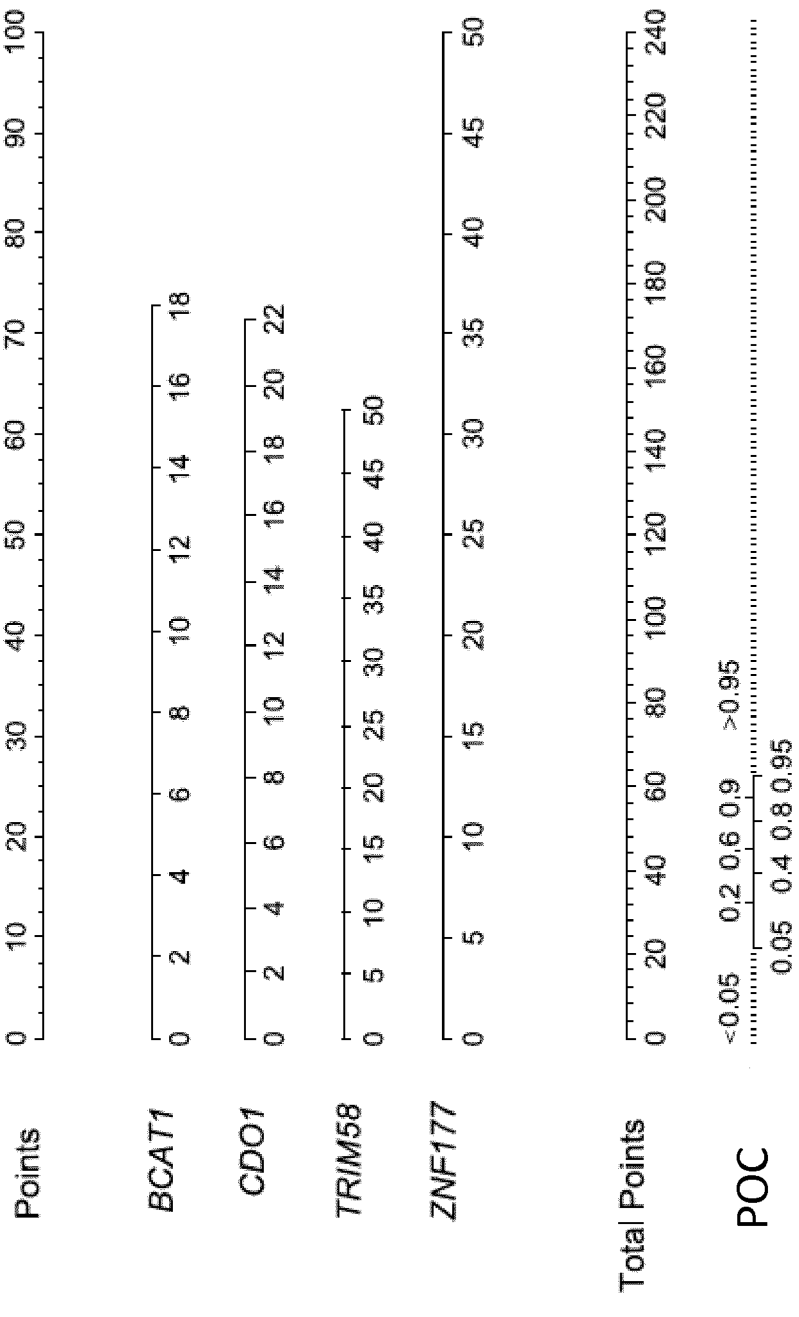

Points
0 10 20 30 40 50 60 70 80 90 100
BCAT1
0 2 4 6 8 10 12 14 16 18
CDO1
0 2 4 6 8 10 12 14 16 18 20 22
TRIM58
0 5 10 15 20 25 30 35 40 45 50
ZNF177
0 5 10 15 20 25 30 35 40 45 50
Total Points
0 20 40 60 80 100 120 140 160 180 200 220 240
POC
<0.05 0.2 0.6 0.9 >0.95
0.05 0.4 0.8 0.95


FIG. 2

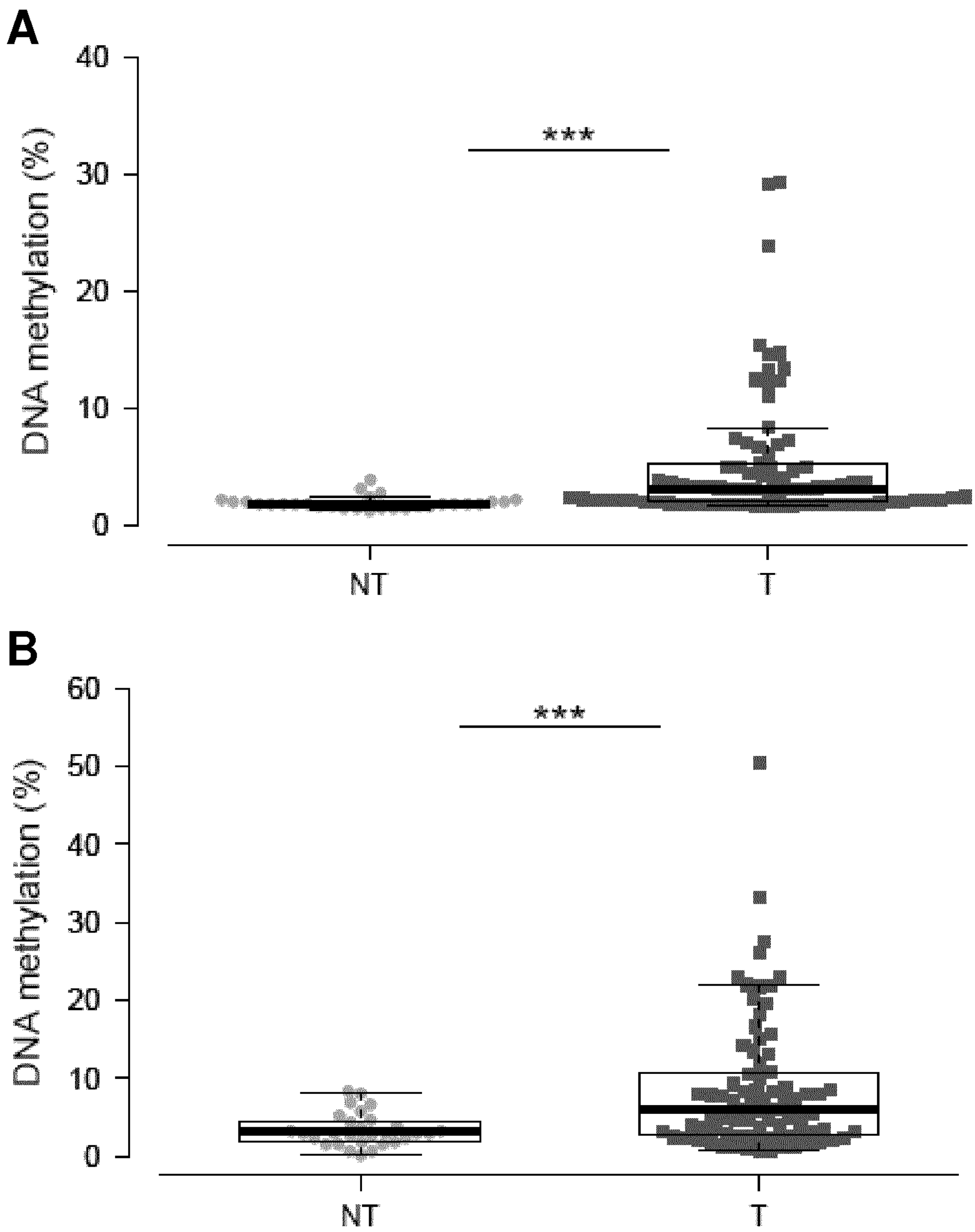
A
40
30
20
10
0
DNA methylation (%)
***
NT
T
B
60
50
40
30
20
10
0
DNA methylation (%)
***
NT
T

FIG. 3

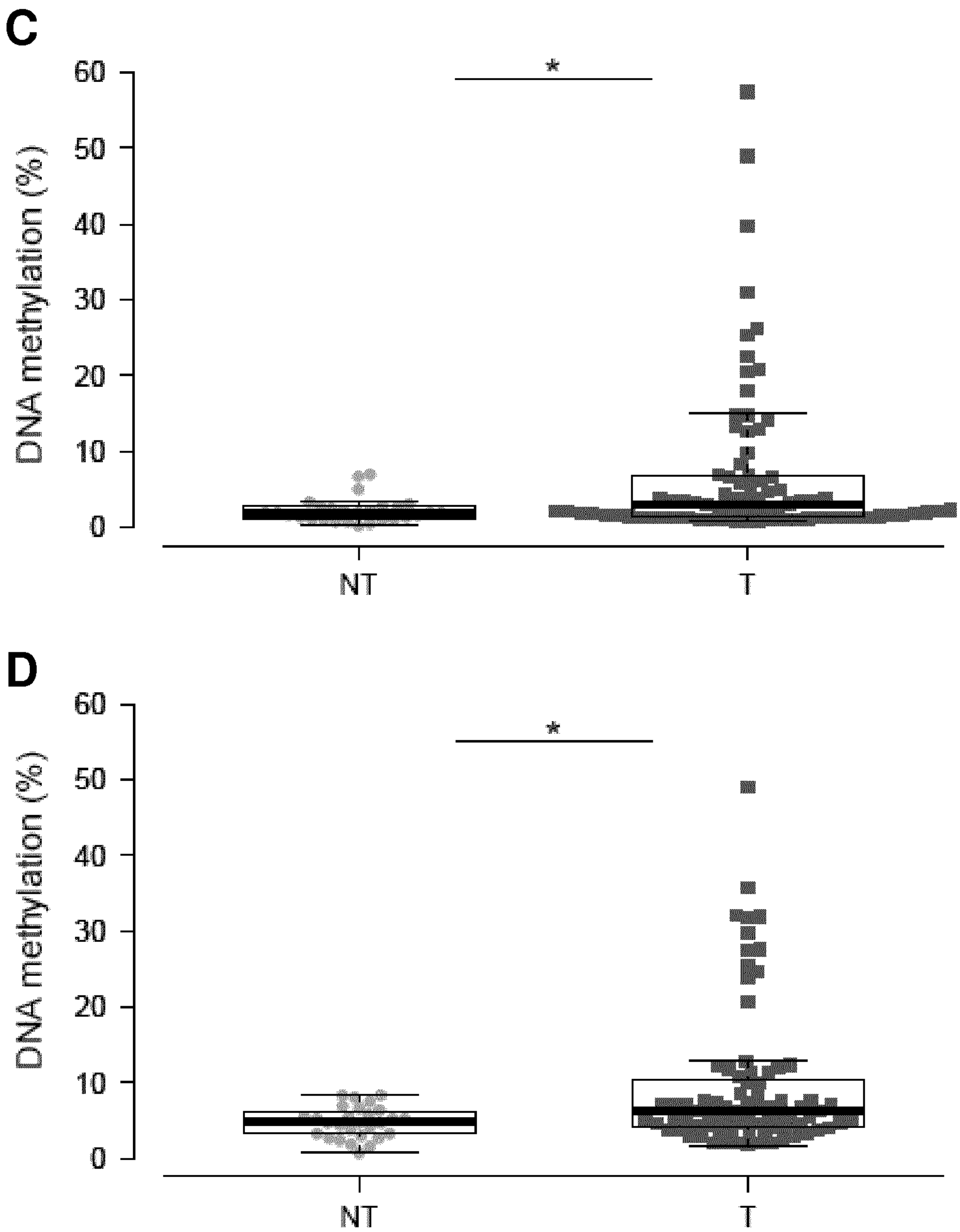
C
60
50
40
30
20
10
0
DNA methylation (%)
*
NT
T
D
60
50
40
30
20
10
0
DNA methylation (%)
*
NT
T

FIG. 3 (cont.)

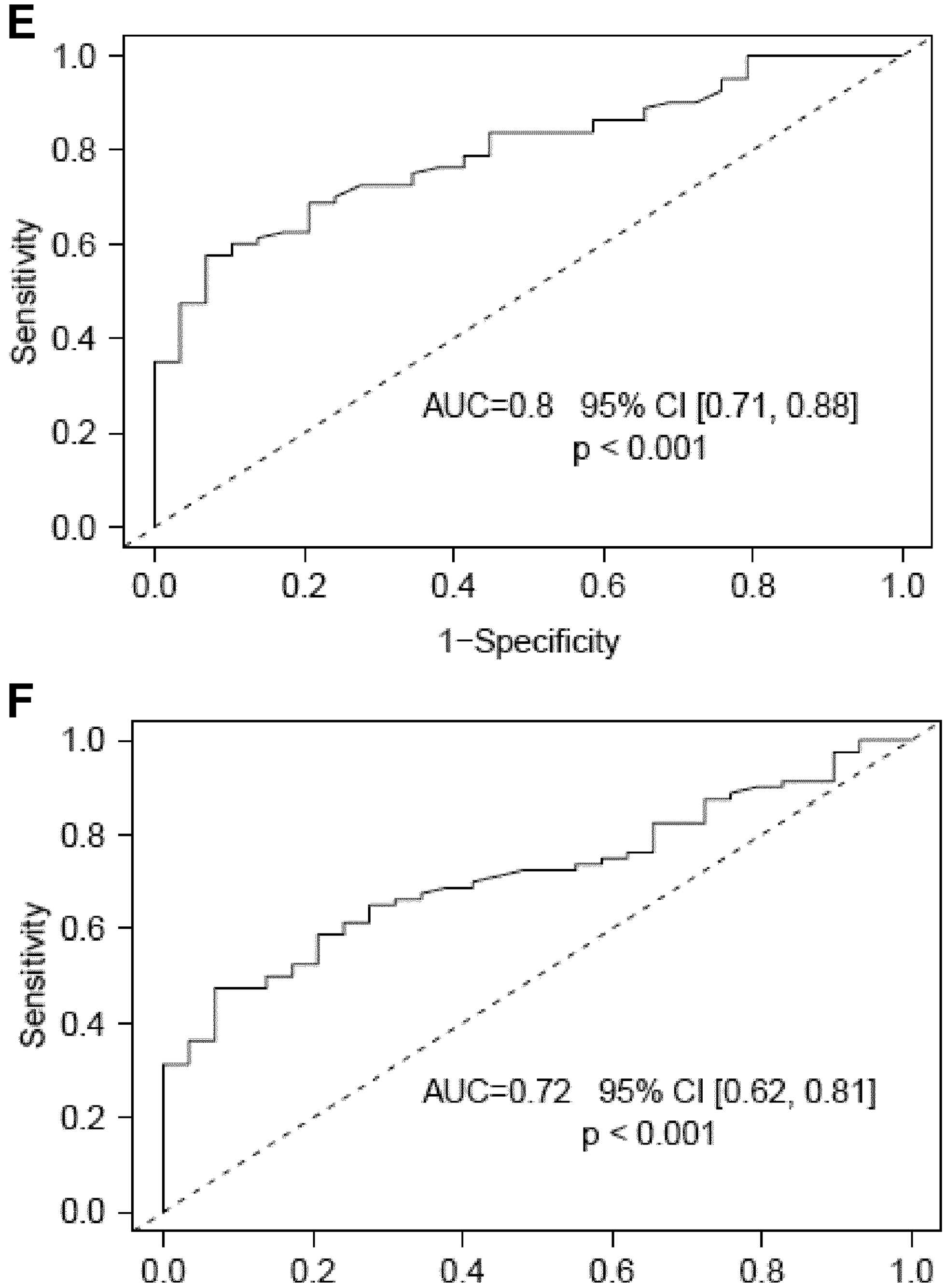
E
1.0
0.8
0.6
0.4
0.2
0.0
Sensitivity
0.0
0.2
0.4
0.6
0.8
1.0
1−Specificity
AUC=0.8 95% CI [0.71, 0.88]
p < 0.001
F
1.0
0.8
0.6
0.4
0.2
0.0
Sensitivity
0.0
0.2
0.4
0.6
0.8
1.0
AUC=0.72 95% CI [0.62, 0.81]
p < 0.001

FIG. 3 (cont.)

G

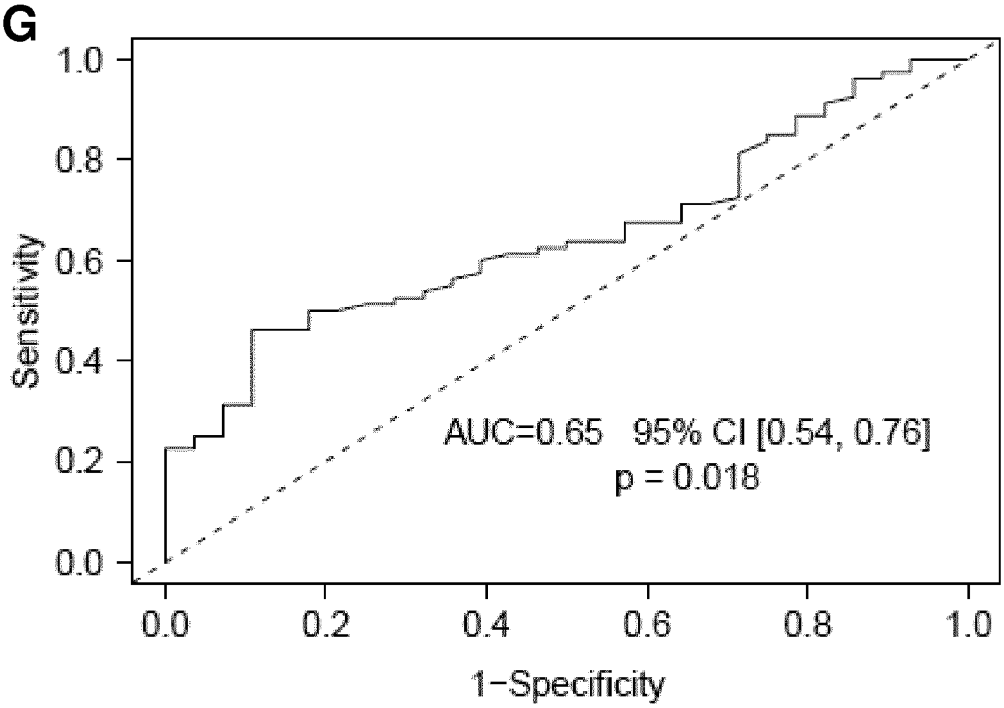
1.0
0.8
0.6
0.4
0.2
0.0
Sensitivity
0.0
0.2
0.4
0.6
0.8
1.0
1−Specificity
AUC=0.65 95% CI [0.54, 0.76]
p = 0.018

H

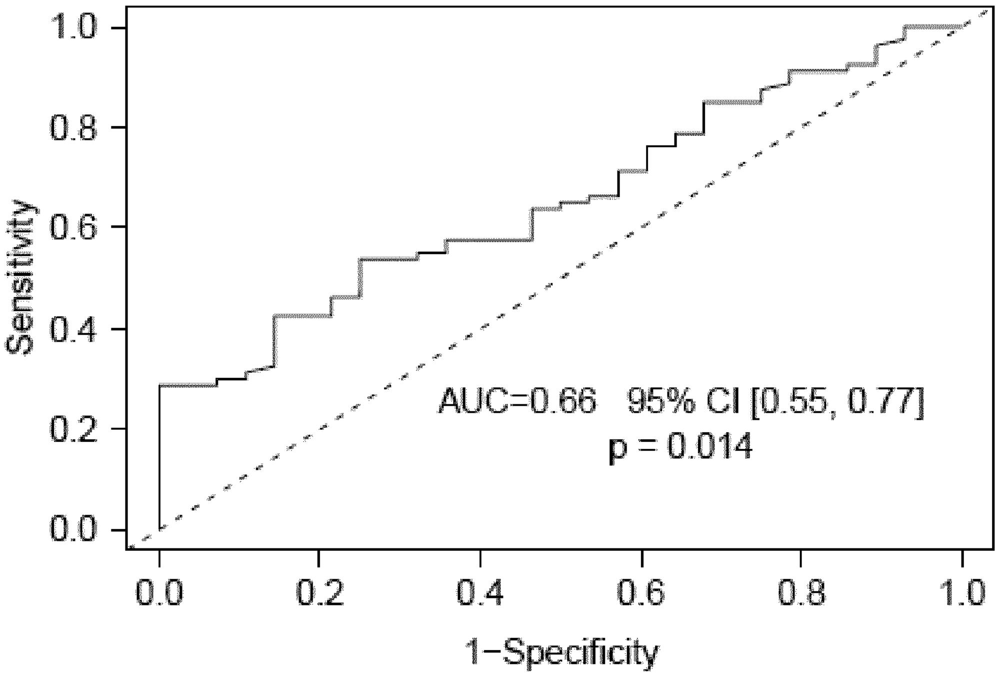
1.0
0.8
0.6
0.4
0.2
0.0
Sensitivity
0.0
0.2
0.4
0.6
0.8
1.0
1−Specificity
AUC=0.66 95% CI [0.55, 0.77]
p = 0.014

**FIG. 3 (cont.)**

I

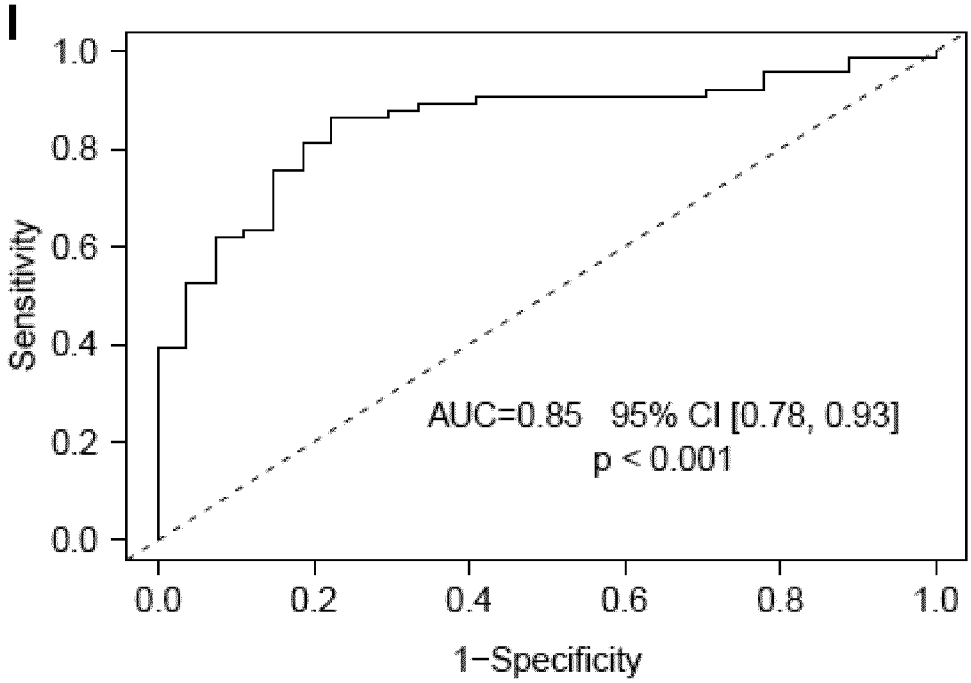
1.0
0.8
0.6
0.4
0.2
0.0
Sensitivity
AUC=0.85 95% CI [0.78, 0.93]
p < 0.001
0.0
0.2
0.4
0.6
0.8
1.0
1−Specificity

J

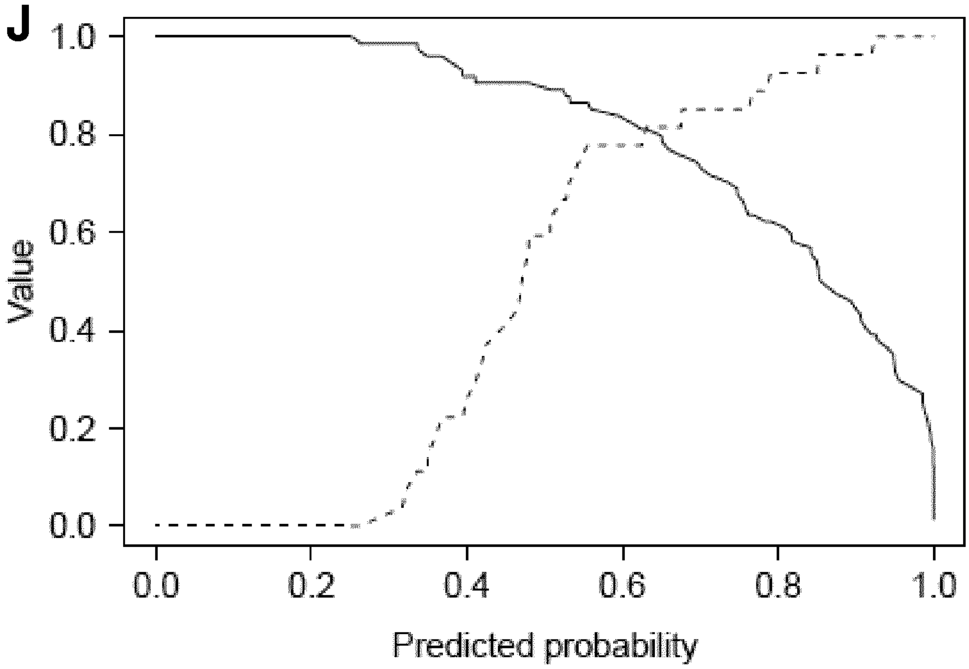
1.0
0.8
0.6
0.4
0.2
0.0
Value
0.0
0.2
0.4
0.6
0.8
1.0
Predicted probability

**FIG. 3 (cont.)**

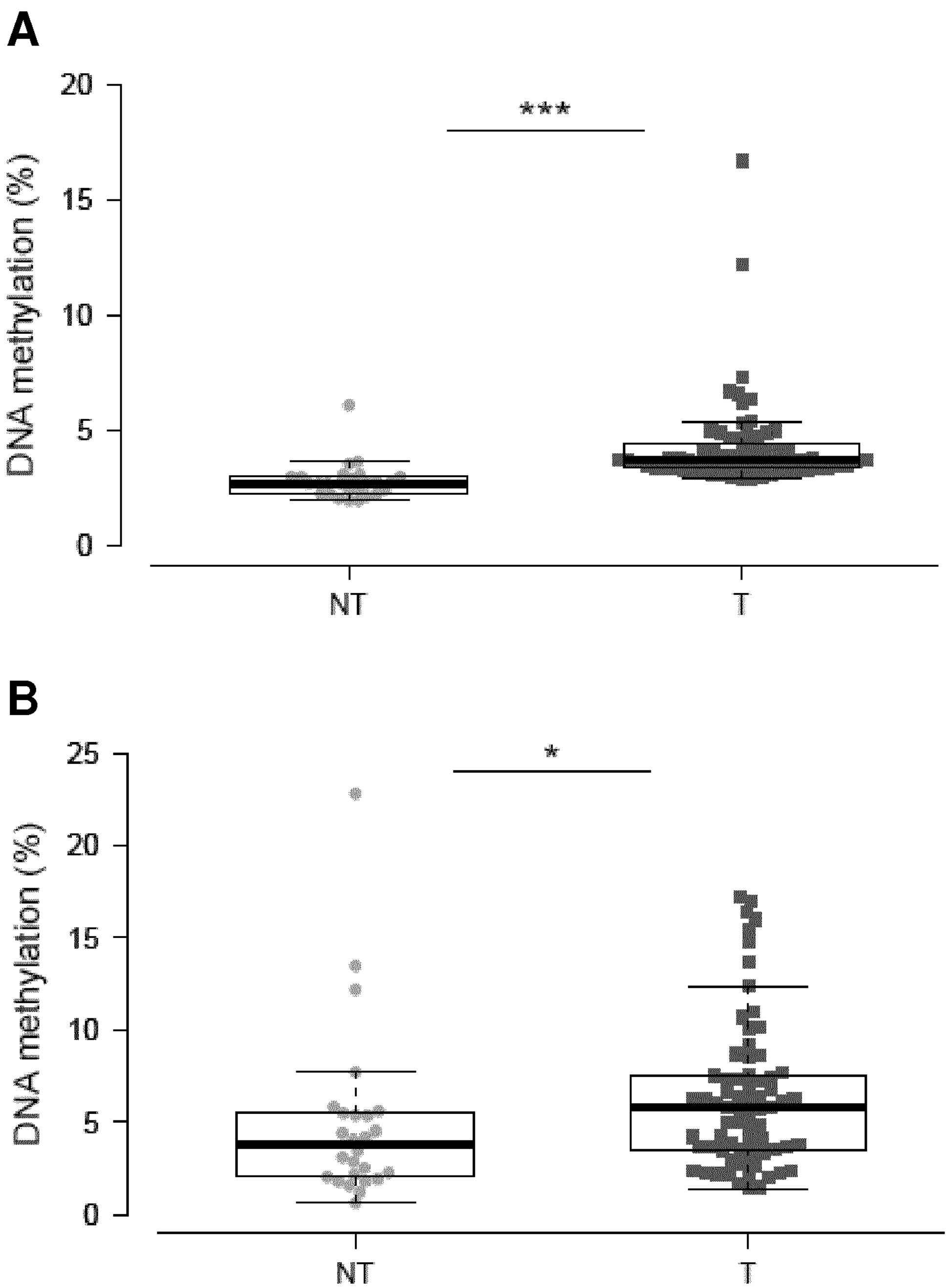
A
20
15
10
5
0
DNA methylation (%)
***
NT
T
B
25
20
15
10
5
0
DNA methylation (%)
*
NT
T

FIG. 4

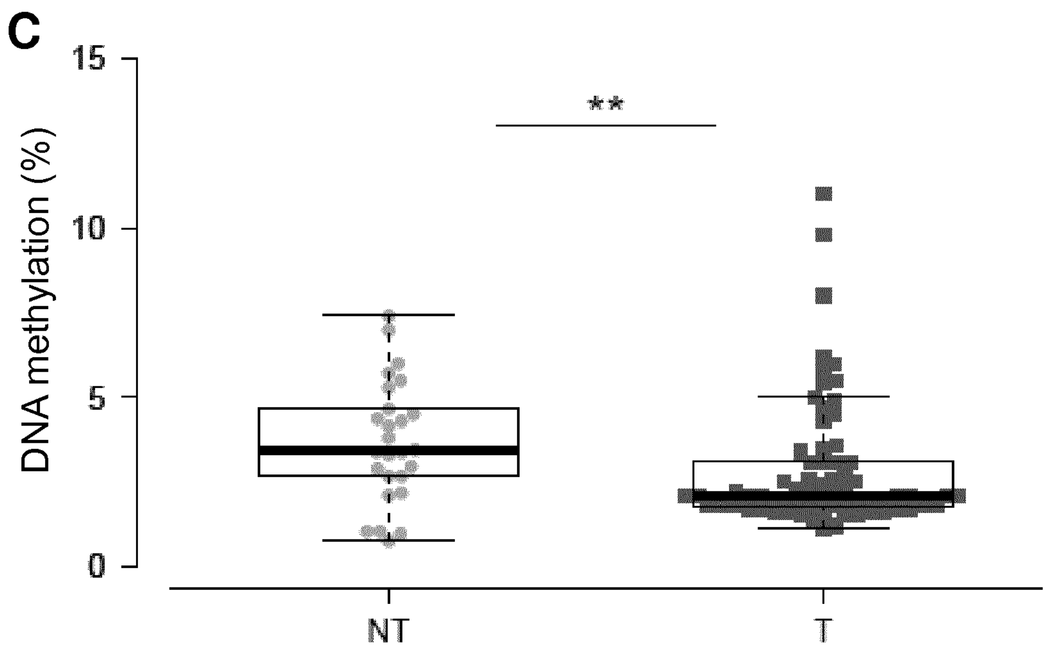
C
15
10
5
0
DNA methylation (%)
**
NT
T

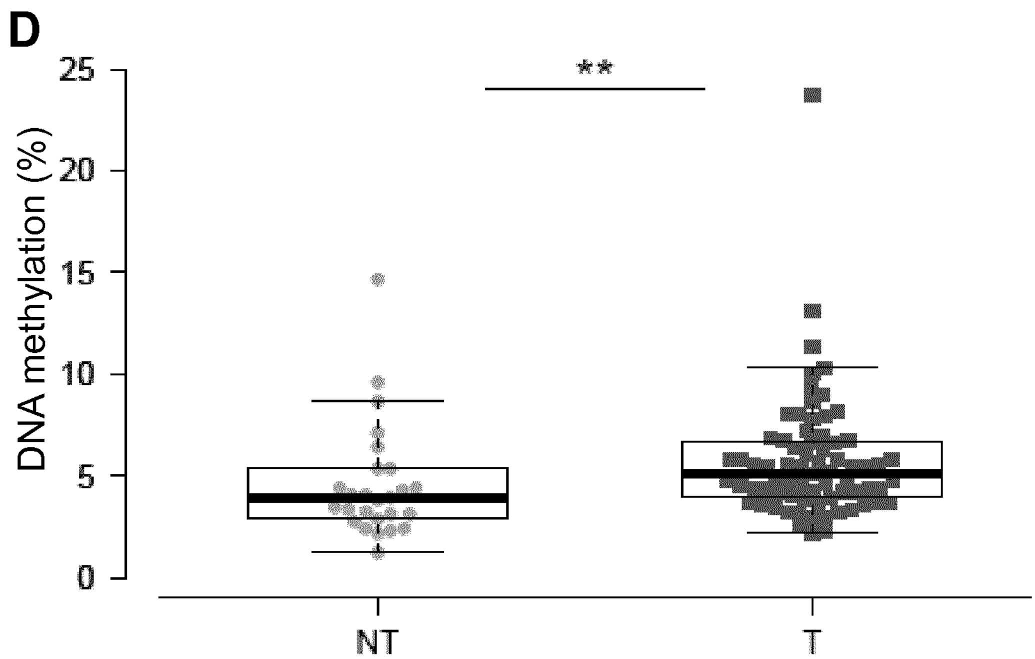
D
25
20
15
10
5
0
DNA methylation (%)
**
NT
T

FIG. 4 (cont.)

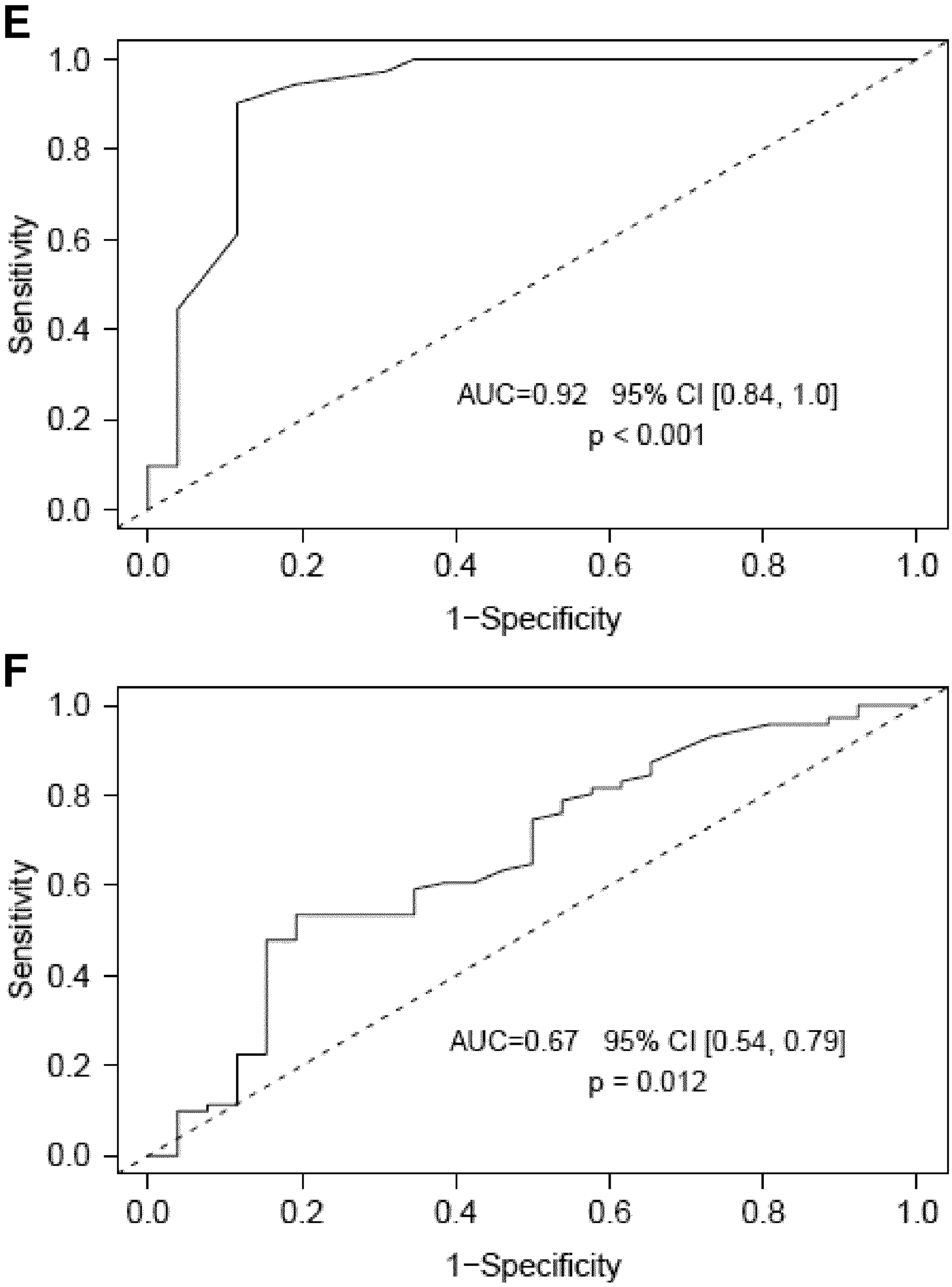
E
1.0
0.8
0.6
0.4
0.2
0.0
Sensitivity
AUC=0.92 95% CI [0.84, 1.0]
p < 0.001
0.0
0.2
0.4
0.6
0.8
1.0
1−Specificity
F
1.0
0.8
0.6
0.4
0.2
0.0
Sensitivity
AUC=0.67 95% CI [0.54, 0.79]
p = 0.012
0.0
0.2
0.4
0.6
0.8
1.0
1−Specificity

**FIG. 4 (cont.)**

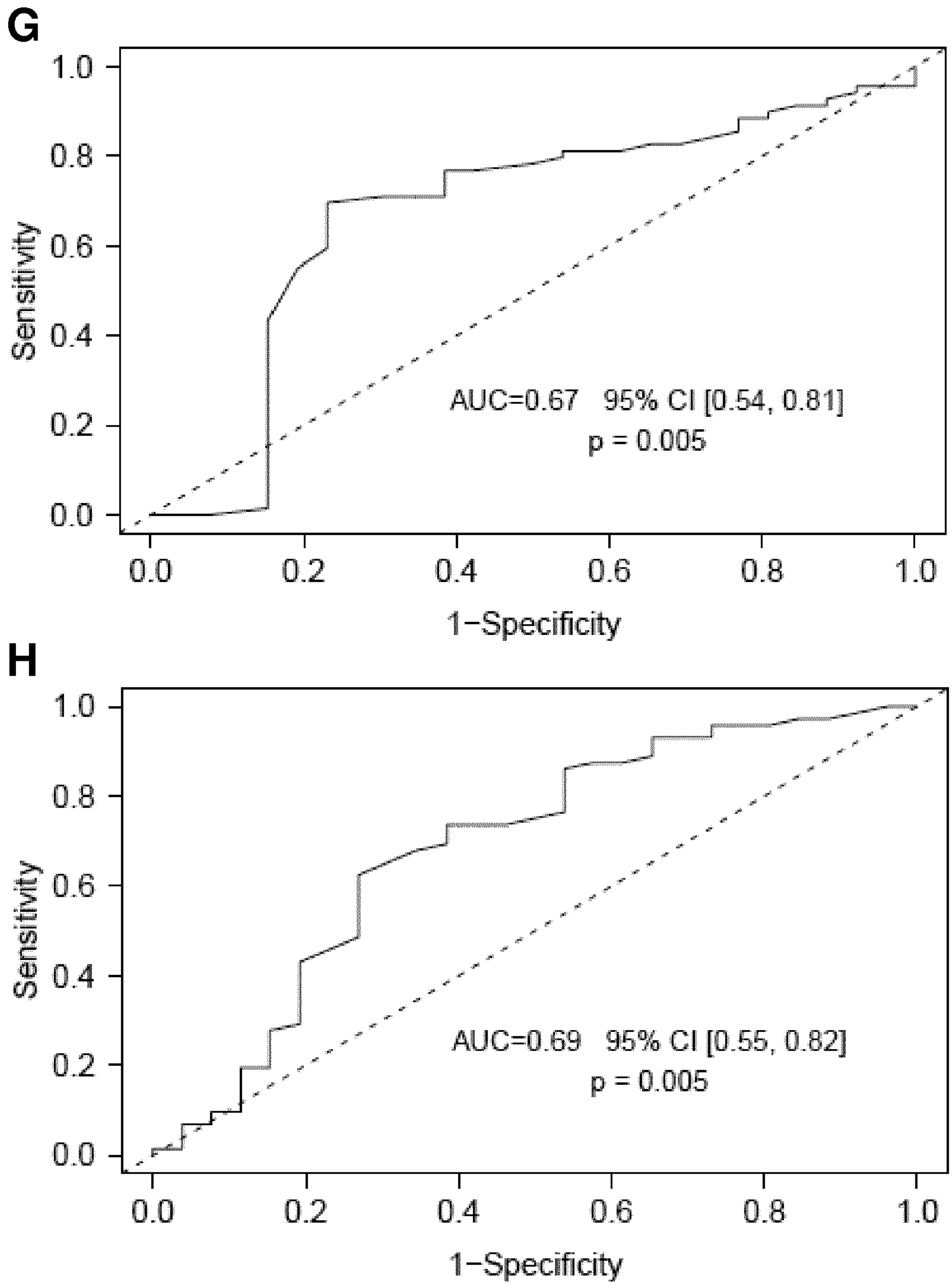
G
1.0
0.8
0.6
0.4
0.2
0.0
Sensitivity
AUC=0.67 95% CI [0.54, 0.81]
p = 0.005
0.0
0.2
0.4
0.6
0.8
1.0
1−Specificity
H
1.0
0.8
0.6
0.4
0.2
0.0
Sensitivity
AUC=0.69 95% CI [0.55, 0.82]
p = 0.005
0.0
0.2
0.4
0.6
0.8
1.0
1−Specificity

FIG. 4 (cont.)

I

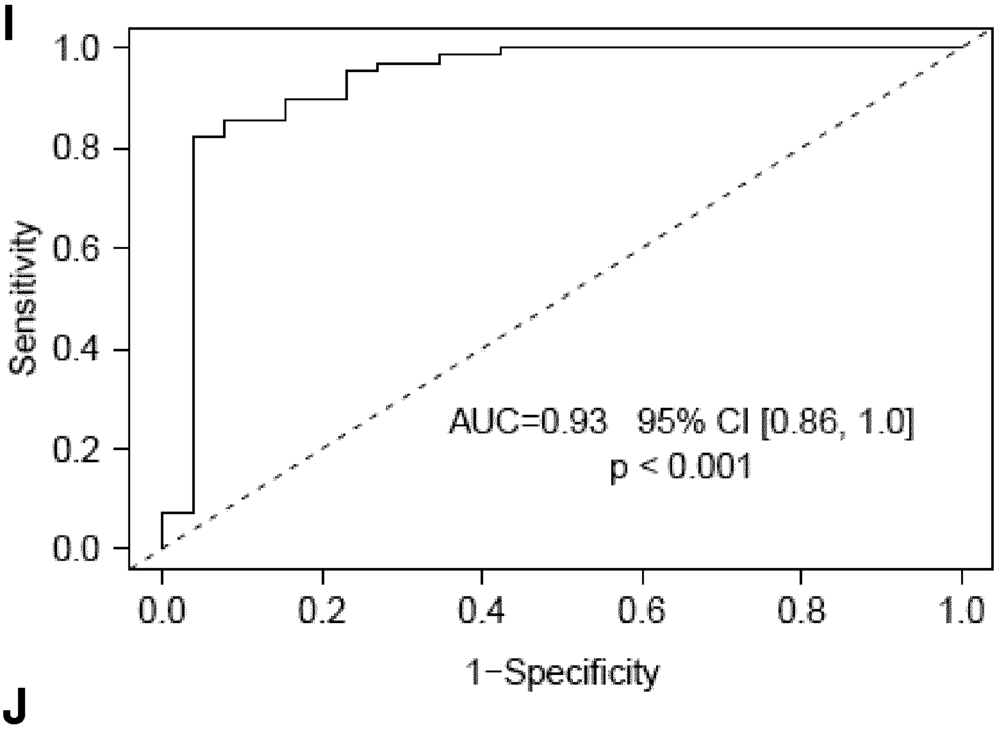
1.0
0.8
0.6
0.4
0.2
0.0
Sensitivity
AUC=0.93 95% CI [0.86, 1.0]
p < 0.001
0.0
0.2
0.4
0.6
0.8
1.0
1−Specificity

J

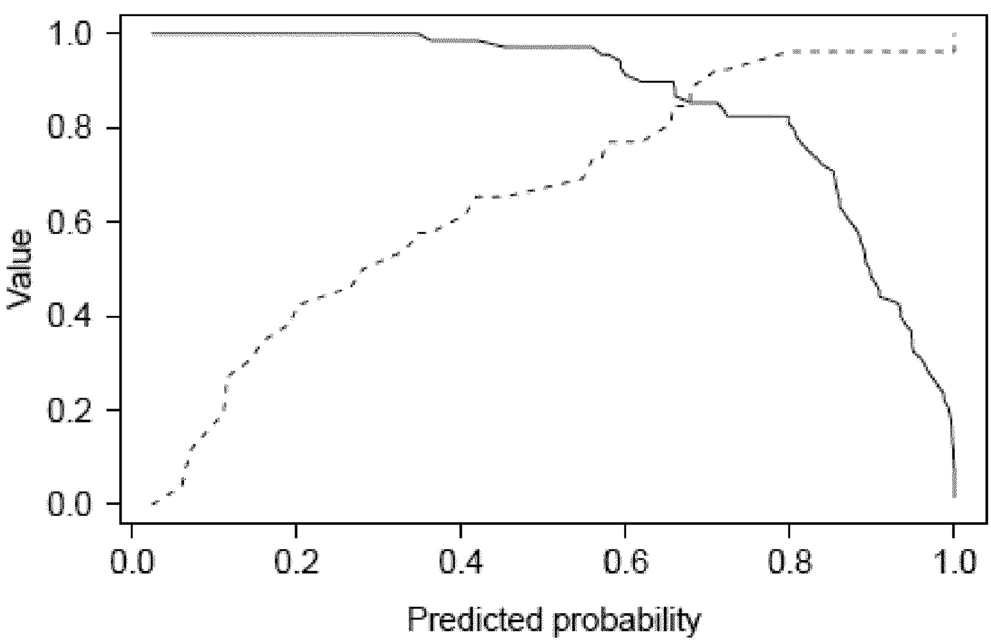
1.0
0.8
0.6
0.4
0.2
0.0
Value
0.0
0.2
0.4
0.6
0.8
1.0
Predicted probability

FIG. 4 (cont.)

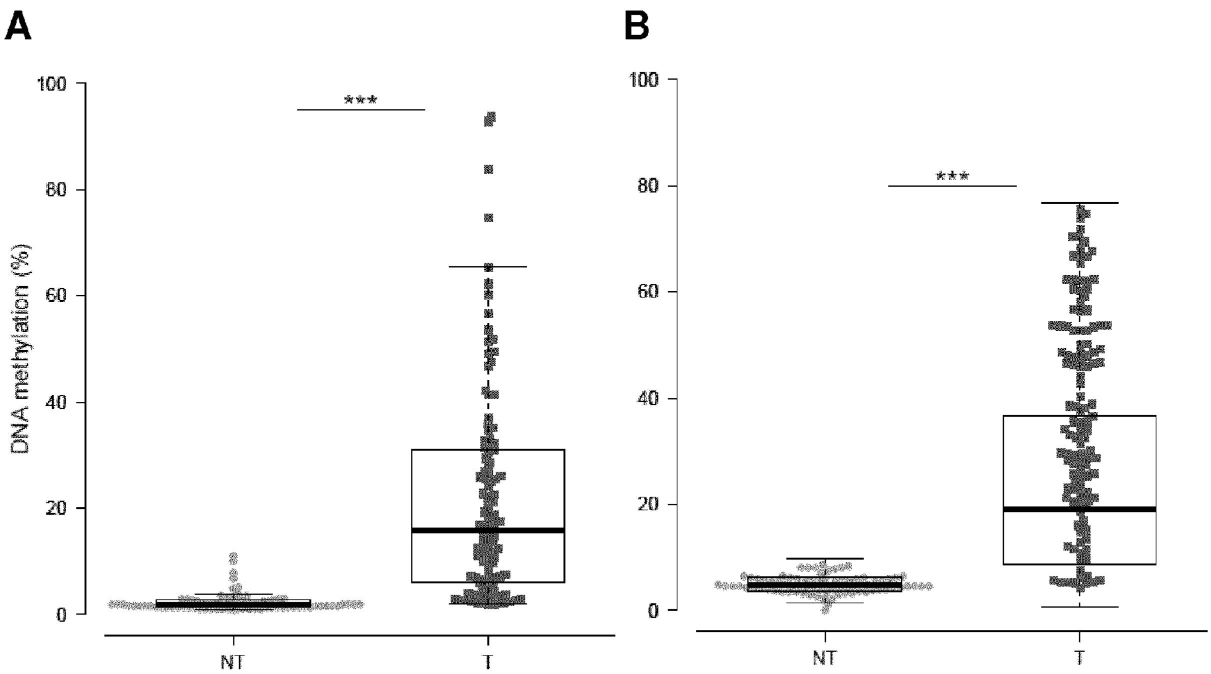


FIG. 5

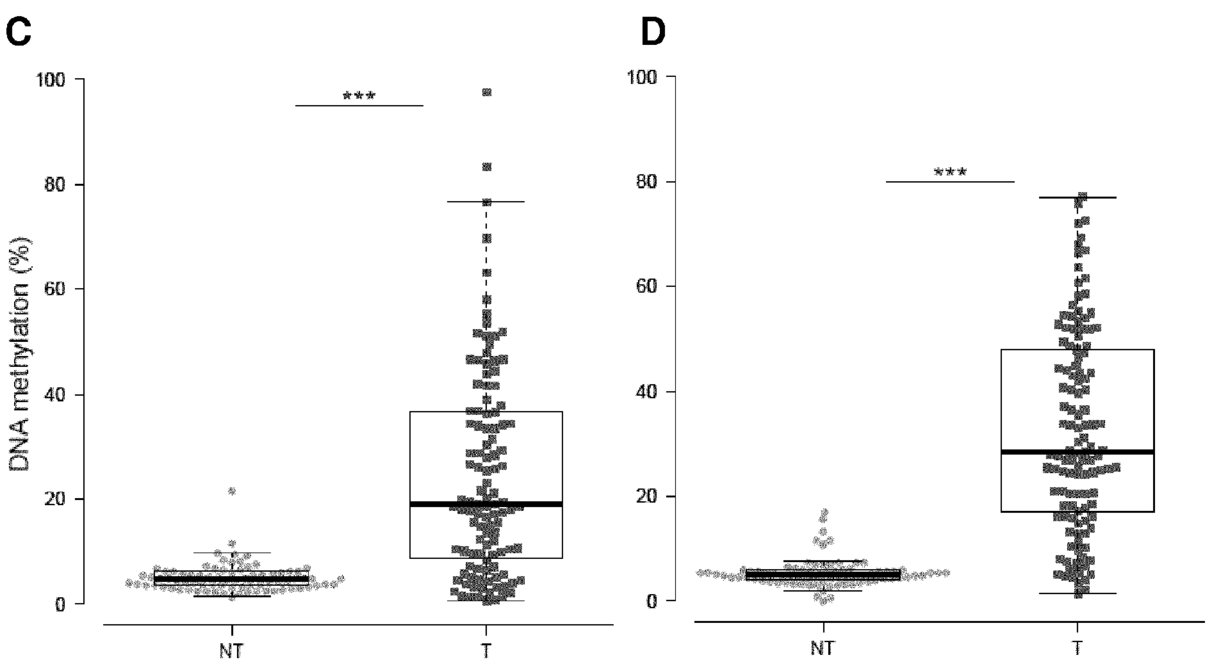


**FIG. 5 (cont.)**

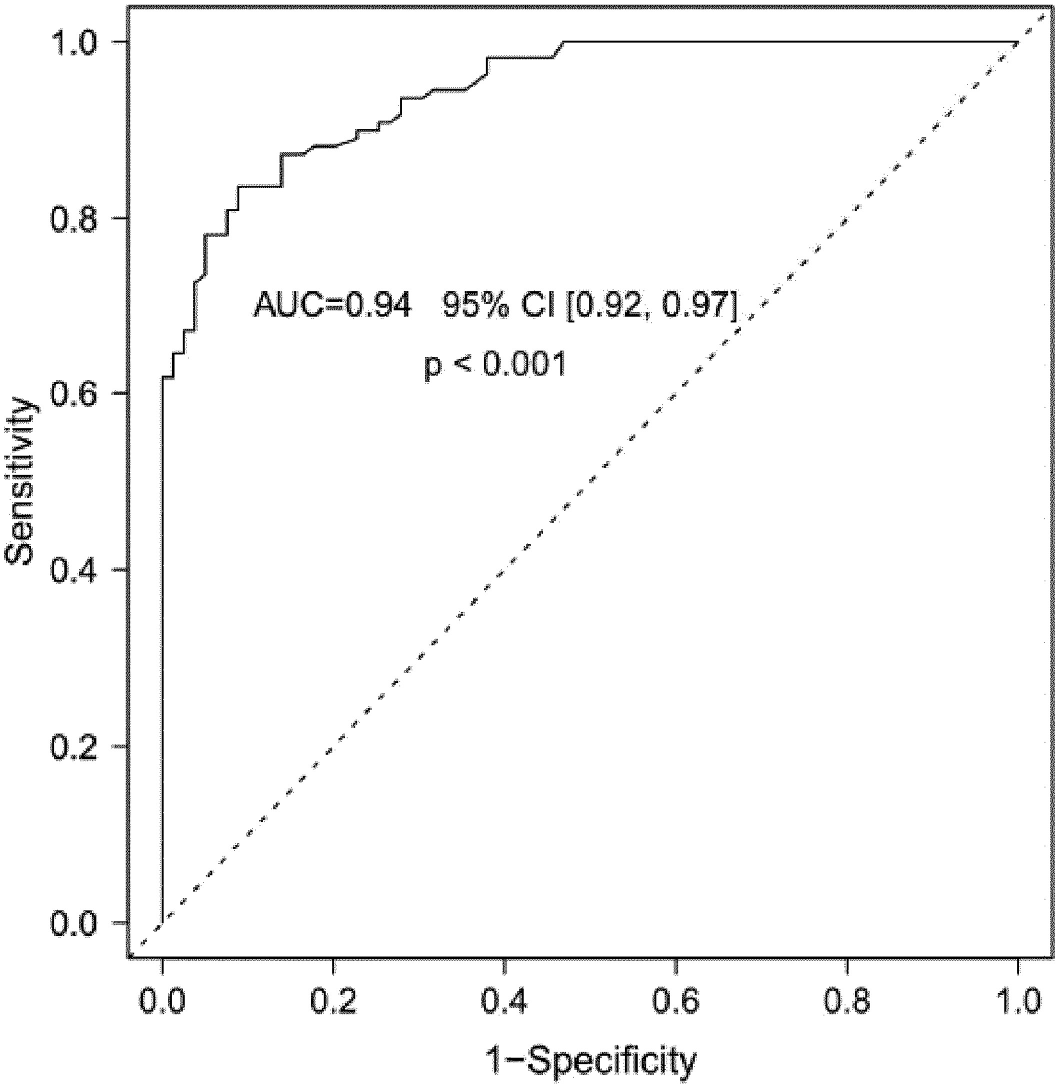
1.0
0.8
0.6
0.4
0.2
0.0
Sensitivity
AUC=0.94 95% CI [0.92, 0.97]
p < 0.001
0.0
0.2
0.4
0.6
0.8
1.0
1−Specificity

FIG. 5E

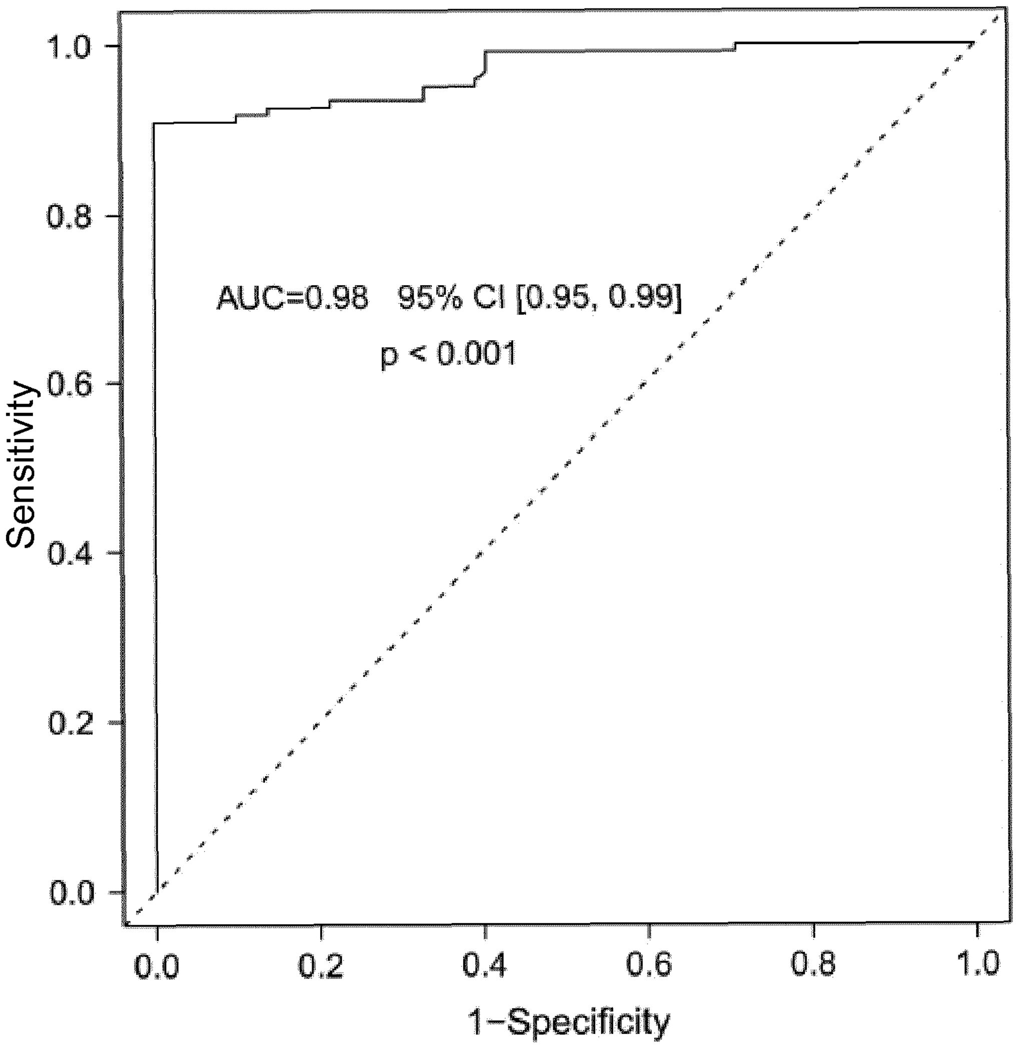
AUC=0.98 95% CI [0.95, 0.99]
p < 0.001
Sensitivity
1.0
0.8
0.6
0.4
0.2
0.0
1−Specificity
0.0
0.2
0.4
0.6
0.8
1.0

**FIG. 5F**

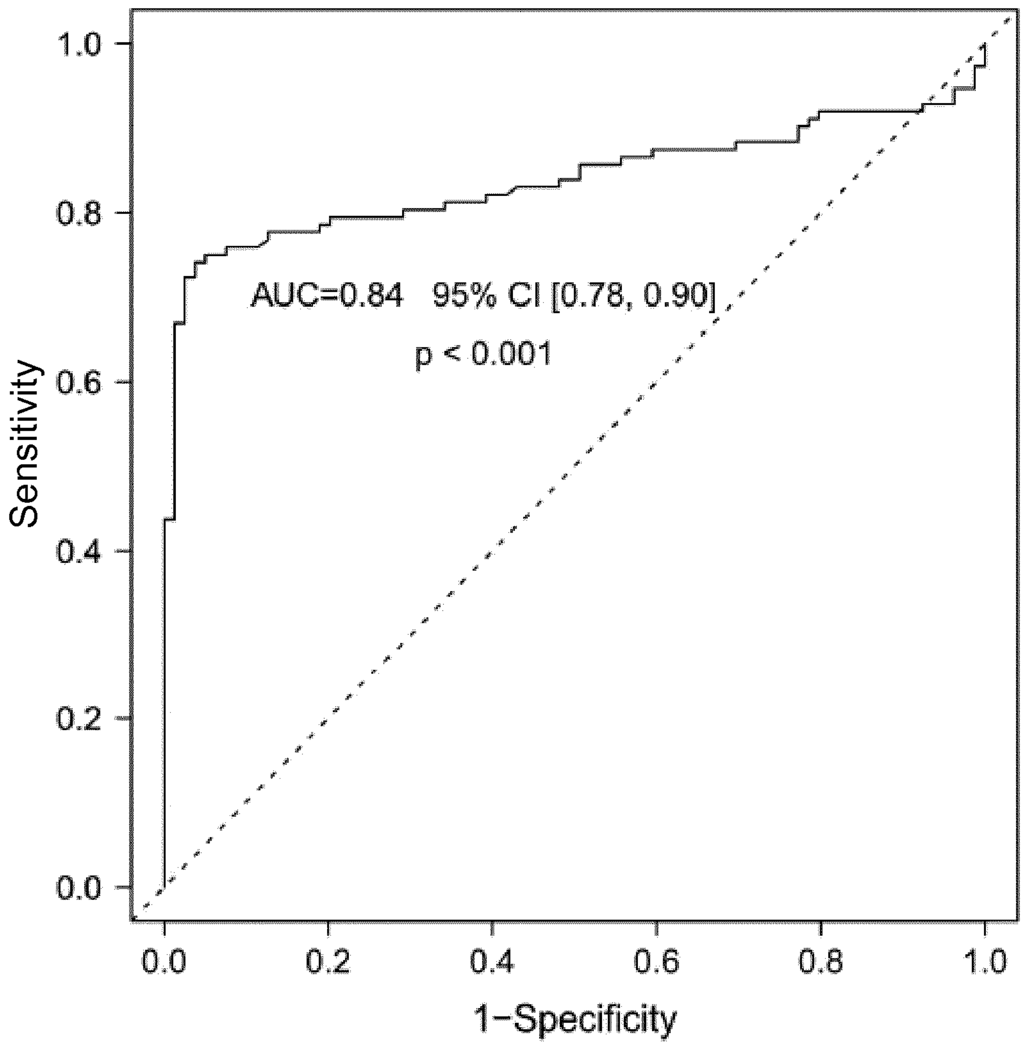
1.0
0.8
0.6
0.4
0.2
0.0
Sensitivity
AUC=0.84 95% CI [0.78, 0.90]
p < 0.001
0.0
0.2
0.4
0.6
0.8
1.0
1−Specificity

FIG. 5G

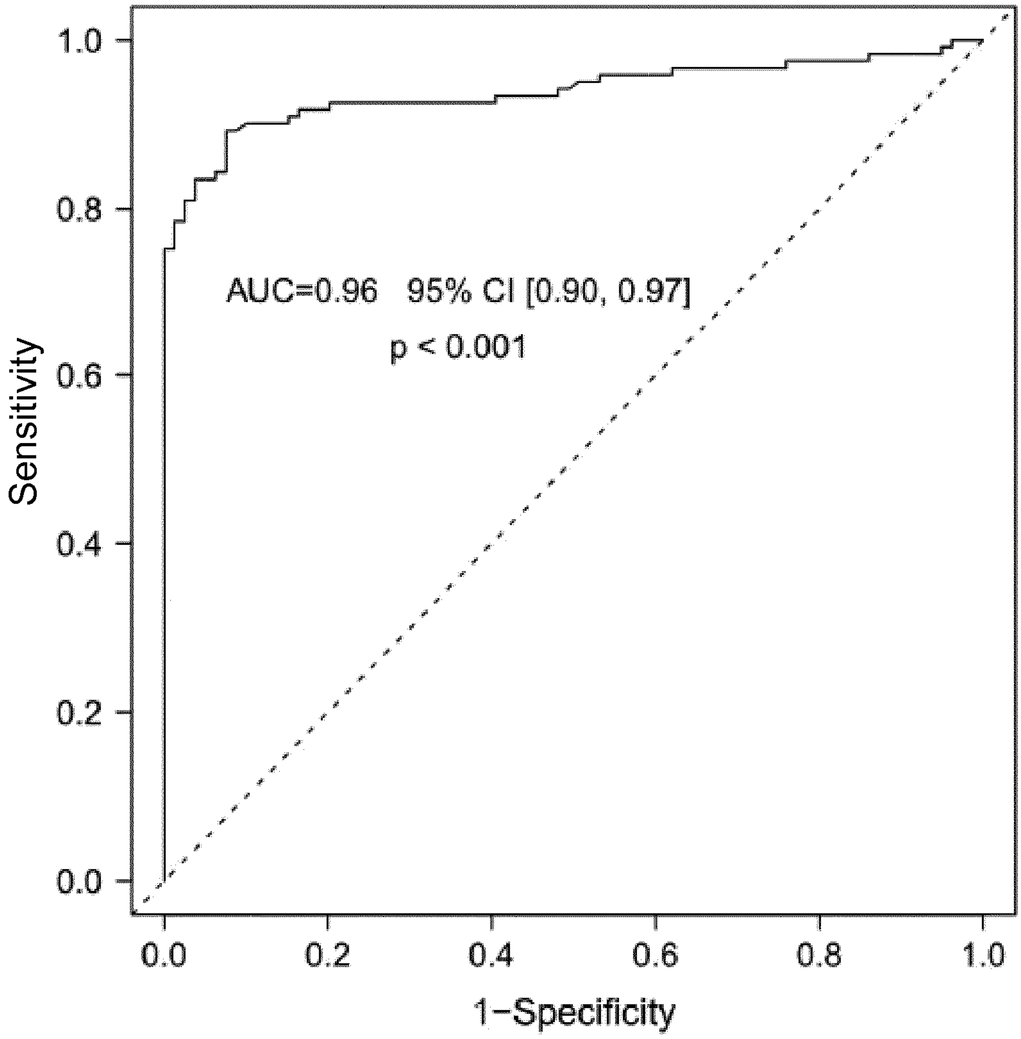
Sensitivity
1.0
0.8
0.6
0.4
0.2
0.0
AUC=0.96 95% CI [0.90, 0.97]
p < 0.001
0.0
0.2
0.4
0.6
0.8
1.0
1−Specificity

**FIG. 5H**

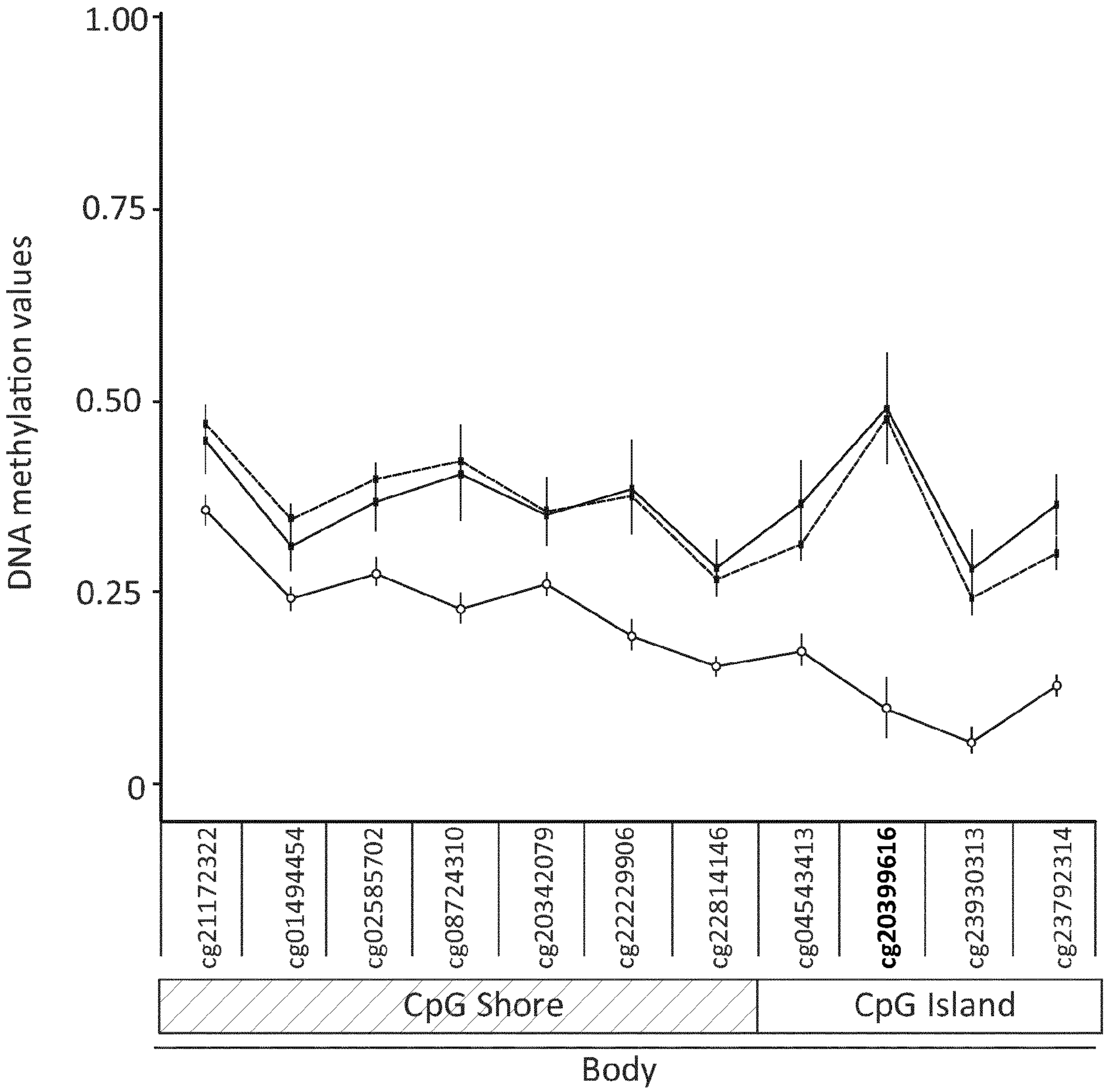
1.00
0.75
0.50
0.25
0
DNA methylation values
cg21172322
cg01494454
cg02585702
cg08724310
cg20342079
cg22229906
cg22814146
cg04543413
cg20399616
cg23930313
cg23792314
CpG Shore
CpG Island
Body

FIG. 6A

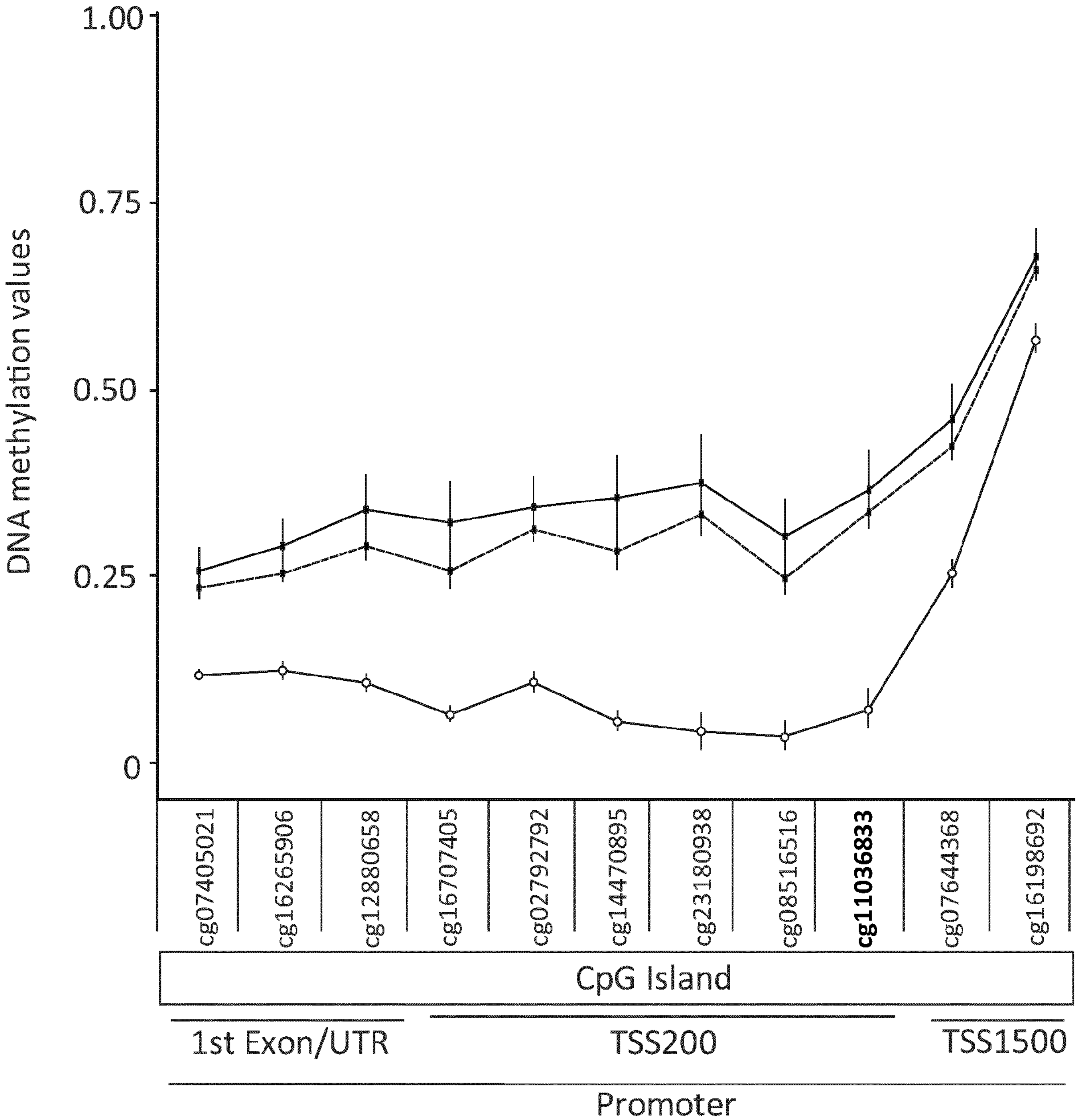
1.00
0.75
0.50
0.25
0
DNA methylation values
cg07405021
cg16265906
cg12880658
cg16707405
cg02792792
cg14470895
cg23180938
cg08516516
cg11036833
cg07644368
cg16198692
CpG Island
1st Exon/UTR
TSS200
TSS1500
Promoter

FIG. 6B

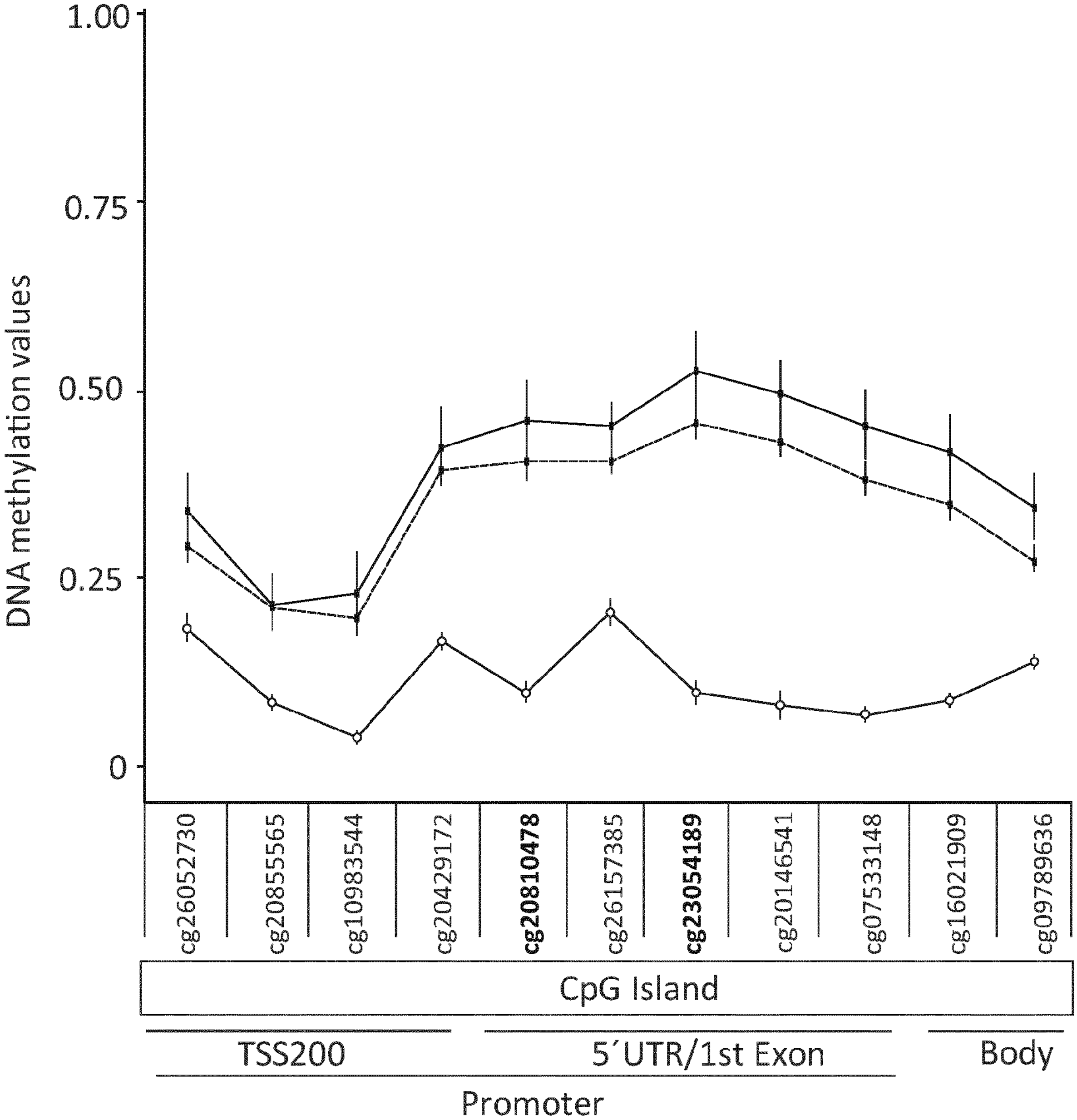
1.00
0.75
0.50
0.25
0
DNA methylation values
cg26052730
cg20855565
cg10983544
cg20429172
cg20810478
cg26157385
cg23054189
cg20146541
cg07533148
cg16021909
cg09789636
CpG Island
TSS200
5´UTR/1st Exon
Body
Promoter

FIG. 6C

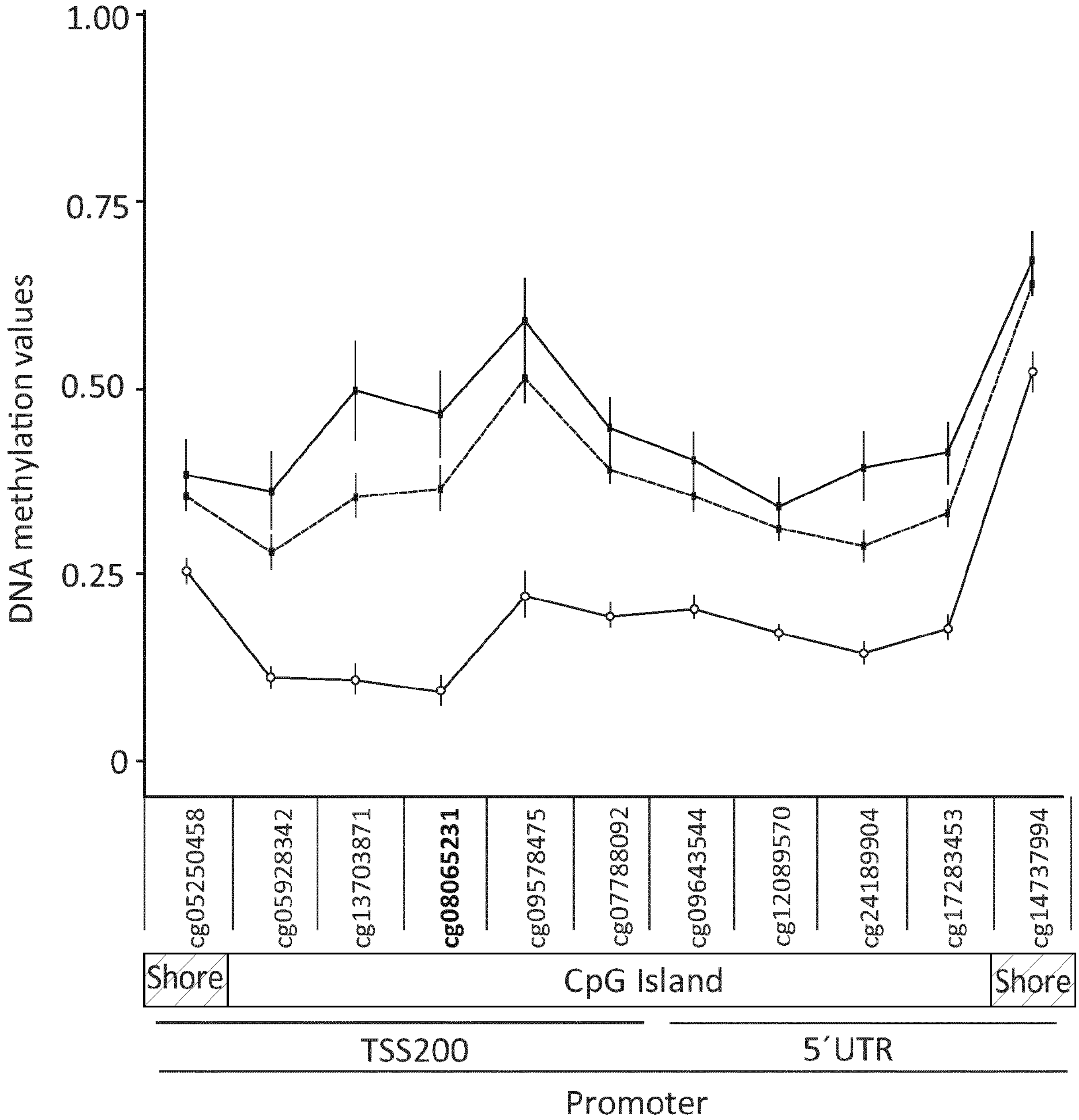
1.00
0.75
0.50
0.25
0
DNA methylation values
cg05250458
cg05928342
cg13703871
cg08065231
cg09578475
cg07788092
cg09643544
cg12089570
cg24189904
cg17283453
cg14737994
Shore
CpG Island
Shore
TSS200
5´UTR
Promoter

FIG. 6D

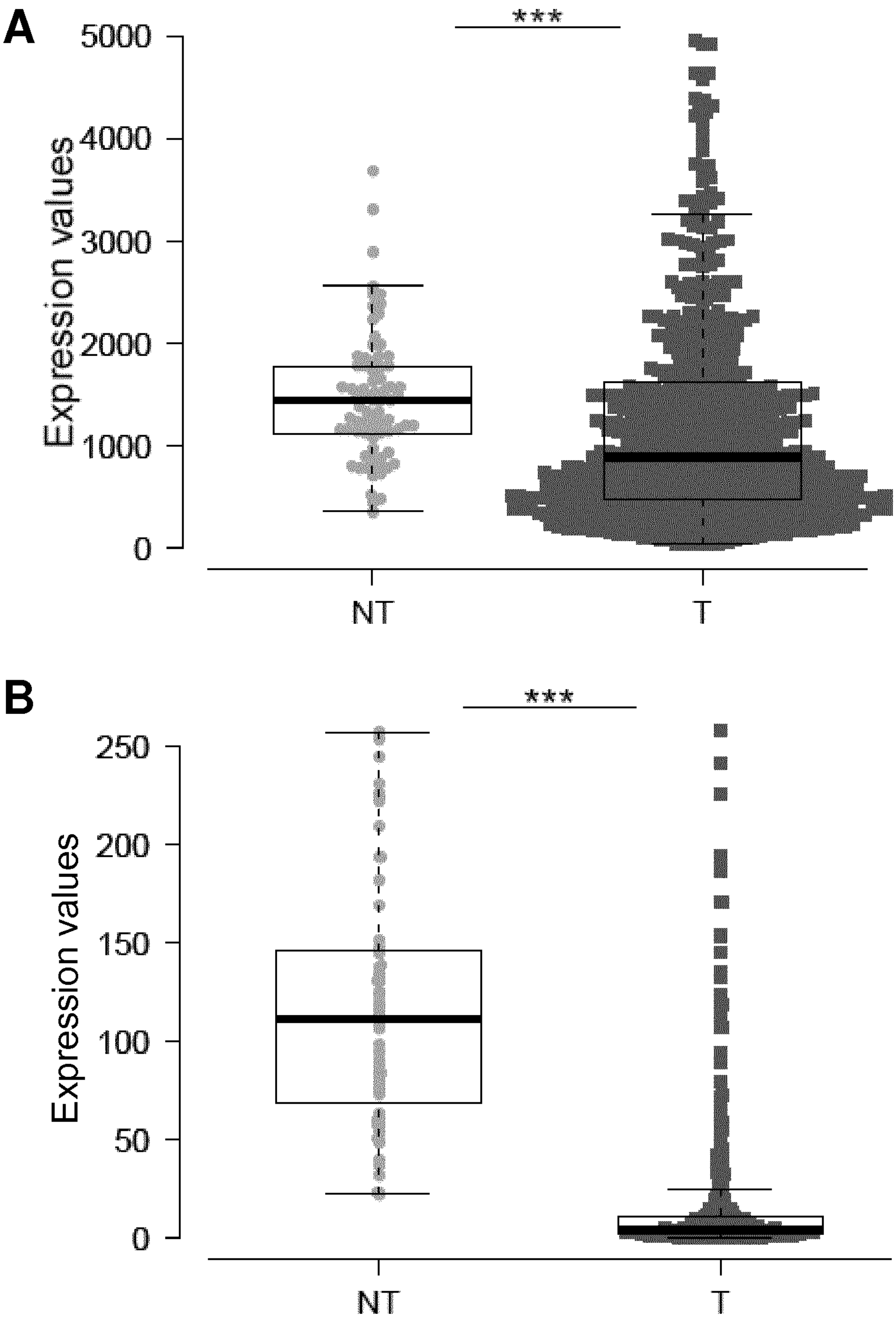
A
***
Expression values
5000
4000
3000
2000
1000
0
NT
T
B
***
Expression values
250
200
150
100
50
0
NT
T

FIG. 7

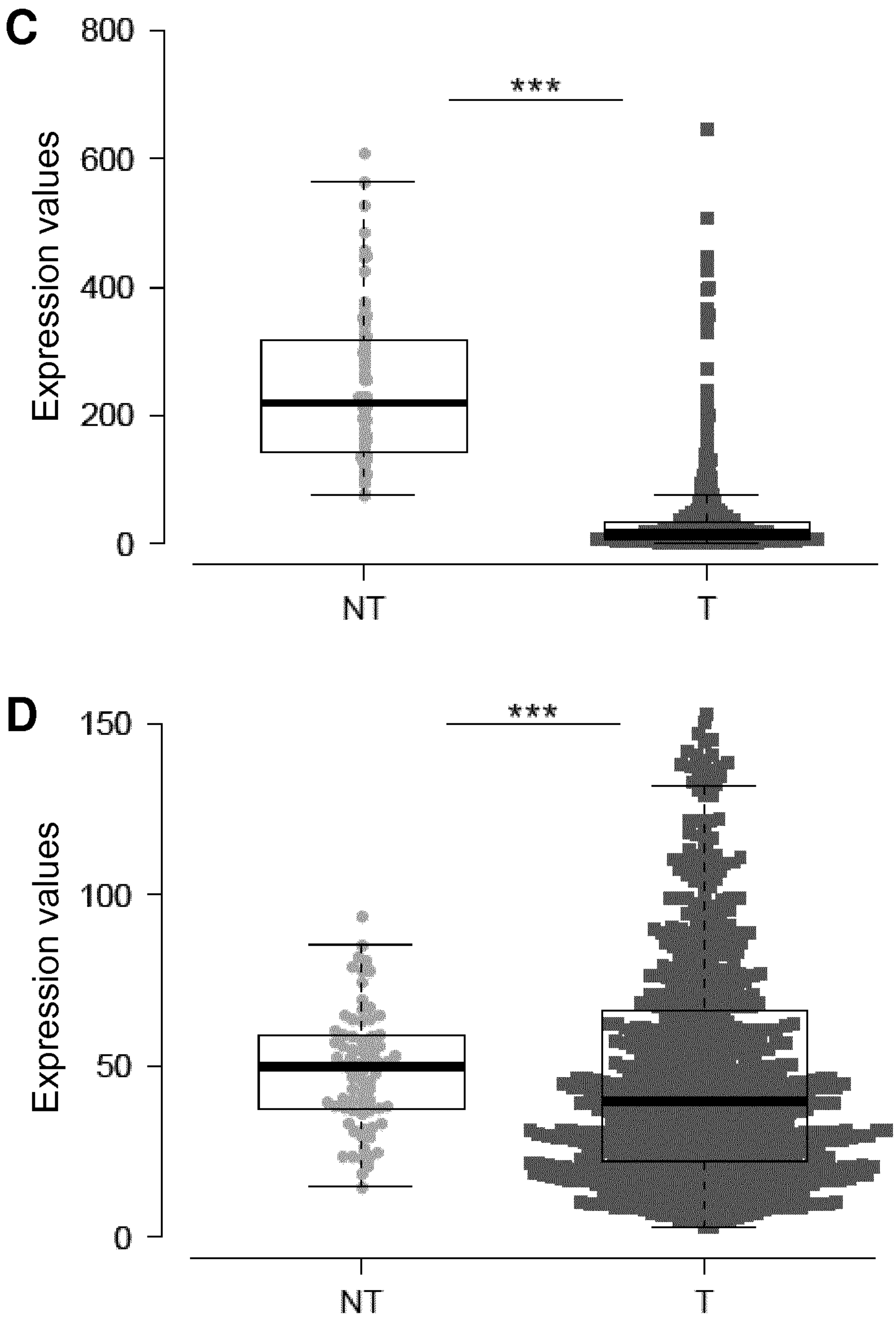
C
800
600
400
200
0
Expression values
***
NT
T
D
150
100
50
0
Expression values
***
NT
T

FIG. 7 (cont.)

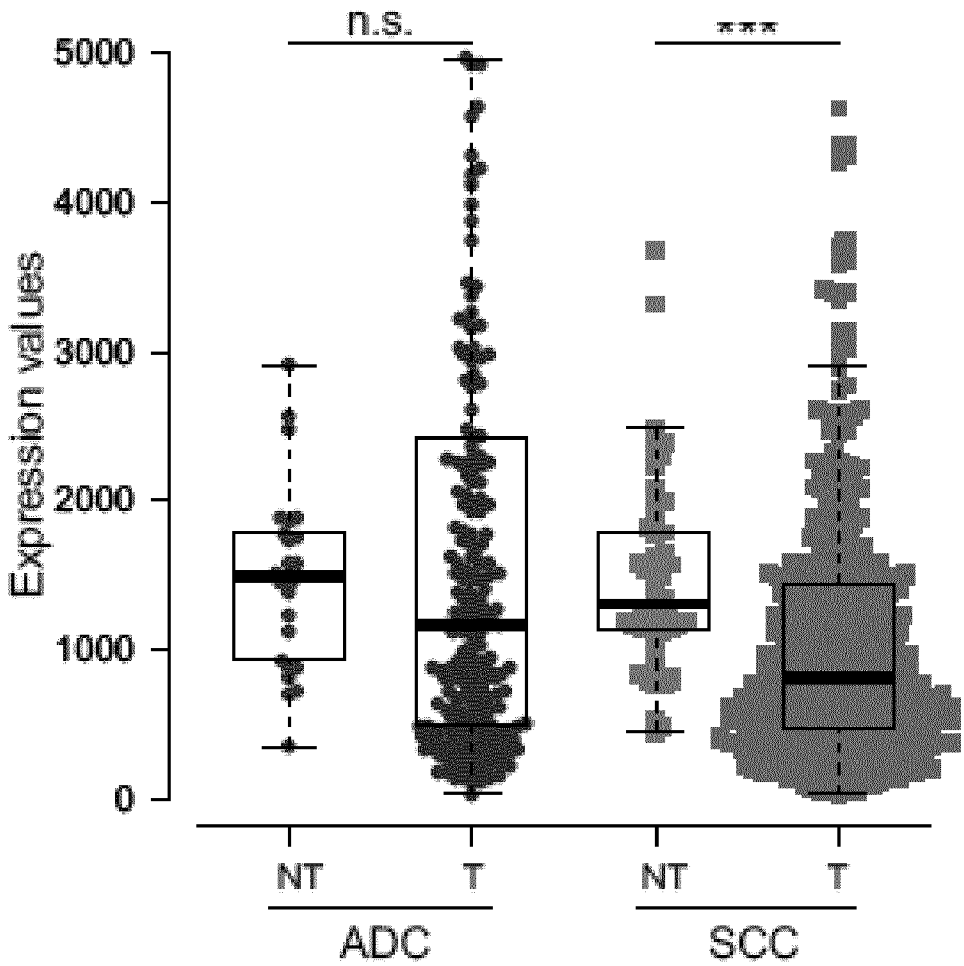
n.s.
***
5000
4000
3000
2000
1000
0
Expression values
NT
T
NT
T
ADC
SCC

FIG. 8A

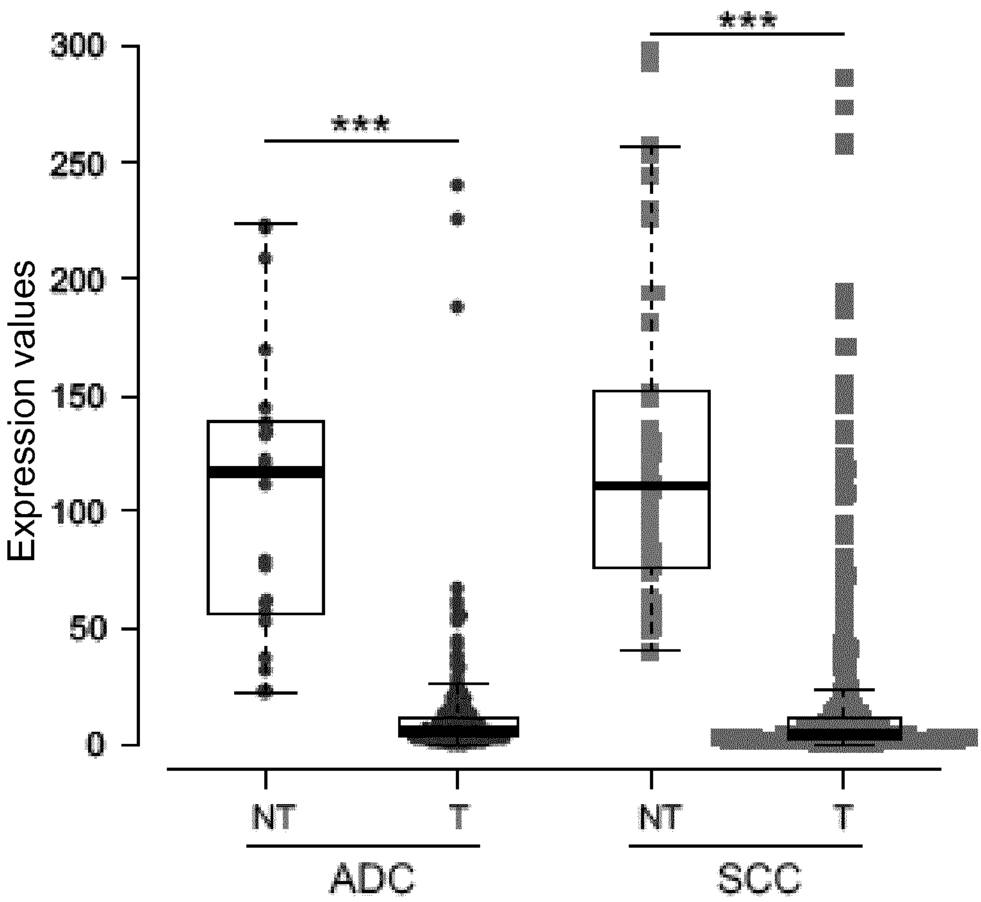


FIG. 8B

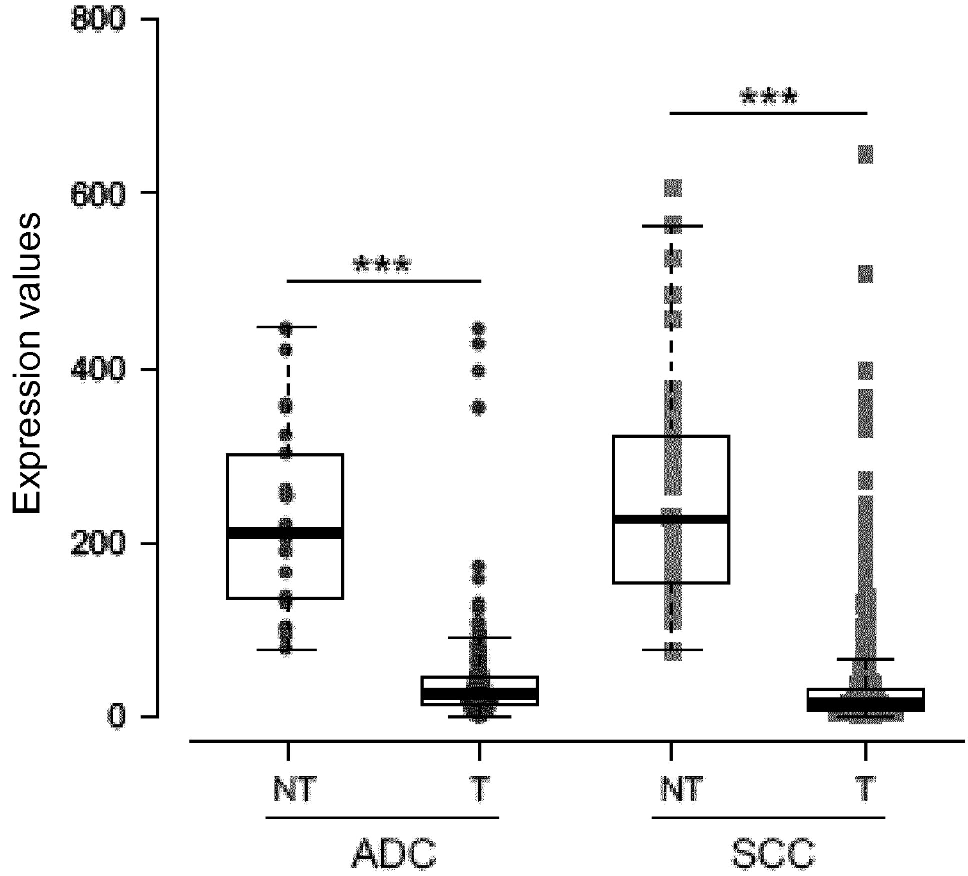


FIG. 8C

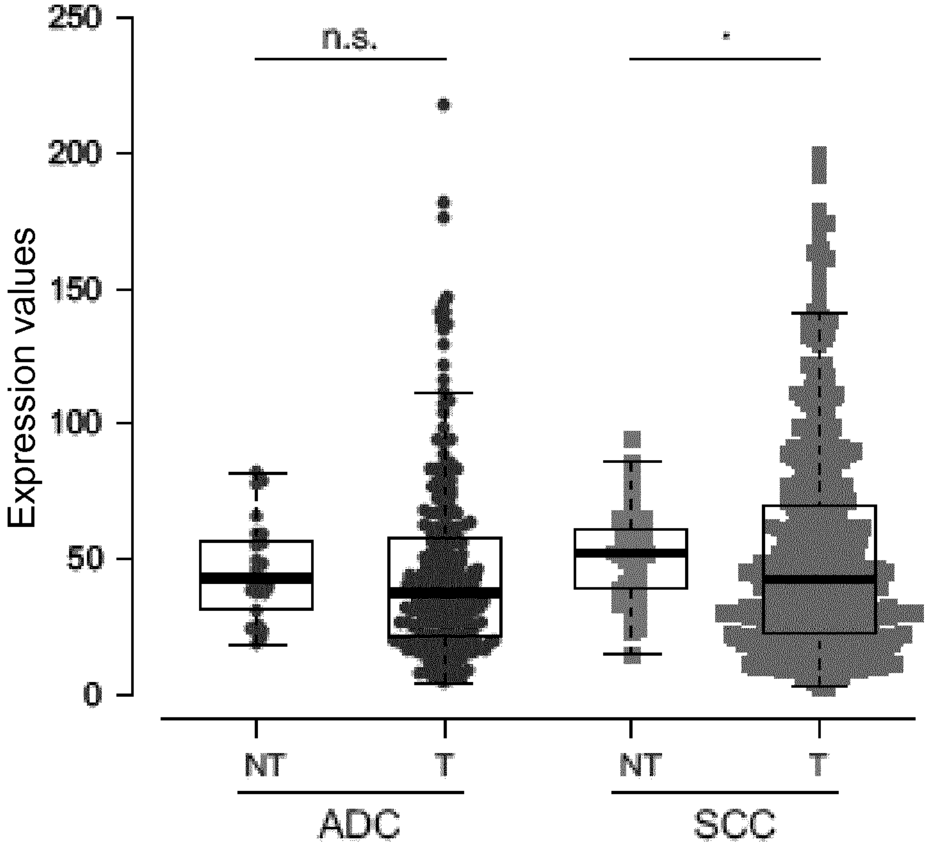


FIG. 8D

Europäisches Patentamt
European Patent Office
Office européen des brevets

# EUROPEAN SEARCH REPORT

Application Number
EP 16 38 2007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | J. WRANGLE ET AL: "Functional Identification of Cancer-Specific Methylation of CDO1, HOXA9, and TAC1 for the Diagnosis of Lung Cancer", CLINICAL CANCER RESEARCH, vol. 20, no. 7, 31 January 2014 (2014-01-31), pages 1856-1864, XP055282059, US ISSN: 1078-0432, DOI: 10.1158/1078-0432.CCR-13-2109 | 10-15 | INV. C12Q1/68 |
| Y | * abstract; figure 2 * * page 1858 * | 1-9 | |
| X | SUSANNE K PEDERSEN ET AL: "Evaluation of an assay for methylated BCAT1 and IKZF1 in plasma for detection of colorectal neoplasia", BMC CANCER, BIOMED CENTRAL, LONDON, GB, vol. 15, no. 1, 6 October 2015 (2015-10-06), page 654, XP021229326, ISSN: 1471-2407, DOI: 10.1186/S12885-015-1674-2 | 10,11 | TECHNICAL FIELDS SEARCHED (IPC) C12Q |
| Y | * page 656 * * abstract * | 1-9, 12-15 | |
| Y | US 2007/264659 A1 (AN SUNGWHAN [KR] ET AL) 15 November 2007 (2007-11-15) * page 7 * | 1-15 | |
| Y | L. VAN NESTE ET AL: "MC13-0087 Epigenetic assay detects early stage non-small cell lung cancer in sputum", EUROPEAN JOURNAL OF CANCER, vol. 49, 1 November 2013 (2013-11-01), page S36, XP055282036, * abstract * | 1-15 | |
| | -/-- | | |

The present search report has been drawn up for all claims

2

| Place of search | Date of completion of the search | Examiner |
|---|---|---|
| The Hague | 1 July 2016 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 16 38 2007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2012/175562 A2 (UNIV TARTU [EE]; ANNILO TARMO [EE]; TONISSON NEEME [EE]; VOODER TONU [) 27 December 2012 (2012-12-27)<br>* abstract; claims *<br>----- | 1-15 | |
| A | YING-CHIEH CHEN ET AL: "Quantitative DNA methylation analysis of selected genes in endometrial carcinogenesis",<br>TAIWANESE JOURNAL OF OBSTETRICS AND GYNECOLOGY,<br>vol. 54, no. 5,<br>1 October 2015 (2015-10-01), pages 572-579, XP055282084,<br>HK<br>ISSN: 1028-4559, DOI: 10.1016/j.tjog.2015.08.010<br>* abstract *<br>----- | 1-15 | |
| A | WO 2014/173905 A2 (INST INVESTIGACIÓ BIOMÈDICA DE BELLVITGE IDIBELL [ES]; FUNDACIÓ INSTIT)<br>30 October 2014 (2014-10-30)<br>* abstract; claims *<br>----- | 1-15 | TECHNICAL FIELDS SEARCHED (IPC) |

The present search report has been drawn up for all claims

| Place of search | Date of completion of the search | Examiner |
|---|---|---|
| The Hague | 1 July 2016 | Reuter, Uwe |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 38 2007

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2016

| Patent document cited in search report | Publication date | Patent family member(s) | Publication date |
|---|---|---|---|
| US 2007264659 A1 | 15-11-2007 | NONE | |
| WO 2012175562 A2 | 27-12-2012 | NONE | |
| WO 2014173905 A2 | 30-10-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5786146 A


### Non-patent literature cited in the description


- **BARAULT L et al.** Digital PCR quantification of MGMT methylation refines prediction of clinical benefit from alkylating agents in glioblastoma and metastatic colorectal cancer. *Ann Oncol,* 2015, vol. 26, 1994-9
- **HOQUE MO et al.** Quantitative methylation-specific polymerase chain reaction gene patterns in urine sediment distinguish prostate cancer patients from control subjects. *J Clin Oncol,* 2005, vol. 23, 6569-75
- **DIETRICH D et al.** Performance evaluation of the DNA methylation biomarker SHOX2 for the aid in diagnosis of lung cancer based on the analysis of bronchial aspirates. *Int J Oncol,* 2012, vol. 40, 825-32
- **TRAVIS et al.** Histological typing of lung and pleural tumours. Springer-Verlag, 1999
- **SAMBROOK et al.** Molecular Cloning: A Laboratory Manual. Cold Spring Harbor Press, 1989
- **ALTSCHUL et al.** NCBI NLM NIH. 1990, 20894
- **ALTSCHUL, S. et al.** *J. Mol Biol,* vol. 215, 403-10
- **NYRÉN, P.** The History of Pyrosequencing. *Methods Mol Biology,* 2007, vol. 373, 1-14
- **SANDOVAL et al.** *J Clin Oncol,* 2013, vol. 31, 4140-7
- **SANDOVAL J. et al.** A prognostic DNA methylation signature for stage I non-small-cell lung cancer. *J Clin Oncol,* 2013, vol. 31, 4140-7

SEQUENCE LISTING

<110> INSTITUT D'INVESTIGACIÓ BIOMÈDICA DE BELLVITGE (IDIBELL)
FUNDACIÓN PARA LA INVESTIGACIÓN DEL HOSPITAL UNIVERSITARIO
Y POLITECNICO LA FE DE LA COMUNIDAD VALENCIANA
FUNDACIÓN PARA LA INVESTIGACIÓN MÉDICA APLICADA

<120> METHOD AND KIT FOR THE DIAGNOSIS OF LUNG CANCER

<130> EP20151025

<160> 27

<170> PatentIn version 3.5

<210> 1
<211> 48771
<212> DNA
<213> Artificial Sequence

<220>
<223> BCAT1 cg21172322-cg23792314

<400> 1
cggggtaacc tacgtgacgc tcaccatgat accgtgcgct cctctccagg acccaggcaa 60

acacaaaaaa ggaggctcag acaaccaagc acccaggccc gaacctccaa caagcgtgtc 120

ttgggagcgc tgccctgcac ttcccaccct gccggggtcg cggcggtttt tgtcctcctc 180

cgccggctca gggaagactg gttaaattcc aggtcagccc tacagagcca gggttcgccg 240

gcaaagaaca aaaaaacaat tgtctccctt atatccgagc aaatagtcta gactggggtg 300

ttaagccatt tatagaaaaa tctccagggc gcgctcagcc tcgtggtctt gtcaatcaca 360

gacgcacaat agcaagcctg caaagggaac ggggacgggc gtgaaccatt tcctccacca 420

gcagggtcct ccgatgccgc agcatccacc ccacacctta aacctcatgg tattagtggg 480

caatttaaaa gataaagaca cagggaagcg ggactaattg ggaaaacctg cagacatttg 540

ttttaatgcg taatctgcta aataactacg ggggtggggg tggggaagga agagatccaa 600

ggaggcagaa ggctgcggtc aaaatatttt ggggtggcag agtcacgtag gatgtggctg 660

tgggttctgg cagcccagag attcagctcc cgcctcctcc ctcagagcga gtccatagct 720

accctcacgt cccccgtggc ggtcctcgcc acgctccgga gcgggttacc catgagggtg 780

ctagacctgg gcagcgggaa cctcgaagag gtggagattg caggctggga ctccagattt 840

cgggcaggga tgcggggaag ggaagacgcc tcgctggagg cggaatggag ggcaaggcga 900

aggaggatgg tgcaggaaac ggcgacaagg cgcccggcca ggcccgcgag ctaccgagac 960

ccgggttcca atcctccccc cttccgcaaa cgcccgggtt cgaggtacct ggcgggcaag 1020

ggccgcagcg gagcgaagcg ggctggccat ggggaggctg cggggacgcg gggctgcaga 1080

gagcggcagt ggcacggagc gcgcggctgg aagcgaaagc aggcggtgtg gccaagcccc 1140

ggcgcacggc ccatagggcg ctgggtacca cgacctgggg ccgcgcgcca gggccaggcg 1200

cagggtacga cgcaacccct ccagcatccc ttggggagga gcctccaacc gtctcgtccc 1260

agtctgtctg cagtcgctaa aaccgaagcg gttgtccctg tcaccggggt cgcttgcgga 1320

ggcccgagaa tgcgcgccac gaacgagcgc ctttccaagc gcagatattt cgcgagcatc 1380
cttgtttatt aaacaacctc taggtgaatg gccgggaagc gcccctcggt caaggctaag 1440
gaaacctcgg agaaactaca ttagggcagc ttttccaccg actccaaatc caactgacaa 1500
aaagcagttt ctgccctcga gagtttgcgg gcggggattg acatttgtgc gtctgctctt 1560
gtctgccact gaccgctatg tgcaaactga agggggagaa cgtgaatcca gcttttagat 1620
ttccctgcgc cacctaccca aaccgaattt gtaactcggg gtgttatggg gctaccaggc 1680
tcgcattccc taagggccat ttctgcccaa agatctcaat gcctttcatc gttttcaggc 1740
aaagcagacc atcaagagct ccaatcatac tgttttcata gttttccgat gtaggctcgt 1800
gatcgcaata tttagaaaga ggactggaaa agtgatgtta gaagtactat tcggtttaga 1860
aagggaaagg aggattggaa tagctattgt cttatatgca gtgttcgcct ggggcaacgt 1920
cagcctaaat tatgagcctt cctggttttt aaattaatag gaagtggtaa ctggggctga 1980
cttgatcttg gaaagagggg gagggcagtt tattctgggt gaaagcggtt aaatccggtt 2040
tggtttttta aatggtttca tacaacgcta ctgataatat actgtagctc taatcttatc 2100
aactcagaaa acctacactt ttcctctcct ttatacaagg cacagaaagg cctcttacgc 2160
tggggtgggg tcccaagctc caaagaccac agagtccagg caggtcacgt accaccatag 2220
agcggcgagt gtccctggaa gtccagggtc gcttataaga taagttttgt ccttgttgtt 2280
ttgagacgga gtctcgctct gtcgcccagg ctggagtgca gtggcgcgat ctcgtctaat 2340
tgcaacatcc gcctccccgg ttcaagcaat tctcccatct cagcctcccc agtagccggg 2400
actacaggcc tgcgccacca cgccgggcta atttttgtat tttttgtaga gaccgggttt 2460
tgctatgttg cccaggctgc tctcaaactc ctggactcaa gccacccacc tatctcagcc 2520
tcccaaagtg ctaggattac aggcgtgagc cacggcgccc ggcctccatc tgtattaact 2580
gcttctattt cctccccatt aagggcttct gtccaattat tccacctaaa taaggtctct 2640
aatagccttc attttgttcc tgccaatggt tttgcttctc gtgcattttc atggctgcac 2700
ctatgtgctg atgactccca aatatatttt ttcagtccat ctgtctcctg agcagtaggt 2760
acttgctact caaaaatctg tctaaaataa aaacggtgta tctatccacc atgttcgaag 2820
cactgggcta gttgctgggg gagggttgag agcataccat catcctgtta tcatgcattg 2880
cccccagcca gagagaaaag tgttaattgt gttagaaagt gttaaggaat cccacagaca 2940
gatgtaaaat tataacaagt gctgcctgtg ctctaagagc ctctaataat ggattgtatt 3000
gactcagaaa ggcctggaaa ggctttcagc aagtgatcct tgagcagaaa tctgaaagat 3060
taaagaaatt tactaaagga agaagggatg aagagcacct ggtatgaaag tgagttccag 3120
gacaaaagaa cttctctgag gagggctgga acaaagggcc aaaagagagc ctggggctgc 3180
ggtagacaga agaggatgga gcttgggcta gtgagtagcc aaagggccag ccaagtagtg 3240
ccttagggag agctaaagaa taattttaaa aagcaaaaat aaggtaaaag cctgactgtc 3300
atacaggtat aaaataaact aaattataga gtttatttaa atcatttaat gaagttacca 3360

ggcagatata aaaactggtt caaagaacag gcatgtttat aggtcagaaa tattgaaatg 3420
aatatgcaaa tgaaattggc ttcttccaca gtaggggaga gaagttaatt gaaccctgac 3480
cttcagattt tacttgcatg actgcatgca ataattattt tgcatttcta tcagtcatct 3540
gtaaaataac tcaaaaacaa tgaaacaatg taagacccaa tgaaagggcc catggaatca 3600
gaatcagata accttaaagg tttgctctaa ataattcagt tttccattga agtacaaatt 3660
tttccctaca gtacggtaat ataatttctt cattcaagaa gtgctattag tcagcaacag 3720
ctgaagtaaa ccagacatag tagtcactgt acttactagt tacactaaga agctgtagct 3780
ttagcagttt tcaatttagc ttaacctagg ggcaagagaa accattgaaa ggttaaggat 3840
taggtggagt atgcaggagc ggtgacagaa ttgaaggtat tggctgcagt ttggagattg 3900
gaagagggtc tgggtggaga ggaatggatt ttgaagaagc tattacagaa cccacatacg 3960
agatgatcct tttaataagt cagatttggc tggtggttgg taatagaagt ggagagaaac 4020
agccagattt cagcaagaat ggagataagg tggcagcctg ggcaacatgg caaaaccctg 4080
tctctggtaa aaatacaaaa attagccggg ctcggtggca tgtgcctgta gtcccagcta 4140
ctcacgggat taaggtgaga ggattacctg aacttggtga ggttgaggct gcagtgggcc 4200
aggatcacac cactgcactt gcctgggtga cacagtgaga caccatctca aaaaaaaaga 4260
aaaagaaaaa tagatgatat aaggtgggac aaggatgaga gaagtgccaa ggaggcctcc 4320
ttggatctga cttgcttatc tatttggtga tgttcactaa gaaaaaagga atgttgaaaa 4380
agaatgtgtt tgaggggatg aggaggagtc tggtttggga cctatgattt ctgaagtgtc 4440
tccaagactg gctgtgagag atctgcctgt ctgaagctca gagaagaggt ctggattaga 4500
ggtattattg aatcattggc atagaggtgg taactgaagc tatgggtgcg aaagagaatc 4560
tctatgtcag ttttctccgc tgtaaaatga gacaatagca acacttcatg tttgtcaact 4620
gggatagatg agcattaata tatgaaactt atgtatatta cttagaacca tgcctgatgt 4680
cagccatgca gaataggtga aagaacgggc atggctctgt ttccattaaa ctgtatttga 4740
aaaacgggac agagcaaatg tggtctgtgg actgtaagtt gcttactcct gctctatgta 4800
gttgctctag tagaaacata gcagttgtcc ctcaaatgca tccaagtgat gatcaccaat 4860
gtctatccat tcttgctcca aataccctga cagttgccat ttctccaacc atgttatccg 4920
catcctagta ccaaacagct tccaagcagc tcctctgacc ccagccttgc ttctctttaa 4980
gccattctta gcatgacaac cagtgaaaat gtctcaaacg caaatctgaa gacatctcct 5040
cttggtcaaa aaaccttcaa ggcttcacat cacccatttg acaaaatgat tggcaagctc 5100
ccagcccatc ccagattctg ccttctgcct ctgactctcc agctcacctt ttgctaggct 5160
ctttctttct ctctcccccc acacatttaa tttatttcag tcctttcgga catcaggcct 5220
tctcacaggt gcccaaatac ctgtaacatt ccctgccttt gccaggcact gtcatccttc 5280
cttgttcagt ccctgtgcca tgtccttggc aggaacgtct ctgaaccctg tctaggctac 5340
agtgggttcc tctgctgttt atttacatac aatgcttgcc atttcaaaat aatggtaata 5400

ttttattcta atcatttgat taatgctctt ctaattttat gatgggaagt tcatgtctgt 5460
caggcctctg agcccaagct aatccatcat atcccctgtg acctgcacgt atacatccag 5520
atggcctgaa gcaactgaag atcaacaaaa gtgaaaatag caggttcctg ccttaactga 5580
tgacattcca ctgttgtgat ttgttcctgc cccaccctaa ccgatcaatt gactttgtga 5640
caatacaccc tccctgccct tgcgataatg tactttgtga tatttcccca cccttgtgaa 5700
tgcactttgt atgatacacc ctccccaccc ttgagaaggt actttgtaat atccttccct 5760
gcccttaagg tactttgtaa tattctcctc gcccttgaga atgtactttg taagatccat 5820
cccctgccca caaaaaattg ttcctactcc accgcctatc ccaaacctct aagaactaat 5880
tataatccca ccaccctttg ctgactctct tttcagactc ggcccgcctg tacccaggtg 5940
attaaaaagc tttattgctc acacaaagcc tgtttggtgg tctcttcaca cgaacacaca 6000
tgacaatgcc tatattattc acctagccat cccacctcac ccagtgccgg acatgctgtg 6060
gagagttaaa aatctgttga actgcagctc tctaatattt tagaaagcat gtacttaata 6120
tactttggaa ttttaactgc caaagtttag aatataatag actaacatgt tctcagggtc 6180
cactccactc ctagatatga gcttcttttc ttcatagaac tgctctccct caccctaaat 6240
ctttcttctt cctttttttcc tattttctgc cttcctctac tcgttgtctt cccttgtaca 6300
gtcataataa ggactttata tgcattgtga ctgttcaaag agattggatc aatgctttcc 6360
agtagaactt tctgtgctga tggaaacgtt ctatatctgc caggtagcca ctaagccaca 6420
tgtagctact gagcacttca agtgtggtta gtgtgattga gaaaccgaat ttttaatctt 6480
gtttcatttt aatttatatt taaatgtaaa tagctgggcc gggcatggtg gctcacacct 6540
gtaatccccc cactttagga gggtgaggca agtgggcagg tcacttgagc tcaggagttc 6600
aagaccagcc tgggcaacat ggtgaaactc acctctacca agaacacaac aaattagttg 6660
ggcgtggtgc catacacctg tggtcccagc tacttgggag gctgagatga gaggatcact 6720
tgagcccagg aggtggaggt tgcagtgagc caagatcaca ccactccact ccagcctagg 6780
tgacagagca agaccctgtc ttaaaaaaaa taataaacat taaaaatttt ttcaaatgta 6840
aatagctaac tacatggggc tacagtctga gcaccggttt gggcattgaa gtcccttggt 6900
tgagattcgt tatcctggtt ctatcactta taagccctat gacttttggc aaattactaa 6960
cctcttttga gtctcatatt taaactcaga gtaagtaatg ggcagtgacg acactcaaca 7020
tgaagatatc tataaaggac ttagcagtac ttagcactta gtaactgctc agtacatgtt 7080
agctagtgtt atataccatg ttttaatttt tttaagaccc atttattttt cagttatatg 7140
tagcccttgg attacggtat actgatgatt aggatattat gtgagagaaa ccttaaaaat 7200
atacatctag tgtttctagg tttttgtttg tttaataaag tttctttaaa aaaaataagg 7260
cccagtttgg ttcagaaaac aaactgcaca gctattattt acatgcttcc tggttggtat 7320
tgggaagaca atgacagatg agtataattc ctacctgcca aaccctcccc ttactcctgc 7380
catgtcacgt ctctggaaaa gtgagccata aaaaaatctt gacaaaagaa ttctcatttg 7440

ggcttattca atgccaagtc ccattcaacg aagtaaaatg agcaattcta tgtactgtgt 7500
atgtagttac tgtatcattt gcctaggacg gtagtaacaa agtgccacaa actgggtagc 7560
ttaaacccac agaaagtgat tgtctcatag ttccagaggt gaggtgtgga caggcccagc 7620
ttcctttgga acctgtaggg gagaatccta cctttcttct tctagcttct ggtaacccag 7680
gtgtttcttg gcttgcagct gccaacactt caatctctcc ttctgttgca ttgtgttttc 7740
ccttcctgtc tgtctgcttc ctgtaactgc tttgattaat ggactatgga gaaatatgga 7800
agtgacactg ttctaagact aagcatatac actaactggc caggcagttt ctgctttctt 7860
cctcttggaa ggatcttgga actaccccac ctagaaccca gtcaccccag ctataaatag 7920
cccaaatcac atggaggagc cacgtgcagg ctcattagtc aacagtgcaa catccagcat 7980
cactgtcagc cctgtgagtg gctgtcttaa acatccagcc cagtcaagcc ttcagatgat 8040
gccggcccct catgactgat taaaaacaca tgagagacca taaggctgac tcagactgct 8100
cagccgggcc caggcaatct agagaccatg agagataaaa ataaattatt gttggccatg 8160
cacagtggct cacacctgta atcccagcac tttgggaggc caaagcaggc atatcacctg 8220
agatcaggag ttcgagacca acctggccaa catggtgaaa ccccgtctct actaaaaata 8280
aaggaaaaat tagctgggct tgatggcggg tgcctgtaat cccagctcta cgggaggctg 8340
aggcaggagg atcacttgaa cccaggaggt ggaggttgca gtgagccgag atcatgccgc 8400
tgcactccag cctgggtgac atagcgagac tctgtctcaa aaataaataa ataaataaat 8460
aaataaataa ataaataaat aaataaatta ttgttttaag gccacaaagt ttggggtggt 8520
tggttatata gcaatgttaa ccaaagcagc atgcaaacct aacatgctta tttcattatt 8580
tatttaaaaa cctaataata ataaatcatg cccaaaaata gggataaaag ggtgagataa 8640
acatgaaata agtaatcctt taaaatatct ttctaactat ggttatttta ttgtaccagc 8700
cctagctaaa ggcccagtat acacaagggc caagtaatca gtaatgacag tgatacaaat 8760
ccttctttta aagtcttctt gatgatcaaa attgtgtcac ccactatttg tcagttccga 8820
tgaaatcatt gttgtttttt taatctcatg atatggttaa tggagaactt ataataagaa 8880
ctggggccag gcacagtggc acacgcctgt aatcccagca ctttgggagg ccaaggcggc 8940
cgatcacctg aggtcaggag tttgagacca gcctggccaa catggtaaaa ccctgtcttt 9000
actaaaaata caaaaattag ctgggcatgg tggcgagtgc ctgtaaaccc agctactcag 9060
gaggctgaga catgagaatt gtgtgaacct gggaggtgga ggttgcagtg agccaagatc 9120
gcactactga ctccagcctg gtgacagagt gagactgtct taaaaaaaaa aaaaaattga 9180
aaaaatatta gttatcatga aaaacatgac acatttcctt gttttacatt actaatattt 9240
tcattattat tctggcttta tccttttaaa tttttattta tgtttgttta gagttttttt 9300
tgcagacatc tcttaaagcc aatgtccctt ttgcaaagat tatcagttaa caccagatga 9360
cataatctgt aactctcctt aaccaggctc acacagaccg ccacaaatca caaccctctg 9420
gaagagaatg aatgcgcagg gctacacagg caaccccctg ggagcaggag ctatgccctc 9480

```
ctctcccctc aggaccctgc tcgaagtttg tgacagcgtg gcagggtgac acacagaatg      9540
agcacgtgcc aatctatgtg agttaaatac aaatacagtt taatctttcc cacatgttag      9600
aggaaaaata aacataagtt cttctgacat tccagtggag tctgttgcat atctcaatat      9660
atacacacca cgatgaaact cactctgcga gcagacaaag tgcaaattcc tgagcttagt      9720
ctgtgggctc ctgcacttgc tgaatcacct gactccctct tccctcccca cccctcgtcc      9780
tcatttgcac ttcccatctt cttctgcaca gggaggaaac cctaagcgtg gcaagcctct      9840
aggtcatctc caggtacctt aaaaaggagt gacagatgga cagagagaca aacacatgaa      9900
gattctggat aaaaagatta ggaatttcat ttcctgtgtg gaaaacaatt aagcttataa      9960
ttttgcgttt tacagaaaca gaatcactta acttctgaaa ggagaaatta atcctaatta     10020
aatgaggctg ctttttaaa atccagatat tatatactgg attgctttgg agaaaatttt     10080
gttttatacc agtacctaaa tagcttttaa gagttcaggt taacctatgc tgaggaaatt     10140
aatagcaaaa agaaaaggcc acaatcaaga cggaaaggat ttaagtttta ttaatgatta     10200
ttaagtgcat tatttatagt agaatccaca acatatgctc acgaaaataa accagttcta     10260
gtaaatacat gataaatata aaaaattaga agagggctgg gcgcagtggc tcacacctgt     10320
aatcccagca ctttgggagg ccgaggcggg cggatcacga ggtcaggaga ttgagaccat     10380
cctgtctaac acggtgaaac cccgtctcta ccaaaaaaga aatacaaaaa aattagccgg     10440
gcgtagtggt gggcacctgt agtcccagct actcgggagg ctgaggcagg agaatggcgt     10500
gaaccaggga ggcagagctt gcagtgagcc aagattgcgc cactgcactc cagcctgggt     10560
gacagagtga gactccatct cagagaagag aaatggttga tgcctgacaa tgagcaatat     10620
acatacaccc aaaggagaaa atggggccgg gcgtgatggc ttatgtctgt aatcacagca     10680
ctttgggaag ctgaggtgag aggattgctt gagtccagaa gcttgagact agcctggaca     10740
acacagtgag accccatctc taaaaaaaaa aaaaaaaaat ggagaacgtg ggtatttggg     10800
aagacagtaa tcttttggaa aaagtgtttt tcctatgaat gtgatatatg ttcaagaaaa     10860
tagactgact tatctgatat ataccttgtg gcagcaaagg gaatacttcc ccatcacttt     10920
cttcagaagg ttgctcaaaa tcattgacaa ggggcagatt aataggagaa aagtcataca     10980
aatttatttg atcataattt tatgtgacac gagagcctac agaacgaaga cccaaagata     11040
taagggaaac tgtccatttt tatggtgaaa ccctgtcttt acaaaaatac aaaaatttta     11100
atggacagtt tttgttttgt tttgtttgtt tttttaagag caaggagagt atgcttttta     11160
aatgccattg gttcatgtgc cacagaacct aaaacagctt caaatggaca ccaagtaaaa     11220
aataccagtt ttcagaagtc tgtcattatg tatttacaca aattacataa tcctgtatgt     11280
atttacaatt acagattatc aagtagataa cacaaagttg tagtgttaat gagacaaaat     11340
agaataaaaa caccacaagg aagccatttg cttatctacc aagcaggacc tagcaaggcc     11400
gatcctggca tgtgcttcaa cattgcttgg ggacatttat gtgacagatg ggaagatttg     11460
ttcagtctgt gctaggtaag tctttgacca cagtgcaaac gttggcacca agtagatggt     11520
```

agtttggctg tctgagtatc tatttgtgaa actggaaccc cccttccacg atggctcagg 11580
gttgccatcc aacaggcttt gggcctttta caacaagaac tggatgcaaa cccatgagct 11640
ggggatgact cactctattc cccaacagac aatgcccagg ccaaaaccct gagcccctt 11700
aggttaggtt aaacaaagta tggagagcca cgtagaaata tgattggaca aaaaaggtat 11760
ggtctaatgc taatagactg agcagggaaa tccagcacag cctgtctgtc tggacccttc 11820
ttgcctctct gagcatgcat ttcttccttc tgggtgtggt gctggaccct ctctggaatg 11880
ggggtcttat aaccttcagt caaacaaagt gagtcagata atttctttat ggccttacac 11940
agacaggctg ggggtggtgg aagttagagt actattttta ggttttatgg ttggctttgg 12000
ggaaaagggg ttttgtttct atggcccact ttggggaaga ggggttgtag tcttgatggc 12060
tagcctcaag ggagaatgaa aggtcagaga caggagagca ggagcccaac attccccagt 12120
tgagaaattg ggttggtaga ttatcttttt tttttttttt tgagatgggg tctcactgta 12180
ttgcccaggc tagagtgcag tgtcataatc tcagctcact gcaagctctg cctcccaggc 12240
tcaggcaatc ctcccacatc cacctcccga ggagctggga ccacaggcac atgctactat 12300
gcccggctaa tttttatat ttttggtaga gacagggttt caccatgttg ctctggctgg 12360
tctcgaactc ctgagctcaa gcaatttgcc cacctcagcc tcccaaagtg ctgagattac 12420
aggtgtgaac tactgtgccc agcgggtgga ttattttata agccattgaa ctagtcttgc 12480
agtcgtgaga acaggccgct ccaattaaac ggttaacagt tatatctcat ttcaggcagt 12540
ggtgttgctt ccaccaaagt caggcctcta tgaatcaagc aatcagatgt ttaataaaag 12600
gcatttccat gaaaacaaaa gaaaaacaaa gattaacatc tggagttgtc tataaactaa 12660
ttttcctaga gtctctgaag tagcttcaga ttgcagtggc aatcttacag atatttctgg 12720
attatagttt gaatcaggtg ttcaagtaaa ccttctgagt aatccataca tcagctgaca 12780
tgaacactgc tcatatatta agttgctgtc attatttctt ccaaagttta tatcaagttg 12840
tctagctaaa gcctgcaggg cttggtgatt ccaagacaga aaaatggtag aaaaattagg 12900
aaacattagt ttggagactt gtacccagga aggaattcag gattcagccc aaattgtagg 12960
caaataacaa aaactcaaaa aaacaattat caagactaga atctaataac aagtatggta 13020
taattttctt ctgaaatata attttctctt ctacagtcat cccaactttt acccacaaag 13080
ataataataa taaaactaat ttatttgcaa agtcagttta gtctctggca tgattatctg 13140
cttaaagtgc agtaagaatg gtgatttatc atgaaggctc tttttttttt gctttgatat 13200
tttttattta ttataaaaac tgagttttca acaaaggcag tttagcagga ctatagttgt 13260
gaaaataaat ctgaatgtag ccattcttta aacttaaact aaaaatcatt gtagaataaa 13320
atgtcaagca agtgaaaact tttctgtgat atatacagaa atgatataga cataaatatc 13380
ttcacataaa caaaagcaaa gatcaagaaa aaaattaaat atcttggtgg gcaagagaaa 13440
tacacagatt aaaaaggtta tttttatttt acttcaatgc ttctattgag cacgcctgtc 13500
agagcaatag gaattagata aatctttaca tttcttcagg gaattacata caataaagac 13560

acctctctag aagaaaatat attagcatca ttagactcct gaaagtcatg actttcaatt 13620
aagttacact ttttgctcta ccgtgaagct acatgcttta tcagaatttt gccagttgaa 13680
agaaaataaa gctaaccctg gtaagatcca gcacagacac aggtggcagc aaattaggca 13740
caatgatgtc tggattttcc tctcaaagtg gattacccat gcctaggaga taacggcttg 13800
caacgcaaaa caaacattgt ggcataaaac agacgatatt taaatagata tattttctac 13860
agggatggct cttaagttgg ctttgttgga attttttcat aaggaatctc agattaagac 13920
ttttgaaacc tctcaagggc tgggcacagt ggctcacgcc tgtaatccca gcactttggg 13980
aggctgaggt gggtggatca cctgaggtca ggagttcaag accaccctgg gcaacatggt 14040
gaaacctcat ctctaccaaa aatacaaaaa attagctggg agtagcaaca catgcctgta 14100
atcccagcta ctcgggaggc tgaagcagga gaatcacttg aatccaggaa gcagaggttt 14160
cagtgagcca agatctcacc actgcactcc agaatgggtg acagagtgag attccgtgtc 14220
aaaaaaacaa actcttgaag caatgaagcc aagccaaaga ttcgccatca gactgtgcct 14280
gtaaaacctg tatgaattgg gtgaattgct ctcttttcaa cgtctccaaa atatcttgag 14340
gtccctgggc ctgtcagaaa gtgacattct ttacttattg caaagttaga aacgctataa 14400
aggaattgtg tgggcaaggt accaggtgtg tctttttcca agtctactgg ctgtataaag 14460
tcaacctcaa tccctcgaag cagtccggtt gcactcaaaa aaaagacatt ccagtcaaag 14520
ccttggtaaa ataaccactg tttccatgtg tccagttaca aaagaaaata agctcttttt 14580
ttgtttgttt gctttttgtt tttttgtttg tttgttttga gatagagtct cgctcttgtc 14640
ccctaggctg gagcgcagtg gtgcgatctc ggctcactgc aacctctgcc acccaaggtt 14700
caagctattc tcctgcctca gcctcccgag tagctgggat tacaggcgcc tgccaccgcg 14760
cctggttaat ctttgtagtt ttagtagaga cggggtttca ccatcttggc caggctgttc 14820
tcaaactcct gaccttgtga tccaccggcc tcgccctccc aaggtgctgg gattacaggc 14880
atgagccacc atgcacagcc gaaaataggt tcttattgaa cttatgcaaa taactatatt 14940
gccagaaaat aagaacacta atgaatagtt tccaaattct gaagaattca ggtagaaaga 15000
aaggtaaatg tttccatttt gctcacagaa gtatacttta ccccaatgct gtaagctata 15060
aatagctcaa aagaaaaaaa atattttctt gagtctggaa aacaaaacat aaaaaaaatc 15120
agtaatgttt caaacaaaaa ccctttaaaa tagtaatttc aatccttcat tcactcagtc 15180
ccatgtaatt ctcgttctcc ttgatgttgg gttagcaatc tgcatgaatg catcagtttt 15240
tcattagagc tttggacttt tttttttttt tttttttttt ttgaggctgg agtgcagtgg 15300
cgcgatctcg gctcactgca agctccgtct tgccgcttcg cgccattctt ccgcctcagc 15360
ctgccgggta gctggaacta caggcgcccg ccaccacgcc cggctaattt tgtttttgtg 15420
tttttagtag agatggggtt tcaccgtgtt agccaggatg gtctcgatct cctgacctcg 15480
tgatccgcct gccttggcct cccaaagtgc tgggattaca ggcgtgagcc actgcgccca 15540
gacgagtttt ggatgttttt acctagtcca aaggtgtgat ctccaaagtt atcagaaacc 15600

tgtatttaag actacttgtc aaggtccctt tcatgaattt ccttgaatac acagcacttc 15660
aggatttgca aaaggctttt agaaaaaaaa atcagaataa agcaatttac tgcatatacc 15720
atgacatatc agccttttat ttttattttc actttttatt tttggagtaa tgtggaactt 15780
tttaatttgg aaggcaaaag gttacagtta attgaaggca gaagtcaggt taataaatgt 15840
tacaaagttg ttctgacaga gagagggaac ttctctgggc tctcctccac accaaatcag 15900
ttggtagtaa gcacaaattt gaagaaaatt catgtgtcaa acaagctgcc atctcaagaa 15960
ctcttaactc ttatccagga aaatcaaagt gagcattgtt ccagtctctt actcttcaat 16020
taagtaaatg ggaatgattc agccaacaaa gttcatgacg ataaggtaca agatggtgct 16080
agcaaagaga aagaagcaaa gtctcactcc aaggagatgc tttcgaagtc cactttgttc 16140
tgtgggttca cctgtattct caggcaaact actaggatga aactccccac ccaagatgtg 16200
aaacccaaga ggaaagagtt gaaggggaag gtccccatga gaagacagta agtaaactgc 16260
agcgccctag tcagcagttt atacagcagg tatatatcca gcaacttcag acactgcaga 16320
gtagagctaa gtactcttct aagaaccatc gggtgcctga cactaccaac gctgacatga 16380
atggatgcaa ggtaaccggc cagtgctccc taggctcata gaggacccaa caaccacact 16440
ggatgtccat atcagatttt taggaatctc atacaatgtt ggaacacata ttaacaacat 16500
atccatataa atataactca aagaaagtgt aacaccattt cttatttaac aacacttcct 16560
gtatgatttt aacataccaa ataagcctca tgtctctctt ggacttccag gggtcctatt 16620
tattattaat aatatatgtt aatgtattat agtattatgt tcaagttagt taagggcaaa 16680
aatacttaat tttagaatat gaaatttgat tttcagaagt atatcatata tcaaaagttt 16740
aaaacccttg ctatcaaaat aaagatttaa gacctatgtt caaaatagaa tcgcaagtca 16800
ctgtgacctg atcgaggacc tgggaactga ctggtttcct aacacattcc tgtcctgtgt 16860
acttctctgg aaagtcttcc tgtatcccca agagcacaag ttttgcatgg tagagatgct 16920
agtttaacat tgcatgtaat agactagtta tgacacttga ttattaccct gcttgtttat 16980
gtgtcagcct ccaactgtct aggcagtgac ttcattctct ttgggtttcc agtgcttagt 17040
acacagtcag ataaatgtgt gagcctcgat aaatatctac tcaataaatt gattgaatat 17100
attaatctac catgtttttg cttaaattaa aaattgatca atgggctgat atattttata 17160
acacatgcat acacacataa gtagagacag aaagagagac agagcagaaa atagatacca 17220
gcataaagtc aattgtgtta ggcacaaaag aaccaaatta gattttaaat atggctattt 17280
attcaggcta cttactgaac tgcctttatc tgtctatgag atactttgcc ttttctccta 17340
acttcctcgc atttctggat agacagtgaa atgtccgcag gacttttccc aaatcttatt 17400
aagccatgag ttctgagctc attagaagat tttgttttaa gctgtagctc cattgtccag 17460
ctgggtgtaa gttctagctt tattgtcaga tagacaatta tctagtatct agtagataaa 17520
gcttgggtaa accacctaat gtctctaaac cccactttaa tgtccctgaa cctcggcctc 17580
ctcccctgta ggagtcaaaa ggaaatgcaa actactgaac agaatatgtc atggttattt 17640

gttctaacat aaggttatca agcttatagg tagaccctat aatataccag taaatcacat 17700
agactctgga gtgacattgg cagatttaat tccagcaata ccacttacta gctttgcaag 17760
gtttgtctaa ttacttactg atatgggtta gctatgtctc cacacaaatc tcatcttgaa 17820
ttgtagctcc acgtgttgtg ggagggaccc aatggaagat aactgaatca tgggggcggt 17880
ttcccccata tagtttctgt ggtagtgaat aattgtctca tgttacctga tggttttata 17940
aggggaaacc cctttcgctt ggttctcatt ttctgtcttg ccaccaccag gtaagaagtg 18000
ccttttgcct tctgccatga ttgtgaggcc tcccagccat gtggaaatgt gagtccatta 18060
aacctctttt tctgtataaa ttaccctgtc ttgggtatgt ctttattagc agcataaaaa 18120
tggactaata cacttaccct ctctgtgctt ccatttctct atctgtaaaa taatattaat 18180
aatagagttt tatgagaact aaatgagata aatcatgtaa agcctatttc agcatccagg 18240
aagatctcaa tctgtgtcag ttataattgt taagttgaag attcatattg agtctttaga 18300
ccttgtgagg taatatggca tgtttggaag ctgcgttatt tataatttta gagagaagaa 18360
tgaatccaca ggaactccac ctctctgcaa accgcactct gtggcatggc ttaattagag 18420
gcctgctgca gggctgagaa gtaatgcttg accttagatg ctccataaat cctcttggca 18480
aactgaccac tccagatttt tatttttatt ttcatttttt atttttgggg tagcatattt 18540
tatttttgga gtgtttctac cactatacta agaggggctg tttcccgaaa aactctcaaa 18600
ccatttttcc tctgctctca cacaaaaaca atcaacacgg aagacttctg gaccccaaaa 18660
tatataggga tttctcccca gcaggaagca agcaatcagt tctgcagtgg acacaagcta 18720
ggtgtcctcc aattcagctc caacactgtc tacccagaga tagcatcaga tccacaagtt 18780
gagggctcag tcccacaaga gcaccccgtc cttccaacca gtcataagtt caggtctctg 18840
gaactgctga ccaacaggtt tcaagttggg gttgccatga ccccctcttc cggtttgatt 18900
aatttgctag cgtggttcac agaactgagg aagacactta catctaccag tttatcactc 18960
aggatatttt ttaaaataca aataaaagcc aatgaagaga tacaaggtcc aagtctggaa 19020
gggttctgag cacaggagct tttgtcctcg tggagttggg atgtgccacc ctcccagcat 19080
gtgaatgaat ttttgcttac cttcccgtga ccctccacgt gttcagcaca ggaggctcac 19140
gggaaggtaa aaaacacttc ttagaagctc cccagtccat tccctttgga ttttcatgga 19200
ggcttcatta cataggcatg actgattaaa ccactggcca ttggtgatca acttgaccag 19260
ccccactctg aagctatcag tcaacactaa tatacaaaaa gacagcactt tggagattcc 19320
aaggattttt agtacttgta tgccaggaaa caggaacaaa gaccacatat atgtatttca 19380
caatatcaca gggacactgg ctttttttag gcctttcctc agatcgctag agcagcatac 19440
aggaaaacca gttttccact agggttcctt agatgttcag acttggtgtg atcctccagt 19500
gagttaaaac atacctcctc caaaagtctg attcatttat ctctctactt agaaaaaact 19560
gctatggaaa ctctttttca ccaatgatga tgacatacat ttttcccagt aatgtaagcc 19620
caatatatgc cagtctcaat ttacaagaca gatacatcat aggatctttg atacacatac 19680

ctaggaagat ctttgaatta cttaataaaa cattcaggaa tattccctcc accccagagc 19740
tgacaaggaa caacagttat agaaaaatag ttgtagaggc agtgaatatt taagatgggt 19800
tggtaccttt tgttttaata gcttccatta gtcacaactt gataaactgt aacattcctt 19860
tctaaatctg cttcctgctc cttcctcacc tctgtcattt tctgcagcag aatatgtagt 19920
acacattggc tctataaatt gccaggtgca catcaaagga aagcatgaaa aaaatagtaa 19980
tgaaaaagta aacctcccaa aggacttcgt cgttagataa gaaaactagc acacatgtgc 20040
acacatacac acatacacac acacatacac actttaatga aagaactagg ggtcctgata 20100
agctggcaca taaacctgtt gttcccatca aatgaataat caaagatttg agagttaatt 20160
tgagtgactg tgtcagtatt attccaacaa agacatgagc acacacgcaa acacaagatt 20220
gttccttttt tttgagacag actctcatta cattgtccag gctggagtcc agtggctatt 20280
gatagacgtg atcatagcgt actacagcct tgaactccta ggctcaagcg atccacctgc 20340
ctcagtcccc caagtagctg ggactacagg catgtgccac cttgcccagc tacaagatgg 20400
tttttacctg aagagttaat atgatccgta ataataacca cccactgtat gactcatcct 20460
acatccggcc cctagtttca cacatatttc accatatttt tcatacttaa aacaacccta 20520
taaggtagat atcatcatta ttatagcaac ccattttaca tatgagaaaa ttggaagcag 20580
tttggtgatt ctctaagttg attcacagga ctcaatactt tcgactataa tttattacag 20640
gataaaaatt atggccaggc acagtggccc aggcctataa ttccagcact ttgggaggcc 20700
gaggcaggcg gatcacgagg tcaggagatc aagaccatcc tggccaacat ggtgaaaccc 20760
cgtctctact aaaaatacaa aaattagccg gtgtggcagc gcgcgcctgt agtcccagct 20820
actcgggagg ctgaggcagg agaattgctt gaatccagga ggcagaggct gcagtgagcc 20880
gacatggggc cactgaactc cagtctgggc aacagagtaa gactctgtct ctcaaaaaaa 20940
aaaaaaaaaa tttacaaggc aaaaatcagc aaaaggaaaa ggcacatggg acaaaggaaa 21000
tcagacatgt gcttcccaga gtcctctccc agtggagtca cacaggatgc acttaactcc 21060
tccagcaatg agttgtgata atatgtgtaa aatattactt actagggaat ctcattagac 21120
actcagtgcc caaggttttt atgtggggct gtcatgtaaa cacccctacc tagcacatcc 21180
taaaattcca gactcccaga aggaagcaga tgttcagcag aaacttcatt gtttgtacaa 21240
atattttagg catcgtgagc cattcttttc agggaatggt gagaaccctc cagaaattca 21300
agttcccaga caccagacaa gggccaactt tgcaagcctt tctaagacta gcagcctcag 21360
gcctgcttta attcttttct acacaggagt agactgagaa gccttggtca actgtgcaga 21420
agcagaggac ggggctgtca ctgagcaagg gacatgaagc attcaagtgt gtgtatatat 21480
atattttaga cagggtctct ctctgtcacc caggctccca ggctggagtg cagtggcaca 21540
atctcagatc actgcaacct ctgcctccca ggattctcct gcctcaacct cctgagtagc 21600
tgggactaca ggcgtgcacc accatgcctg gctaattttt tgtttgtttt ttgtagagtt 21660
gaggttttgc catgttgccc agcctggtct caaactcctg agcccaagcg atcctcccac 21720

ctcggcttcc catagtgctg gaattacagg cataagccac catgcccggc cttcaagtat 21780
atttcttatg ctcaaatcag agagaaccaa actgagtatc aagaagattg attgccttga 21840
ccaaagtaac acacctgtct gcagagggag agctggtact agaacccagg tcttcagacc 21900
acacagggca attaacaaat atcagagtga aaagcagaat ctgctcagtc catccccttc 21960
tctgccatta gtgaagatac tgatcaatta atttaatttt tattcataca ctataaggat 22020
ttattaaaaa tcaaacaaaa caaaacaata caagttgatt tttcacaggt ggctttttaa 22080
tctgcatgat ggcataatta cagcactaga cattttcaac cgattatgtc gataatatac 22140
attctcgttt cctccctttt tttcccctca gctttctgac ccatcctccc ttttctagag 22200
tgagaagacc ataaattacc aatgatttat tgaaaaatac tgcctttgag taattcttag 22260
taacttgtaa gaaaataaca gtaacaaatt ataatgagag gagggagaga aagagaaccc 22320
cttctttaca caagaatgct actcaatgcc aagaatgata taattagatc agttaatgct 22380
aaagtcatca gaagaaaggt tgtcagagaa tgggatattt acacagtctc aaagtcttgt 22440
gccacaaatt acatattaat ttaaaaggag aaaatgtccc tttacaagag tgagaaatgg 22500
tggcgaccac cttcaccagc taatcaaact taacatgact aatgcaggaa ccaatgcata 22560
tcatgtgtct ttgatgcagg gcaattagga gtacacaacc tcacttcagt gttattcttg 22620
ccaagaatta gagcctggct ctaatcataa agataaaatt agagaaattc aggatgtgaa 22680
atagccaatg agcctgaatt attcaaaaca atgtcattaa aaaaaatagc agcaaaaaca 22740
cacaaatatt gatccgtatt aaaagaaaca taactataaa gagtaatatg taaacaaaac 22800
aaacacattt tgaggtcagc tagggaaaat tatatatgaa ccagatatga catgatctct 22860
ctgaattaat actaatcttc ttaggtgtgc tatgtaggag aatgtcccat atgaagtatt 22920
tagataggaa atatcacaac cataatttat tttcatatga ttcagtactt gggagaaaga 22980
gaaagcatat agaaaggtta accattgggt gaatccgttg ataaatgacg gattcaaaaa 23040
cagttgtttt ttgaaacagt agttctttca tctctacaga gtgatggtta atttcatgtg 23100
tctgcttggc taagctatag tgcccaattg cttagacaac acaagttcaa atgttgctgt 23160
gaaagtattt tttagatgtg attaccattt aaatcagcag actttgagta aagcagattg 23220
ccctcaatgt gggagggctt catccaatta gttgaaggcc ttaacagcaa aaaagacgga 23280
ggtttcctga agaaggaatt tggcctcagg actgcaattg caatagatac tgtacctgac 23340
ctgccctgca aatttcagac tcacgactgc aacatcaact cttaccagaa tttccagctg 23400
accaacttgc cctatggatt tcagacttgc cagctcccac aatcacatga accaattcct 23460
taaaataaat ctctctctct cgatctcttg atagatagat agaaagatta tagatataga 23520
tatagataaa gatataaata tagatataga tatgccctat tggttctgtt tccctggaga 23580
accctgccta acacaatagg tttggaaatt gctaaataaa aaattgcagc tccccctccc 23640
cctctccctc tccccacggt ctccctctcc ctctctttcc acggtctccc tctgatgcca 23700
agccgaagct ggactgtact gctgccatct ctgctcactg caacctccct gcctgattgt 23760

cctgcctcag cctgccgagt gcctgtgatt gcaggcgcgc gccaccatgc ctgactggtt 23820
ttcgtatttt tttggtggag acggggtttc gctgtgttgg ccgggctggt ctccagctcc 23880
taaccgggag tgatctgcca gcctcggcct cccgaggtgc cgggattgca gacggagtct 23940
cgttcactca gtgctcaatg ttgcccaagc tggagtgtag tggcgtgatc tcggctcgct 24000
acaacctcca cctcccagcc gcctgccttg gcctcccaaa gtgccgagat tgcagcctct 24060
gcccggccgc caccccatct gggaagtgag gagcttctct gcctggctgc ccatcgtctg 24120
ggatgtgagg agcccctctg cctggctgcc cagtctggga agtgaggagc gcctcttccc 24180
ggctgccatc ccgtctagga agtgaggagc gtctctgccc ggccgcccat cgtctgagat 24240
gtggggagcg cctctgccct gctgccccgt ctgggaggtg aggagcgcct ctgcccggcc 24300
gccccgtctg agaagtgagg agcccctcca cccggcagcc gccccatctg agaagtaaag 24360
agcccctccg cccagcagcc accccgtctg ggaagttggg ggcagccccc gcctggccag 24420
ccgccccgtc cgggagggag gttgggggtg cctctgccag gccacccctt ctgggaagtg 24480
aggagcccct ctgcccggcc gccaccccgt ctgggaggtg tacccaacag ctcattgaga 24540
acgggccatg atgacaatgg cggttttgtg gaatagaaaa gggggaaatg tggggaaaag 24600
atagagaaat cagattgttg ctgtgtctgt gtagaaagaa gtagacatag gagactccat 24660
tttgttctgt actaagaaaa attcttctgc cttgggatgc tgttgatcta tgaccttacc 24720
cccaacccgg tgctctctga aacatgtgct gtgtccactc agggttaaat ggattaaggg 24780
cggtgcaaga tgtgctttgt taaacagatg cttgaaggca gcatgctctt taagagtcat 24840
caccactccc taatctcaag tacccaggga cacaaacact gcggaaggcc gcagggtcct 24900
ctgcctagga aaaccagaga cctttgttca ctcgtttatc tgctgacctt ccctccacta 24960
ttgtcctatg accctgccaa atccccctct gcgagaaaca cccaagaatg atcaattaaa 25020
aaaaaaaaaa ctgcagacaa atttttaaaa tcctcacttg tatgttacat gtgggacaga 25080
ataattttaa aaaacacata taatctccaa accccagttc aactataagt tataatcttt 25140
tctccctgat taagatttgt atttgaaaca atagtcattt gctttgagtt aaatttcatc 25200
tcttggatga taatgaaatg agtaaaaatc aagttataag atgtctgtaa tcccggctat 25260
ttgggaaggt gaggtgggag aatcacttga acccaggaga tggaggcagc aatgagacga 25320
gattgtgcca ctgtactcca gcctgggtga caaagtgaga ctctgtctcg aaaaaaaaag 25380
aaaagaaaag aaaagaaaaa aatcaagtta taagagagaa acagaagaat gcaaggtgat 25440
ctacatagac aagctacata gcaatactgg aaaaacctag aatgttattt ttccttaaaa 25500
atctaccagt gactccaatt aaacatggta gactgaacac atgaatcttt ctccacatct 25560
ttttagatat tcacttaaac aaataccatt gtcttttaaa gtttaaacca taagggaaag 25620
agaatgggaa acatgataat gagtgacatt aacacatttt ggaaggtaga aagctgacag 25680
tagagaagga actaactctg tagattagaa agaactgaaa ctgtattgca caaagaagga 25740
gatactgatg agaagccacc tttccctcat ctcagaaagt ctcagaatag agaatccagg 25800

tgtttccaaa ggtgaggctg aaaacaggga cttttgatgc aaatctatat aaccattcat 25860
tgaatgacct tgacttggga gagtacaacc agaaatagtt ttatttttct ctcattactg 25920
cccttcacac ccactttcaa aaatctacca tccctatctg gagaagatca cactgatcct 25980
attgagtttc cagtcacttt taagtattag gaaaatcttg ctgcaagtac ttaaaagacc 26040
ggtatgatct gtaataaaac tttgacctca cattgcattc aatgctttac tgtctgagca 26100
gccaccacca ccagccccca gcaagctgag gttgttaaca tctgtaaaag aaaacaaaat 26160
tcttaaaact ctctaaaatt atttattatg ccaagggaga agttaagtac tggaaactga 26220
gtcacatagc atgtttgcaa ttctgcttct taggttatag attcactctc ttctcatcgt 26280
tctagttctg taaatgacta ggagagacca gagaccagac cttccccctt cactgatctt 26340
tgttatggat taatggatta actgcctcct ttattgtcct gtacctaact cacaccagtt 26400
ggcacaaaag accccatggg atgagcacag tagcccatgc ctgtaatcca ggcactttgg 26460
gaggctgagg caggagaatt gtttgagtcc aggagtttga gaccagcctg ggcaaaaggg 26520
ggatctggtc tctataagaa ataaaaaata aattagccgg gtgtggtggc acatgactgt 26580
ggtcccagct agtcgggaga ctgaggtggt aggatcactt gagcccacaa aatcgaagct 26640
gcagcgagct gtgattgtgc catgccagcc tgcaatacag agcgagacag tctcaaaaaa 26700
aaaaaaaaaa aaccaagaaa aacgaaaata caaacccatg actgttacac cttcagtgtg 26760
aaatgtttgt tgttgttgtt gttgttgttg ttgttgttgt tgttgttgtt gtttgagatg 26820
gagtttcatt cttgttgccc aggctggagt gcaatggtgc aatctcagct caccacaacc 26880
tctgcctccc aggttcaagc aattctcctg cctcagcttc ccaagtagct gggattacag 26940
gcacgtgcac catgcccggc taattttttt tattttattt tattttattg tattgtattt 27000
ttagtagaga aggggtttcc ccatattggc caggctggcc tcgaactcct gacctcagat 27060
gatctgcccg cctcggcctc ccaaagtgct gagattacag gcatgaacca ccgctcccag 27120
cccgtgaaat gttaaatatg cctttctcaa atgaaagtga ccaccttgac taatcagatc 27180
attgtaacta ggcattaagc cttacacaga aagatgttga aattctgtta agcttcccta 27240
aactctctct ctataaacaa tcccaaactt ctaaagttca gaacgctgac ttccattctt 27300
tgggatccgt gtttccctag cactgtcctt aaactttgca cttgaataaa gtatctttaa 27360
actagattct gaccctttg attattttag gttggcacat ccaacagaaa tggggttcct 27420
gtttttttcag cattggagct caggacgcac catcccaaaa tatgaccaga atatgccaca 27480
tcaggatatg cttatttgat gtatttccag ttagttattc tgagacactg tagacacagg 27540
agtagctctg aaaaactgtc tttttgtaaa caaaatttat atctataaag aaaaaaactt 27600
tgataagaat ctcatcaacc agggaagatt ttatcaccag ggagaagatt ggacaggtag 27660
accttgtcac aggctattac ctattcttct gatgggggac tctgagacaa cttttatcac 27720
ctgagagact ttttatctgc ataacaagac aacctatgct tgctatacac ttcctctcct 27780
caccctctta taacctgtca ccacctcccc tcaataagcc ccaagccctg attccttttc 27840

tgtagctcag gaatactata taaacttcaa tcatctggcc tttctttgag tctcatattt 27900
tatgggactc ccatgtgtac gcacaaaata aatttatgtc ttttctccta tttatctttc 27960
tactgtcagt ttacttcata aactcagtta tgaaaagggt aaagaaagtt ttctctctcc 28020
cacatcactt tcctctccat tctccagctg ctgtttccag gacccacggg gttaagtgtg 28080
gaccatgaat tagaggaaat ggttaaaaat ctcatttgtc tgtgaccatt ataatctggc 28140
actgggggcc ctctcctatg gcaaatgccc aaattctggc tttctattgc atcatggtca 28200
cttacggatt cttcacaacc tcccctcccc cttgtcctca cctcctgtgg agaccactgc 28260
cttgcacagc cccactgcaa tgatgcacat tctgtctcac ctctaaggat tctccttcaa 28320
ccccagctct ggcattattc cctacacagc ctctgacccc tggggtctcc ttgccccagc 28380
aagtagcctt cttagacagt gtccttttga ggtgtttcta tgacaccttt cctcagagtt 28440
cacatagttc atgacgtctt tgctctacta aactctggaa ctgcaaactg cacccactga 28500
agacccctct cttgactcta accacaggca gcaccagcca gccttgactg tcccagggca 28560
gagctgacac tgctatgttc cgtacaccag tggcacaagt caagtgttcg caagctacag 28620
atcctctagg caaagcattt ctcacttaat ttttagtgaa tgccctgttg ggggaatggg 28680
agaatcactt ttttacctct tccagggtaa agaagtttcc agaaatgcca agctctcctt 28740
gcttggctga caattttttta tttccaaata atcttatttc ctttctgttc aatttccagc 28800
cttctattaa atataaattg gagataagtt agaggctaaa gcttgcagaa ttagtttagt 28860
catcctttag aaggttcttt agatgtgtca aacgtgtttg gaatttggag gtagtatccc 28920
tccatgctcc gcactgaagc ctcagtggaa cagggccctt tttttttttt tttttttttt 28980
gagatagagt cttgctctgt cacccaggtt ggagtacaat ggcacacaat ctcagctcac 29040
tacaacctcc acctcccagg ttctggtgat tctcctgtct cagcctccca agtagctggg 29100
actacaggag cccaccacca cgcctggcta agtttttgta tttttagtag agacagggtt 29160
tcatcatgtt ggccagactg gtcttgagct cctgacctca ggtgatccac tccccgctca 29220
gccttccaaa gtgctgggat tacaggtgtg agccactgca cctggcccag gatgctattt 29280
ttttaaagga ttaatcagaa gaaatatgaa gagtaaaatt ttaaaattca ggggaaaggc 29340
tgaaagataa cattgatgag atctttctcc cacctcaaaa attggacact aggagagaaa 29400
aattaaatta gaagatcaaa caaggaggtc taatgtcaaa caaatagaag tttaagaatg 29460
agaaaagagg gaaagcagag gagacgaaat catcaaagat ataggaaagg aaaattttct 29520
aggatgaatg actaaggacc catgttatga taaagctccc caaattagca ataaaaagag 29580
accctgaata cctcaaggaa caaaaaacag gttacacata aaaaggacct gaaatacgag 29640
ccccttttgga cttcctatca gtaacacagg aaactagaca acagaaaagc aattccctta 29700
aaattctgac gaaaaatcat ttctaaccta ggatattatt caaaatcaag tattaaccta 29760
gagcaaaagt agaacaaaaa gtcaggcatg taaggaatca aaacatctac ctctgatgca 29820
ctctttatct aaaaacaact agagcatacg tgcaacacaa atgaggaagt aaagtaaaaa 29880

ggagaacgcc acacgattca gaaaaccggg gatgtaacac agagagaggg taaaagatgc 29940
caagaggaag tcttaggcag gcagctgtgc agctggctca gagagaaacc agcacaaatt 30000
agtgcaagaa tgagaatggt agcagaaaga gtacagagac aaaaaagaaa atgaactgtc 30060
agatacattt aacctaagta aaattacatt gagaggctgt caggaagttg ggaattggaa 30120
agaattcata ataaatatat agtaaggtaa gtaaataaaa ggaaataatg caagaattaa 30180
ctgcaggaaa aacaaaatga ggtataaggg gaaaaaaatg taaccatagt acactacttg 30240
actcaccagt gacttttaaa aatggaattc catctactac tcaaaacctc tcaggggcat 30300
ttcaccacac ttagaataaa atccacactt accaggacta aggaacttca atgatgtggc 30360
tccactcctg aaatctcccc tctcagatta tgctctggga agtttcattt gctgttccat 30420
atgcatggga cactcttacc tccagaccat ggtactcagg tgtcagctga aagttcacct 30480
tttctgtgaa gctttccctg accagtcagt ataaatttgt accagctttc tctcagttct 30540
gtcactactg catcatttca ttttattcat attgcatcac tgactgaaaa tatttgatat 30600
gttactatat gaggataaat tgtaataata ttagttaata cttacagtac ttacatagta 30660
cttatttcag accagaccct gttataagtg ctttatattt gtttttggat ctttgttgca 30720
gactgtctcc ttctatcgca caggctggag tgcagtggca caatcttaac tcactgcaac 30780
ctccacctcc tgggttcaag tgattctcct gcctcagcct cctgagtagc tgggattaca 30840
ggtgtgtgcc accatgcctg gttaattttt ttggtttttt tgttttgttt tgagatggag 30900
tctcgctccg tcgcccaggc tggagtgcag tggcgcaacc tcggctcact gcaacttctg 30960
cctcccaggt tcaagtgatt ctcctgcctc agcctcccga gtagctggga ctacaggtgc 31020
ccaccaccac acttggctaa tttttgtatt tttagtagag acggggattc accatattgg 31080
ccaggctggt ctcaaactcc tgaccttgtg atccacccgc ctcggcctcc caaagtgctg 31140
ggattacaag catgagccac cacgcctggc cttttttttt tttttttttt tttttttttt 31200
ttgtatttta agcagagatg aggtttcgct atgttggcca ggcaggtctc aggcttctaa 31260
cctcaagtaa ccctcgggcc tcagcctccc aatgtgccga gattacaggt gtgcaccact 31320
gtgcctggca acatgttatt ttatttaatc ctcacatcat ccatgagagg tgggttgtac 31380
taataccacc atttcataaa caaaaaaact gaggcacagt ttaataactt gctggtggtc 31440
actcaatagc aaagctgggg ctcacacacc tgcagtccag tcccagtttg tgctcataac 31500
catgtgttat atggcctttc cctgcaagtt gcaggagagc tgcaacttta cctgctcttc 31560
accctctgat atggtttggt ttggctctgt gtccctactc aaacctcatg tggaatttta 31620
atcccctcac gccaggggag gaacctggtc gaaggtgatt ggatcttggg ggtgaatagt 31680
gagtgagttc tcacttaagt ggctatgttt tctggggtat ataccccagg ttcatcctct 31740
catgccggga aaattcagaa cagacacaca cagggagttt aggagcacag gttaataggc 31800
agaagaagga gaaaggaaaa cagccctctc tctaatgaga gagaggggac ttctgagagg 31860
aaacaccggc gggtggcgaa tgcaccagat tttatagtca ggcttgagga ggtggtgtct 31920

gatttacata gggctcacag attggttcaa tcaggtatga tgttcacata gcgcactggg 31980
aaggctggtc accccaccct aatcttatta tgcacatgaa ttctcctctt ggccggcacc 32040
agcttgtctg ctccttactg tacacttggc tggcagagaa gggaaaatgg agccgccatc 32100
ttaacgtgtc tggttcctag tttctgccgg cattcacccg tgcaagctcc cagcttgctt 32160
gtctatgtgt ggggctgctt ttcatttaaa agaaaagcct taccaaggac tcccgtaccc 32220
tcactatcta cctaagtgat ttcttcttaa ctcctatatc atcataagat ctgatggttt 32280
aaaagtgtgg cacttctccc ttctcactgt gtctcctgcc accatgtaag atgtgccttg 32340
cttcctcttc accttctgcc atgattgtaa gtttcctgag gcctttctgg ccatgatgaa 32400
ctgtaattca gttaaacctc ttttctttat aaatcaccca gtctcaggta gttctttata 32460
gcagtgtgaa aacggactaa tacaccctca cagcctcaag accttgagca gtgtctgatg 32520
cttacataat gagtactcct caaatatttg ttcaataaat gcattataag aatctcagtt 32580
atcctgaggc aagagaaaag aagagggtac aaacttttct ttttattttt ttgagacaga 32640
gtttaactct tgtcacccag gctggagtgc agtggtgcga tcttggctca ctgcaacctc 32700
tgctcccagg ttcaagcaat ttcctgcct cagcctccca agtagctggg actataggtg 32760
cacaccacca cgcccagcta atttttgtat ttttagtaga gacagggttt caccatattg 32820
gccaggctgg tctcgaactc ctgacctcag gtgatccacc tgtttcagcc tcccaaagtg 32880
ctgggattac agggggatac aaatttttcc tccttggcta tcagttgtac cacttgccac 32940
atctcagttt tttccacatt ttgataccag ctgctctggt tcttccagtt ttagggtctc 33000
ttgatcaaga tattgtggtt tcctctctag aaacaggctc cataacattc ttcttcttct 33060
ctctctctct aattaagcta ttgctgtatt agcacttgta agctacccag tgactgcagg 33120
acatgactgc ctcaccttct agtctgtgcc caaggtgaaa tggatgtggt agtgattctt 33180
ccacagacca agtggatcca gtgagttgcc tataggacat ccagaagact cactgacgga 33240
gtgttgtggg aaagagcaga aagctagaaa ccacctctca ttggatcttt agtccagaga 33300
ggaatccagc acgcaaaatg cagaaaaata tgatttgtcc tcacgtggac atgaagccaa 33360
ggcattctgg agagagtcag ggatatggtc agagagctta ggtggccagg aaagagagct 33420
ctacatgtgt ttccacttat ggaatcaagg aactcaatac aaagtcccaa cagaatcaac 33480
tcattcctct ctattttccc ataccccgtc agacccctag tccaagtcac catcatctct 33540
tccctgcatc cctgccacag cccctaacct acgccacaag gaactcaccc ttggcttcct 33600
ctccagagca cactttcctg acaatgaatc ttctaattct agcatctttt acaaaccagg 33660
tatcctgaga atttcccaag tcatcatgtc tttgttcctt tttcttaaca gttcctacct 33720
caatttatcc ctttttctcg ggcattttac tataaaacag caagagaaaa ccaggctaca 33780
ccttcaatac tttacttgga aagctaagcc aaatatccaa gttcaaacaa attctgcttc 33840
ccatataact gtaagacaca attccacagt ttcattccac tgtatgacaa ggatcttttt 33900
cctccagttt ccctaaacat ggtcctcatt tctttctcag ccctcaccaa caacacatta 33960

tgaaaaccta cacttctgcc aaccttctac aatgactttg atattctcta aaatgaaaca 34020
cattttcttt accatgctct tcacttccaa gtcctcacca gcagcgcctt taacatccct 34080
acttctacta acagtctttc taaggcaagc taggcttttc ctatcatgct tctcaaaatt 34140
cttccacatg ctgcccaaca cccaattcaa aagccacttc cacattgttt aggtatctgt 34200
tacagtagca caccatttcc aggtaccaaa atctgtattc gtgtactact gctgttgtaa 34260
cagatgactg cagactttgt ggcctgaaat tatacaaaat tattcttttt ttctgacgaa 34320
gtctcacgct gtagcccagg ctggagtgca gtggcacaat cttagcttac tgcaatctct 34380
acctcccggg ttcaagtgat tctcctgccc cagcttccca agtagttggg tctataggcg 34440
tgcaccacca cacctggcta atttttgtat atttagtaca tacagggttt caccatgttg 34500
gccaggctag tctcaaactc ctgacctcaa gtgatccacc caccttggcc tcccaaagtg 34560
ctggaattac aggcatgagc ccagcctcaa aactacacaa aattattatc ttatggttct 34620
ggaagttcca aatcaaggtg tcagcagagc tgcattcctt ctggaggctc tagagtgaga 34680
atgttttctt ggcttctcca gcttctacaa gccacctgca ttctttggct catccttatc 34740
ctcagatcaa gtaatggagc tctttaaatc tcaatctctc tctctcacac acacacacac 34800
acatacacac acacatacac acacacccct ttgcttccac tgtggcatct ccttctctga 34860
ttctgctttt gaccctctgc ctctctctta taaagacgct actgattata ttggacctat 34920
ttagataatc aaggataatc tctccatctc aagatccata acttaatcac aatctgcaaa 34980
atccctttta ccatgaaagc ttctggggat gaggctatgg gcatccttgg gggctatgac 35040
tcagcctacc acattcacca accccctatt atgggccaag cattggacga catatggttg 35100
cacagtggta aaaaaactgg atatgctctc tgctctcaag aaatgtatgg tctagtggaa 35160
gagtcagata tcaatcccaa agtcatacaa ataatcaccc caatacaatc attacaagtt 35220
gagatgaggg cactgaaagc ctgggaaaca tgggagtccc aagggtctct ttgaggaagt 35280
agcatccaat ctgaggttgg cagggcaggg tgataaggca cccctggatg gggaatcaga 35340
agaagacctt ccaggcagag gcatcagcaa gtgcaaaggc cctgaagaaa aagagtttgg 35400
gggatgaggg aggtggaaac ggcaaaacaa gatgaggctg taaagatgag aaggaccaga 35460
tcccttggga acttctggag catggtgggg actttagctt ttattcaaaa cataacagga 35520
agaggttgaa gaggtttttt gcttaataaa aaattttttg ttggtttttg tttgtttgtt 35580
tgtttgcttg tttttgagat agggtgtcac tctgtagccc aggctggagt gcagtgacgt 35640
gatcacagat cacttcagct cctcctgtgc tcaagtgatc ctcccacctc agcctcccaa 35700
agtgctggga ttacagtgct ggggctgtga gtcaccatgc cccaccgggt tgaggagttt 35760
ttatagcagg tgactgatgt gatcactctg ctctggagaa caaattgtgt ctgggaaaca 35820
aatggggatg agaaaaaaca ggaacacata tattcagtga agagaagaca gtggcctaaa 35880
ctgggcacat ggttgttaaa atagatagca ctgcatagat gtaagatagg tcttggacca 35940
aaaatgactc ataggacttg atggaatttg gtgtgtgatt caaaggtctg ggatatggcc 36000

atagggggtag ctggcatatg aggaatgtag caaattaatg gaaatgagaa attaaaggga 36060
ttaagatgcc aggaaggggga atgggccatc cacatgcacc ttaaaataaa tgggaaaaca 36120
gcacatggga gggaagagag gaggtaagtg ctaaaatcta cagtggataa tggggccaga 36180
gggactgggg tgtcttccaa tgatggttag aggaagagag agcacagact cctggtctga 36240
gtagcagaga gcagatgcta tgcaaggagt ctcatgtgga aacagtactg ggcagggtga 36300
agggcatcag tcccactcca gccctggatg agggacagag aaaatgagca gcttccactt 36360
taaaggctgg cagagaaatg acctcaccta agaccaggag gagcaggaaa cccttaggga 36420
agataaagtg tagtgcggta atattggttg caaaggttat tgggggcaga tttgagtggg 36480
agggtctact gtgggtcgag ttaagagaga aaatgcagag catcaagaga gtgaaggtga 36540
tggggacaat gtggaaacag tgacaagggg aatttaactg ggtgctgggt tgccaatgct 36600
ttaagtccca gtggtgtatt ccctgggggga tgttgggtgc ttaggcctct ctgtatttac 36660
cagtgggctg gaagggaacc ccagcatgct tttcctcaag gtcttctatg attagggaat 36720
gagcaagtga gtctgagccc aggagtatgg ggttgtgtac aagtgctctt acccccatgt 36780
tcatcaggtc ttctttcgtg attctgctta aatgtcacct cccaataaag cctttcctaa 36840
tttttctgac ttgtcttttc cacatacaca gaaccttgtg cagacttcta ctgtagcacc 36900
tactatatgc acattattgc ctttgctatt tatttattta tttatttatt cattgagatg 36960
gagtctcgct ctgtcaccca ggctggagta cagtggtgca atctcagctc actgtagcct 37020
ccacctccca ggttcaagca attatctgcc tcagcctgcc cagtagctgg gattgcaggt 37080
gcatgccacc acacccagct aatttttgta tgtttagtag aaacagggtt tcaccatgtt 37140
ggtcaggcta gtctcgaact cctggcctca agtgatctgc ctgccttggc ctcccaaagt 37200
gctgggatta caggcatgag ccactgcacc cagccacctt tgctttttat gtatatctcc 37260
ctaaatagaa ttgtttctca agagtgagaa taatgcttga attatttctg ttttcctgtt 37320
actgggtaca aagtctggta gccttggtag gagctacaca aatgtctaat taagtaaaac 37380
ttagccattg tttgctggtc attattgtac tatataaatg gtagatttga agaaaagttt 37440
agacactctt catataatgg ttactatgaa tcattataat gtgtatacta tgaagtatac 37500
acattacatt taacttgctt tcttaataga taattttcaa ttagttaaat taaatacaac 37560
acttcggaaa tactgtcaaa aatatgttac tggtcttaac taaaatttgc ttaaatggcc 37620
gggtgcagtg gctcacgcct gtaatcccaa cactttggga ggctgaggca ggtggaccac 37680
ctgaggtcag gagtttgaga tcagcctggc cagcatggca aaactccgtc actactaaaa 37740
atacaaaaac tagccaagca tggtggcagg catctgtaat cccagctact cgggaggctg 37800
aggagaatcg cttgaacctg ggaggctgga gattgcagtg agctgagatc acaccacttc 37860
actccagtct gggcgacaga gcaagactct gtctcaaaaa ataataaaac taaaaataat 37920
aaaatcaaat gtgcataggt accagtattt cagattttct tgataatgac tctggtgtca 37980
agctaatttc attaaaaggc ttcaattttt ttcattcaat cttgattata ataaactcac 38040

gtgtcaaaaa tcaaatatat aaatatatac agtgaaaagg ctcacggtaa ctttcttttt 38100
atctctacct gagagcttac tggttctttc ctcaattgtt ttcttttaat gtcattattt 38160
tttacgccta atgactatgc aaatgacttc cactctttgt aatcacaaac aatgctgcaa 38220
taaataatct tgttgataag tcatttcaca tgtgtacaag ttaatataag gcacttttaa 38280
ttaaactaaa tattaacaat gatgttgaat cattaaatct attgcaaact atgcagttac 38340
gttaatatgg ttaaaacaat ccctcatttc aagcttcgaa gtgttgtcct tgaatatcaa 38400
gtggtagact aaagtactcc tgtacatact gttctaagaa cattgatcaa aaggagggaa 38460
ggtgccagtt aaccagcaaa tacaacatta acacactcag ttattaaggg gcataaactt 38520
tattgacatc ctacatctat atattcatgg aactactgac aatattaata aaatatttgc 38580
tctaatgtta ttgaattttt aaaagccagt ttagttaata tcatgcttta taaccaagta 38640
atggttatat gttatatgat taatgttata tgatttcttt taaataagca taaagaatca 38700
tggaaaacta tcagcagagc caaccatgaa tagttgatgt gtataaccaa aagaaaaagg 38760
aaatcagtta tcaggagact ttcaaaagtg aatatagtag gtgccagaac tctccacagg 38820
gaatttgaga agtcctattt ctcgaggcat ttaaatggaa tagaactgaa agctagaaaa 38880
ataaactcaa aagaacaatc cttcttcagc agaagtaaaa gcccaaaatg acctaataga 38940
ttttttcttt caaaattcta ggattcaatg aacactgctc taagtacctc ttttgttgga 39000
tttgtttgtc agggtatgca ttcattagtt tgcttaaaaa ctgcattgtt tgcaacaaag 39060
aaaaacatgt tgaacagtat tggaattggt taagattccc aaacacatga ctttgttcta 39120
tattctaaga ccattccaag gatgtttccc tttgcatact gccttaatct gaaaactagt 39180
gattgaacag gctggcatta ctgagtttgt tcattacgat ttttgtggtg ttcagtttat 39240
attcctatgt agcatctttt tgtatgaatt ttcctacaat caatttaaaa tcaaccagct 39300
atgtgagttt tccagtagac atatatagca gctatatgag tttggcaatg ggcattaagc 39360
tacatgagtt tgcagctata tgagtctggc aataggcatt aatagcagct atatgtgttt 39420
ggcaataggc attaagaccg tgtaaatact ttactgttct ctgtgttttt ataatcctct 39480
gtcttttgga agtcaattaa agctttaact tcaaagaaat ccaagtgtgg aaagatgtat 39540
gtgccattca aaaacagaga agtataaaac agaatcaatt aatctgaatc ttgaaagtcc 39600
agaaataggc tctcgattta actcaaccaa tatctgtaaa gcaccaacaa tggtgcaagg 39660
ttcctgccat ggtagaagaa aaggtaagtg cagaggagtt gcagcatatg atcaatatct 39720
ttttggggca aggttcatgc tacaacagag gaaaaggtaa aaagcacagt cgaattaaag 39780
cattgatcag tatcttattt cctgacttca aaatgtaaat ctatgcatac cttcccacct 39840
ttcgcccatg ttcattccac tgcaaaaaca tgatcactgt tctcacaagg tgtctgggga 39900
gtgggctgca tggtgagcaa catggtgcaa attatagata ggatggaggg agttaaaaac 39960
cagtaaatgc aaatataaga acacattttg agatagatca aatcctctct tatgactaga 40020
gaaatgcatg cacatagaag aggtgtaggt agattttaaa cggtctcatg cactaaacta 40080

aactcaggag acaatggaga agccatagat tttgcgtggt ggaatgacat cgaacagcac 40140
ataatgaaaa ttaagcttca gaccagttgg gagacttttg gaataattca agcgagaagt 40200
aatgagagca taaaccagta tgcagggcaa tgggaaagag agaacctata gattcagaga 40260
agacagagtc cacaggacgt ggtacctgct tgaatatgtg gaataagaga ataagaaaga 40320
agtaaaagat ggctggctgg gcatgtaatc ccagtagttt gggaggccaa ggcgggcgga 40380
tcacttgagg tcaagagttc tagaccagcc tggccaacat ggagaaaccc cgtctctact 40440
aaaaatacaa aaatacaaag ccaagcgtgg tcgtgggtgt ctgtaatccc agctactcag 40500
gaggctgagg caggagaatt gcttgaaccc gggaggcaga ggttgcagtg agccaaaatc 40560
acaccactgc actccagcgt gggtgacaga acaagactcc gcctcaaaaa aaaaaaaaaa 40620
agatggctgg acttgggcca aatatacacc cttgttacaa tcagctgtgg ctaaggtgga 40680
gtcacctgca aagaacatgg ccatactggc cttctccttt gacaggtaca acaggtaggg 40740
gaagttatct aagtgggaag taggaaggct aggcacccca aactcctaca ctccactaag 40800
aaattgcata ttaaccttgg ctaagaaaac acccacacac acttccaaac tagttgaaga 40860
tcgaagaaat tagggtgcat atggtatgaa ccgagttcta ttctgtaact atacacctta 40920
gatcttgatg ggatgaaaca gagaagaaag tgataactta tgtgctgtga acaaacaaac 40980
cattggttgt ttatctcttg aactaaaagt ggagattttc aatattcaaa tggcagcgtg 41040
aagcttctgc ttatatagtt tcatatctca gctgactgca aaggcaagag ccaatagccc 41100
tccactgctc tgaataacta aaccatttca gtcttttcta cctgtcttcc atgctatatg 41160
cactctattg tcctggcttt aataaccaat aatttagata taaaactttg tgtctatatg 41220
tgtttgcaca ttttgcaaaa gattggccat acttttcata agatttacaa actggcccag 41280
tgcagtgacg gatgcctgta atcccagcac tttggaggcc aaggtgggtg gatcactgga 41340
gcccaggagt tcaagaccag ccagtgcaac atggagaaac cccatctcta caaaaaatac 41400
aaaaattcac cgggcgtggt ggcacatgcc tgtggtccca gctactcagg gagctgaggc 41460
aggaagatca cttgagccca ggaggttgaa gctgaagtga gccatgattg taccactgca 41520
ctccagccta ggtgacagaa tgagacccta tctggaaaaa aaaaaaaaaa aaaaaaaaaa 41580
tttacaaagt ggtgtcagac ccaagaaagc ctagaacctg gagagatcaa tagcacaata 41640
gactaggagt ctgtctactt tctcattttg ctcttttatt tgtattattt atttattctt 41700
aagcaattct agtaagtttt ctgatgcttt ccttagaaga atgttttcag ttaaagtagt 41760
ttgaggccgg gagcggtggc tcacgcctgt aatcccagca ccttgggtgg ccgaggcggg 41820
cagatcacga ggtcaggaga tcaagacctt cctggctaac atggtgaaac cccgtctcta 41880
caaaaaatac aaaaaattag ccgggcgtgg tggcgggcgc ctgtagtccc agctactcag 41940
gaagctgagg caggagaatg gcgtgaaccc aggaggcgga gcttgcagtg agccgagatc 42000
gcaccactgt actccagcct gggtgacaga gcaagactcc gtctcaacaa aagaaaaaaa 42060
aaaaagtagt ttgcatggca tttagaaaat ggaaagcaaa agcaccaatt aagcaaggaa 42120

gtcttgcctc aaacctgcat gtgtttcttc tataaacacc agcactattc ttataagtgt 42180
acagagcact caccgacact gaaaaccatg gaaccctgag tcatgtatta tgcttcccca 42240
tgtagacttc gcagctgtct tttaactcta ccttttatct tcaaagttca aggaaacatt 42300
tattatgatt tcaaattcct gaactcacat ctagtcctaa agtcaaaaca atataacttg 42360
gtatctaaat caactgaaat gaaatgaaat gcacttggca aaatcaccct tttatgtagt 42420
ggtggaatgc taacaactgc cctgtgacca tcctgtgcaa atccacccctt ggcctcatcc 42480
caggatatac ccaaacttcg tctcaactgt gtctctttgt cagtcacgtt aagagacagc 42540
tgtggaatgt ttggtctaga atatcccaac aggtgctcct tccgtctcaa cttctatgga 42600
gtcaaataga atttgatctg taatcctatg tcccaggctt cctaagtctt cctaaagcag 42660
atatccattt atagtcaaaa cagcaaaaac agttctcaca ctgagggata aaccgtaaat 42720
gcaattgatg aaacattcaa atctacacac cgaaataaag aactgacaaa tgaatttgtt 42780
ctttttagat taaagactat tcttaatgga actagctgag ttttgcaagt atttcacaac 42840
tttcaataag aaagagccat atgtttttca gtatgacttc agtttattac ttgtaatttc 42900
ctctattttt ttctttattg gtcttccctg tttccattct tccattttcc tgtatttttg 42960
ggacataact gtctctttct tttagagttt gtttttccca ttaattactg aacacaagaa 43020
aaatgttttg caaaatagtc tttcccacca aaaaggtata acagtagaaa aacgtttatg 43080
tgacatgcta tgctgtattc ataaggtaat ctaatttcca agctatttta aggatatgag 43140
tactcaattt ttaggggaaa gataatttag ctttgtggac tttagagcac aaattataag 43200
catagttgaa actgatacct gaacctggtg ttccagaaaa gacagattct cctttgcgtt 43260
caccattgac attgtgagtc actgacaagg tcttatgatg aaactgttta ccaatatttg 43320
caaagtcatg tttatgaata aaagcaccta aatgcttaat tttgaaacat ttaaaaaatg 43380
tgagttgcac ctcacattta tttcttggct gaactgcata caatcccatt gcagtggcct 43440
tagttgaatg gacagaggct caggaaaggc caacagtagg aacatttacc atatgttagg 43500
tgaaatgtat agaccagttg gacagctgtt aaggggataa tgttacaata caaaagctgc 43560
ctatattttt gacagtaaaa tgagcagatc agccatagtt tcctcctgtg ctcaaaaaga 43620
atgatagcac atgcctgtaa tcccagcact ttgggaggcc gagatgggca gatcgcttga 43680
ggccaggagt tccagaccag cctggccaac atggcaaaac tccgtcttta caaaaaaata 43740
cgaaaattag ctgggcatgg tggtaatccc agcttctcag gaggctgagg cacaagaatt 43800
gcttgaacct aggaggcaga ggttgcagtg agtagagatc actctgcggc actccagcct 43860
gaacaacaca gtgagattct gtcaaaaaaa agaaaagaaa aaagaatgat agcatcatat 43920
agaaagtaga ggatagctgg cacaattatg tctatcatgg aaggtcccag attctcactc 43980
tccagtatag caaccaaacc ttcctgagca taaggctgat catgccactg ggtttccctg 44040
gggtgggcat ctgttattta aagaaactgt ggttaccaac actaaaggga aataaagaaa 44100
gttctttatt ttccaacttc ctataccagt tgagaagagg ccatttaaag aagctggcaa 44160

cctgaaaccc caaaagatgt aactaataca aaggctcaac atagctgcca tttagaaaca 44220
atctttcatt tatgcacagg aataatttac tcataagccc agccctctca gttaggttaa 44280
taacaccact atcagttttc cagctgttta cttccctagc tttatttcta gacatgagaa 44340
ataaggaaca aaatatataa ttaggaaaac aattggaatg tcagcctacg gacaaaactg 44400
agacaaaatt tatataagta gagagtatat tgaggtcaaa tttgaggact gcaaccaacg 44460
aaacaccaaa tcaagttgcc ctgaatatat acagcagtta caagtgggtt tttaaaagaa 44520
aaaagaggca gtttctaagt tgagaagaat ttatattaaa ataacataaa ctatataaac 44580
tattgatagg ctaaacattg tttttttgta tcacaaattc caggaacata atgagtgagg 44640
cagttagtca ggaacaaaat gtctttaaat aattactcct gcgcatgcgt acggggggac 44700
gtgactgaag tctcacactc ctatctctct gggcctgata aattgtgtag ccttcacaaa 44760
gctcagatcg ctctgcgcta tttttctttt ttcagggagt tgcacagcta ctgacagaaa 44820
gcacatcttg gttttagtcc atagtacaaa actcaattgt gatcgaatgt tttcaacttc 44880
ctttcataat aaggttttaa actcatttca aataattaga tctggttcta atgagctgaa 44940
aagcaagaga aattcttgcg taaaaaagga aaaatatttt tccccttttc tctctgtagt 45000
tctgaaatcc agtgaaatta ctcattactg aaacttatca gatgaaaata ttggccagca 45060
ttaaattctt ttcctcccca acccccacct cttttttttcc tttttctaac caccaccctt 45120
ttgttaggcc agcttaacta gctttacatc tctgagccta gcctgagggg ccacatcctg 45180
tgtctggaat gtaaaacctc tctccccact aggcctcccg tcttccctcc acacacacac 45240
acacacacac acacacacac acacacacac acacggactt cccttgcctc caatcccatt 45300
tacatgtgta tcatttttaa atccccatct aaatttatta agtcataata ctcacttacc 45360
atttttgtac acatgctact cagtgtatat cctatgtttc ttatccaagt tgcccattct 45420
gcatgtgtct gtcttaaaat acaagctcat gcatgactta agcccggaga atttgctgag 45480
gacaaaggca atcaagatgc catgtgcaaa taacattttt ttaaaaaatc aagcttgtga 45540
tagcaataca tatttaagag ataagtggaa agatgtttga aatattgtga taaaactaac 45600
ttaatggaac cttccatact tcttcgatca tacctagaat acaatcattt ctttcttaat 45660
cttaggaaat atcaggaaac agaaaagaga ctacttgtag atatcttacc tagttcccca 45720
tgttataaaa gtatcttgtg gaagggacag gggtttttgg acctacaaaa acgcgtaatt 45780
aaccacaact gcacatggac aggaaaaagc tggaaaagca agactgttca ttcttagttt 45840
tctcctttta cttactaaag ctataatcaa ctaaagtgtg tttttcatct gaaatgaaac 45900
aagcatgagg aagccagaaa tagaacctct aactatagaa gaaaagttgt gtgatctctg 45960
ttgaatacaa aaacgcatag tttccactcg gttactgttt aattgtagtg aaagtttttg 46020
tttggcaaca aaggcgctat ttacatatta tttcttctaa gtgaaactat ggtctgggat 46080
gggttgtaga taagagcagt tgagaaccac gcttcatctc cctccttaga aactctgaaa 46140
cgaggcttta ttcctaccag aagttcagat tgcattatgg tcatctcaat tccaaaatgt 46200

tagatggcaa gaatatctgc ccatccttca ctttccttgg aaaaagttgc tcttcgggtt 46260
ttatatgcga ttgcagtttt ccagtgtgtg aaactaggaa aacaaaacac tcaacggtgt 46320
acatccctac acctaaatag tcagaaataa taggcagcta ggctaattat ccttgattag 46380
caagatcaga gccattaggg tgctcactgg tttaacaaat gaatgccctt aggcgtctat 46440
catttgtaac tcctagaagc tttaatttcc acaagaaaca aaataagagg ggccttctgc 46500
ttttaacagt gaaaagatcg ttctccctcc cctctccacc cgggtcaact cttccagccg 46560
ctccctcctg catcacgaac acacgctgca ggaaagcgca tttacagccc gggacatccc 46620
cagacctcct ctccaaaatt ccccacctcc tgtgcatagg agaaactgag agaagccctc 46680
acttcctttc caaacttcac aagcagggga gggagctgta gcagactttc acctccgttc 46740
ccaaaagcga atgtgaaaaa gtccgagaag gcacgtcctg cgagtggagg ttaaaccgaa 46800
atctgaacag aatgcacggt ccccgcaaac tacgattgat aaagaagata ctgagacgtt 46860
tgcgggggat ataagccatg gttgtctcgc cttcctcccc tccctgccaa ctatgtttct 46920
tggagaaatc gccggttcga ttcacgcaca catttttgta aaacacggac aaaaccataa 46980
gtagttacct tcattgttcc gtcggccacg agggaagctc gagctgagcg gagggcagat 47040
cccaagggtc gtagcccctg gccgtgtgga ccgggtctgc ggctgcagag cgcggtcccg 47100
gctgcagcaa gacctggggc agtgcccgag gcggcggcga gtacacgtgg cgggctggat 47160
tgcagaccgg ccctctcgcg gcggagactc gcgacctagc ggattgcatc agcaggaaga 47220
cactaaggct gctcccccag gccgccccca gatggtggag tctctcccag cccgaagatt 47280
cggagccagc gcccagaccc gagcctcact cactgctcac tcccggggtg cagggcagag 47340
gtgccagtgt tgcaagcaaa tgacacggtt acccccgaat cagccactgt gggtgcgtat 47400
ccgagtgtgg ggatgcccgt gtaacattta tatggagacg tcaaggagga ggaaataaac 47460
agatcagagg tcaaatgtga ttgccattcc gtcatcactg gctcctgccc acctccctac 47520
tgtccccaaa gtaactttgc tgcatgctga gaggaccacg gcacaatcct gcccaaaagt 47580
atacatgtat cccccgcggc tactttaaat gtacttttgc agtagtcaag aacatgtgcc 47640
tggtttgccg atctctttcc cagagttcct tagtcaccct caaatataac catttttgcc 47700
aatttattat gtacatgggg gaaaattttt aatcctttaa tttgagcata taaaattctt 47760
tataatgtga aattgcccag tagcctagtt ggtcccctgt aagttccctc cccacatcta 47820
tacaaagctc caggattgct acttttccat gtccagcgcc taggtcagcc tccatgactg 47880
cttaagtgtg ctgctggttg acctccgctg cctctctgtc aagctggcaa actcctattt 47940
ttgtaaattt caacccaagt ctttgctgtg gaagttgctt ccaatgccct caaacagaac 48000
tgggcccttg gtctatgtgc tgcactttgt ccacatcctt tctcaaaatc aattatttta 48060
ttttatcatt ttttaatgga gatggggtct cactaaattg gccaagctgg tcttgaactc 48120
ctggcctcag caatcccctc gcctccgcct cccaaattgc tgggattaca gatgtgagac 48180
tgtgcccagc ttcttaattt tactttaatt atttgtttgc acagttgtca cttcagtgga 48240

ttatgagatt cgtaagggca gatcctttgt catatttatc tcccatacca agtggcctgc 48300
cgtggaagag agggagtgtt gaaaacaaat gtgtcagtgc ttaatgactg ttggtcaggt 48360
gaatgaatgg ttaaattatg tgttcaataa atgtactgag tgatttctac atgctaggct 48420
ggtatataac agtggtttgt aatctgaaat gtctcaaaat ggagccattt atgacaaggg 48480
tttcagcctc cagtgaaatt gctgacccct caaattgata agcatatgta tggtggactg 48540
aggtgataag ataacacctg ctgtggcctg gcaccattgg caagagagta aagagagagg 48600
cagctgagat aatggctatg ggcacttgcg gatgctgtga aaaccaaaaa gtaatgaaaa 48660
cctgaaagta actgaccaca gccaaaatgc ttgcagaagt tctgccctga gaaatctgta 48720
tctgtaagca tttaatcagc cattgagtcc ttgtgaaacc tcctggcccc c 48771

<210> 2
<211> 569
<212> DNA
<213> Artificial Sequence

<220>
<223> BCAT1 cg04543413-cg23792314

<400> 2
cgaagaggtg gagattgcag gctgggactc cagatttcgg gcagggatgc ggggaaggga 60
agacgcctcg ctggaggcgg aatggagggc aaggcgaagg aggatggtgc aggaaacggc 120
gacaaggcgc ccggccaggc ccgcgagcta ccgagacccg ggttccaatc ctcccccctt 180
ccgcaaacgc ccgggttcga ggtacctggc gggcaagggc cgcagcggag cgaagcgggc 240
tggccatggg gaggctgcgg ggacgcgggg ctgcagagag cggcagtggc acggagcgcg 300
cggctggaag cgaaagcagg cggtgtggcc aagccccggc gcacggccca tagggcgctg 360
ggtaccacga cctggggccg cgcgccaggg ccaggcgcag ggtacgacgc aacccctcca 420
gcatcccttg gggaggagcc tccaaccgtc tcgtcccagt ctgtctgcag tcgctaaaac 480
cgaagcggtt gtccctgtca ccggggtcgc ttgcggaggc ccgagaatgc gcgccacgaa 540
cgagcgcctt tccaagcgca gatatttcg 569

<210> 3
<211> 122
<212> DNA
<213> Artificial Sequence

<220>
<223> BCAT1 cg20399616

<400> 3
gaagcgggct ggccatgggg aggctgcggg gacgcggggc tgcagagagc ggcagtggca 60
cggagcgcgc ggctggaagc gaaagcaggc ggtgtggcca agccccggcg cacggcccat 120
ag 122

<210> 4
<211> 1930
<212> DNA

<213> Artificial Sequence

<220>
<223> TRIM58 cg26052730-cg09789636

<400> 4
```
cgactttctg gacgacatta gagactttaa tgtactttat cctgattcag ccttccactg      60
gatgaagtaa catactctgt caaatcattt tgctcctgtc tcagcctccc ccatctgaca     120
ggtacaccac tgagaggcta agcttgaatt attcctctga tgagtatctc tatagctttg     180
tcatctccaa gactggcttt gcaacaatcc tacaacatgt gtgtattcct tgtaggatcc     240
tcattgaact aggccagata tttatttaaa tatttagaat tatttttggc tgggctgtaa     300
acctatagtt acacacacct atgcaggcat cttcccgtgt caggcggtgt ctcatttctt     360
tatcaagagg taattagtgc aagagaaaaa aatgaagttt gaaaaaaaca tttaaaaatt     420
gattatgcca tataaagggg gaaagccttt taagaaacga ttatggcatg agaggaccat     480
ggagtagctc agtaacaatg gaaggggctc ggagggtgga aggcgggcga gggatgagag     540
atcacctaat gagaacaatt tacgctatca cagcgatgcc tgcgctaaag cctagacttt     600
atgcagtgtc tccatgtaac aaaactgcac tggtacccca gaaatctgca ctaaatctat     660
ataatgatga tggaggctgg gcgcggtggc tcgcgcctgt aatcccagca ctctgggagg     720
tcgaggcggg cagatcacct gaggtcacga gttcaagacc aggctgacca aaatggtgaa     780
accccgtctc tactaaaaat acaaaaatta gccgggcatg gtggcgggcg cctgtaatcc     840
ctgctgcttg cgaggctgag gcaggagaat cgcttgaacc cgggaggcgg aggctgcagt     900
gagccgagat cgcgccaccg cactccagcc tgggcgacag agcgagactc cctctcaaaa     960
aaacaaaaac aaaaacaaac aaacaaagat ggaaggaagg agggaattta gcaattctcc    1020
agatctactg ccagtgaaaa tagcatatga ggagggattc cagttagaaa tgcccagttt    1080
cctgggtgtg tgtgtgacgg gggcggggaa ccacaacgtt aattttatcc ccgacatagg    1140
gagcgcctga gtggtggctt ttcaccgggt gtggctcgtc tgagctcttg aactgaagcc    1200
agcggacacc acccgtcggc gcctgctttc ctggggcgtg ggctcctccc cctgtgcaga    1260
ccgcgagggg agacggtgcg ggcggccggg agcgcagccc tccgggaggc gggtcatggc    1320
ctgggcgccg cccggggagc ggctgcgcga ggatgcgcgg tgcccggtgt gcctggattt    1380
cctgcaggag ccggtcagcg tggactgcgg ccacagcttc tgcctcaggt gcatctccga    1440
gttctgcgag aagtcggacg gcgcgcaggg cggcgtctac gcctgtccgc agtgccgggg    1500
ccccttccgg ccctcgggct ttcgccccaa ccggcagctg gcgggcctgg tggagagcgt    1560
gcggcggctg ggttgggcg cggggcccgg ggcgcggcga tgcgcgcggc acggcgagga    1620
cctgagccgc ttctgcgagg aggacgaggc ggcgctgtgc tgggtgtgcg acgccggccc    1680
cgagcacagg acgcaccgca cggcgccgct gcaggaggcc gccggcagct accaggtgag    1740
gcgccccccg gcgggggctg cgggcgctgc ggtgaccggg aagcgggcga cagtccggag    1800
cggagccgcc gaggccaccc gtctcctgag cggctcccac ggccgctccc cccaccgcgc    1860
```

gccgtccccc ccgcccacgc ggctcactca gtgtgggtct ctttgccttg gctgtggtaa 1920

ccccctttgc 1930

<210> 5
<211> 181
<212> DNA
<213> Artificial Sequence

<220>
<223> TRIM58 cg20810478-cg07533148

<400> 5
cgtggactgc ggccacagct tctgcctcag gtgcatctcc gagttctgcg agaagtcgga 60

cggcgcgcag ggcggcgtct acgcctgtcc gcagtgccgg ggccccttcc ggccctcggg 120

ctttcgcccc aaccggcagc tggcgggcct ggtggagagc gtgcggcggc tggggttggg 180

c 181

<210> 6
<211> 62
<212> DNA
<213> Artificial Sequence

<220>
<223> TRIM58 cg20810478-cg23054189

<400> 6
cgtggactgc ggccacagct tctgcctcag gtgcatctcc gagttctgcg agaagtcgga 60

cg 62

<210> 7
<211> 122
<212> DNA
<213> Artificial Sequence

<220>
<223> TRIM58 cg23054189

<400> 7
cgtggactgc ggccacagct tctgcctcag gtgcatctcc gagttctgcg agaagtcgga 60

cggcgcgcag ggcggcgtct acgcctgtcc gcagtgccgg ggccccttcc ggccctcggg 120

ct 122

<210> 8
<211> 122
<212> DNA
<213> Artificial Sequence

<220>
<223> TRIM58 cg20810478

<400> 8
gcggctgcgc gaggatgcgc ggtgcccggt gtgcctggat ttcctgcagg agccggtcag 60

cgtggactgc ggccacagct tctgcctcag gtgcatctcc gagttctgcg agaagtcgga 120

cg 122

<210> 9
<211> 3052
<212> DNA
<213> Artificial Sequence

<220>
<223> CDO1 cg07405021-cg16198692

<400> 9
cgctaggcac ctatttaatg caaaaaatac tgtagaattg acttctctcc catctcctgt 60
cttcccccaa ctgcccctc gggactacat ccattatgtt aatgagctgt acctcagata 120
tctgtgaaaa gaccgatcaa cccagctggt tgcacttcaa aagctttaga gtttgtcaat 180
cgagactagt tcaaggtcct tgaaaataca cttgttctaa agattaaaga ttaaaaactg 240
ttacaacctg tcctggttta acagtggttt agagtgtagg ctctggaaca acaatgctaa 300
ccccgggagc ctggagccgc tatcgataga agacctagtg caagtcattc aacttcttca 360
ttgcctgcca ttcctcatat gtgtcaagtg tctggaagag tacctgtccc ataacaagtc 420
ttcaattacc tggagcaaat gcaagaaaat ttaaaagcta gactctctgc tagctttcac 480
ggctacagaa gggctctacc atgaactaag ctgataggag ccaaaggaat ttcctcaagt 540
cacttgtata tgaaaaatgg gatccttagt ggtcttcact ttttctattt caaatcctaa 600
aatacagttg aaaccgccca tgccaacaaa actttacgaa acatcccaca tgttgaagag 660
tgactaaaag aacctgctcc caagaaaaac aaacttactt ctttccttat tcttttcttt 720
cacttacccc tttctcctaa ccctaaaccc aaaaaaccac aaagactgac gcactaactg 780
ctcagaaaaa gtagaacctc tcacacttcg gggtgattaa attttacctc tggccccgga 840
ggattggtag agagattcga cttaatcacc aagccggggc tgcaaaaaag ccagcttacc 900
agggcacctc actcaagtac atctgggaag taggggtctt taatttacat tacagatcgt 960
cagaagcaag gataagataa atcagatcat ttgggggctg aaaaaaacga cggtcgaata 1020
tttaatctgc gaaacttggc agtggatttg aataaaccca cagtcgggga agttagttaa 1080
attggtttgg gaaagggaac agcagaaatt ccgtagtggc agctgacggc cacaagacgc 1140
tattttacat gagtaaaaat gcttgctata aggtgcccgg ttgcaggcga ccgaaggcgc 1200
agtgatggag aactgacgcg ggtgacttgt cttcttcaac atcacacact tctcccgatg 1260
gctgatgtag tgctcccctg ggagcacccct ctgctagtcc ggccagacgg ttccttaagg 1320
tgtcaggagg gcccgaacct gagtgcggtc tctccgcatc tccgcgcccg gggttaacga 1380
tggcttggga gtgtgggcat tgctcccgag gccaggggca ggaggactgg tggtttagaa 1440
gcagaggggt ttgcgggaga gaagagaaca gaaaatctcg cagacgcgcg gtggcctccc 1500
ttcccggcgc ctgtccgccg ttcccggccg gaggtcagca aggccagtag gatccacctt 1560
ccgcctgcgc tgtggccgcg gagccccaag cgagtcgtgc cagccccgcg gctggccagc 1620
gagggggcga gcggcggacg cagcgcggcc cgagcttccc gagccagtca ctttgggctg 1680
cgtcccccac gtccattcct cctcagacgg ggcgagtggg cgccgcctgg cattgaagct 1740

gcagcgcgct cacctgtact ggtcgaactt ggcgtacatt gcccactcgg tggggtcgct 1800
ctcgtaggct tccatgatgg cctgcacctc ctctacattg acctcatcgc cggcaaagag 1860
ctggtgcagg atgcggatca gatcagccag ggtccgtggc ttcagcactt cggtctgttc 1920
catctcgtgg ggagctggct gcgcgcgcgt ctcactgctg ggctgcggtg gaggagctga 1980
gcgagccaag gagctggggg cgagggagcc taacagcccg ctagaccgct aagcagacac 2040
acacgcacaa acccagcatt agagtgccga aacgtaagga tgtcgtcgca gagacagcaa 2100
gagacccacc cccaggcccc tggcagcgca gtggatccgg gatcgctgga gacgcggtgc 2160
acacacaaat caggttcaga tctgtggggt tcatcctccc gggccccttt taagcgcttg 2220
gagtcactag gaatgtacca acggccctcg gagggaggac gaggcggaga gccacccaag 2280
aaaggtggcg gaggcgggga gaccctgcgg gcacggctca cgcgcacatc cccggcttcc 2340
ccgggctccg cgccttccca agagccccgt tgtctccggc gtcccaggga tcgcgtgggc 2400
tccgcgcaat ctctccccca ctttaacggc gcgttttagc cgcccggcct aacgcctctc 2460
cccgctccac ctccgccgct gtggttcgcg acgctgggac gtagacaaaa agggtcggag 2520
gagatggctg cggggactga cgctgagtaa aggaggaaaa gaaaagggaa ggagagggac 2580
acttcttcca aatagagaat tgtgaggagc ctgcgaaaat tgaggatgga ttccactcct 2640
aggccagaga gtgcctgttt tgtcgattga aactatccac gatatgcccc aggctaggtg 2700
aaaataagtc caagacttta tatatatata tatttaagta tacacacaca catatttttt 2760
aaaatgcgta ctgaaatata ttttgacaaa ggagactcgg gtctctagta agacaattag 2820
tgaaatgatg gaaaaccaag aactctttga ttttaaaatc aaatattttc ttttaaaaaa 2880
tatccagtgc aaataacctg atatttcatt tgatggtgta aaagggggct ttttctgagg 2940
ggatagggcg ggctctgacg caataattta agaggctata attatatttg aatctttcaa 3000
ccttaggcat gtgcttgaag gagcctgcta gtacatacat ataaggaaac ac 3052

<210> 10
<211> 83
<212> DNA
<213> Artificial Sequence

<220>
<223> CDO1 cg16707405-cg11036833

<400> 10
cggccctcgg agggaggacg aggcggagag ccacccaaga aaggtggcgg aggcggggag 60
accctgcggg cacggctcac gcg 83

<210> 11
<211> 122
<212> DNA
<213> Artificial Sequence

<220>
<223> CDO1 cg11036833

<400> 11

ggcggagagc cacccaagaa aggtggcgga ggcggggaga ccctgcgggc acggctcacg 60
cgcacatccc cggcttcccc gggctccgcg ccttcccaag agccccgttg tctccggcgt 120
cc 122

<210> 12
<211> 1919
<212> DNA
<213> Artificial Sequence

<220>
<223> ZNF177 cg05250458-cg14737994

<400> 12
cgtgcatgaa tacaggtgga ggccaagacc agcattaaca aaggctgtgt gtttgccatg 60
gagctcagga gggctaacag tcattttact tctgcctccg accatcacac tttgtcccca 120
gaacaaagcc aagagtcctt ggtcaatgaa agtgtgtaaa tatcctcaca cttcccctag 180
tagttccttt ttaggctgta atcatgtgct aaagaaaaga acctaacttc ttaaaatctt 240
tcccttttg gatgtagatt gaaattttg ttttaaacac aatggcccta atgtctaaat 300
ctaatgttag taattaatcc ctagtgcttt aaggatcttc tgtttatagg cagttatttt 360
atggagctgg tctctgtgag aggcacagac tgccaggagg ggttgagatt tcctttaacc 420
aggattctta gttctgaatg gctctagtga ataacattaa caatgattct ctggccgggc 480
actcacgccg gtaatcccag cactttggga ggcctagacg ggtggatcac ttgaggtcag 540
gagttcaaga ccagcctggc caacatggtg aaaccccgtc tctactaaaa atacaaaaat 600
tagccgggca tggtggcagc cgcctgtaat cccagctact tggtaggctg aggcaggaga 660
attgcttgaa cctgggaggc ggaggttgca ctgagcccac agtgcgccac tgcactacag 720
tctggatgac aagcaaagct caatctcaaa acaataattt ttaaagtaat gattatctta 780
accattctta aatccttctg tctagtagga atcttattca tgggagtgtc tggaaaaggg 840
acaaagagcg gctattgaga tttttttaaa gcctggattt gggatgtctg aacagtgaat 900
aactccaaag ccatttaaaa atcatctttt tttctaccac cggactgagc ttttcagttt 960
taattccaag gttccaatca tatagatgtg agtcaaaccc tgttgggcca aacaggatct 1020
cctttgttgg cggataattt caagtgctca cagagaccct ccgttttttt ttaaatacct 1080
ggacttaatt gtttaaactg gagtccggac caaaacatct ctgacctcgg gggtcttcct 1140
ttggtcagga agaggggagg gagcaattgg accccttcc ctctgcggct gcagagtcca 1200
attttcctct ggggacattt gctttttccc ttggggaaga cagtacggat tctagttctg 1260
tggactcttt tacttagctt tgagacccaa gcaagttaat tggcttgtgc ctcttatttt 1320
actctaatgc aatgaataaa gacagtccca gccttcgccc taagggagca ggagcacctg 1380
cgatgccccg ttcccaagtc ctcagggcga atccgccaaa tgtgcgagct gggcagccca 1440
cccctttcag ctgctggccg gaagcggaag tgggcgtccg tcgcctcgcc atctcccata 1500
gctgtcgcct gcagctgaga aagggttgct gtcccagcag gccaggtcca ggtgcgcccc 1560

actagtgagg ccggcggaat cgggagggaa ggggtcaagg gcacagtgcg cagccccggc 1620
tgctccagac cttgcctgca gcttcccgcc ccagcctccg gctatcgcgg cgtttcttct 1680
cagaggccgc cggctttggt tcctcccggc actgctgggc ttggggctgc cattccgggc 1740
attggttccc gggaggaggc aagtgggttc atgtggtcag agtgcgtcgg cggggccttt 1800
tctccacttt ctgtaccctt ctctttaaac gtcgtcccca gtaggagact attttttctc 1860
aagaaaatgc tctcttggac ctcactttcc gttgttagaa ggcatatctt gggatgcgc 1919

<210> 13
<211> 283
<212> DNA
<213> Artificial Sequence

<220>
<223> ZNF177 cg05928342-cg17283453

<400> 13
cgttcccaag tcctcagggc gaatccgcca aatgtgcgag ctgggcagcc cacccctttc 60
agctgctggc cggaagcgga agtgggcgtc cgtcgcctcg ccatctccca tagctgtcgc 120
ctgcagctga gaaagggttg ctgtcccagc aggccaggtc caggtgcgcc ccactagtga 180
ggccggcgga atcgggaggg aaggggtcaa gggcacagtg cgcagccccg gctgctccag 240
accttgcctg cagcttcccg ccccagcctc cggctatcgc ggc 283

<210> 14
<211> 95
<212> DNA
<213> Artificial Sequence

<220>
<223> ZNF177 cg05928342-cg07788092

<400> 14
cgttcccaag tcctcagggc gaatccgcca aatgtgcgag ctgggcagcc cacccctttc 60
agctgctggc cggaagcgga agtgggcgtc cgtcg 95

<210> 15
<211> 122
<212> DNA
<213> Artificial Sequence

<220>
<223> ZNF177 cg08065231

<400> 15
gccaaatgtg cgagctgggc agcccacccc tttcagctgc tggccggaag cggaagtggg 60
cgtccgtcgc ctcgccatct cccatagctg tcgcctgcag ctgagaaagg gttgctgtcc 120
ca 122

<210> 16
<211> 26
<212> DNA
<213> Artificial Sequence

<220>
<223> Primer

<400> 16
gaggtttttt tttaagggat gttgga 26

<210> 17
<211> 18
<212> DNA
<213> Artificial Sequence

<220>
<223> Primer

<400> 17
tccaatcctc ccccttc 18

<210> 18
<211> 21
<212> DNA
<213> Artificial Sequence

<220>
<223> Primer

<400> 18
aactaaccat aaaaaaacta c 21

<210> 19
<211> 27
<212> DNA
<213> Artificial Sequence

<220>
<223> Primer

<400> 19
tgttyggtgt gtttggattt tttgtag 27

<210> 20
<211> 19
<212> DNA
<213> Artificial Sequence

<220>
<223> Primer

<400> 20
cacrctctcc accaaaccc 19

<210> 21
<211> 18
<212> DNA
<213> Artificial Sequence

<220>
<223> Primer

<400> 21
atagtttttg ttttaggt 18

<210> 22

<211> 27
<212> DNA
<213> Artificial Sequence

<220>
<223> Primer

<400> 22
aatgtgygag ttgggtagtt tattttt 27

<210> 23
<211> 26
<212> DNA
<213> Artificial Sequence

<220>
<223> Primer

<400> 23
ctactaaaac aacaaccctt tctcaa 26

<210> 24
<211> 23
<212> DNA
<213> Artificial Sequence

<220>
<223> Primer

<400> 24
agtttatttt ttttagttgt tgg 23

<210> 25
<211> 20
<212> DNA
<213> Artificial Sequence

<220>
<223> Primer

<400> 25
gttaaagtgg gggagagatt 20

<210> 26
<211> 25
<212> DNA
<213> Artificial Sequence

<220>
<223> Primer

<400> 26
tcatcctccc caarcccttt taaac 25

<210> 27
<211> 15
<212> DNA
<213> Artificial Sequence

<220>
<223> Primer

<400> 27

gggtttttgg gaagg 15